(19) Europäisches Patentamt
European Patent Office
Office européen des brevets

(11) EP 2 436 366 B1

(12) **EUROPÄISCHE PATENTSCHRIFT**

(45) Veröffentlichungstag und Bekanntmachung des Hinweises auf die Patenterteilung:
**29.07.2015 Patentblatt 2015/31**

(51) Int Cl.:
***A61K 6/083*** *(2006.01)* ***A61K 6/00*** *(2006.01)*

(21) Anmeldenummer: **11183345.5**

(22) Anmeldetag: **29.09.2011**

(54) **Kompositmaterial umfassend ein Monomer mit einem polyalicyclischen Strukturelement als Versiegelungsmaterial**

Composite material comprising a monomer with a polyalicyclic structure as sealing material

Matériau composite comprenant un monomère doté d'une structure polyalicyclique comme matériau de scellement

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.09.2010 DE 102010041800**

(43) Veröffentlichungstag der Anmeldung:
**04.04.2012 Patentblatt 2012/14**

(73) Patentinhaber: **VOCO GmbH**
**27472 Cuxhaven (DE)**

(72) Erfinder:
- **Blömker, Tobias, Dr.**
**20251 Hamburg (DE)**
- **Stepputtis, Dr. Manfred**
**27449 Kutenholz (DE)**
- **Maletz, Dr. Reinhard**
**27472 Cuxhaven (DE)**
- **Plaumann, Manfred Thomas**
**27472 Cuxhaven (DE)**

(74) Vertreter: **Eisenführ Speiser**
**Patentanwälte Rechtsanwälte PartGmbB**
**Postfach 10 60 78**
**28060 Bremen (DE)**

(56) Entgegenhaltungen:
**EP-A1- 1 413 569** **EP-A2- 2 016 931**
**EP-B1- 0 969 789**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 436 366 B1

## Beschreibung

Die vorliegende Erfindung betrifft bestimmte (vorzugsweise lichthärtbare) Kompositmaterialien umfassend ein Monomer mit einem polyalicyclischen Strukturelement bzw. deren Anwendung in einem therapeutischen dentalen Verfahren als Versiegelungsmaterial zur Versiegelung von Fissuren und/oder Grübchen und/oder kariösen Läsionen. Beschrieben werden nachfolgend außerdem neue polymerisierbare Monomere umfassend mindestens ein polyalicyclisches Strukturelement und bestimmte ethylenische Strukturelemente, die für den Einsatz in einem erfindungsgemäßen bzw. erfindungsgemäß anzuwendenden dentalen Kompositmaterial besonders geeignet sind, und deren Verwendung in einem erfindungsgemäßen bzw. erfindungsgemäß anzuwendenden dentalen Kompositmaterial.

Versiegelung von Fissuren

Unter einer Fissurenversiegelung wird das Auffüllen der zum Teil sehr tiefen Grübchen, rauhen Furchen und Rinnen auf der Oberfläche von Zähnen mit einem gut fließenden Kunststoffmaterial verstanden. Die oftmals engen Einziehungen auf den Kauflächen werden Fissuren genannt. Bei Kindern und Jugendlichen, aber auch bei Erwachsenen, entwickelt sich häufig Karies zuerst in den Fissuren der Kauflächen, wobei die okklusalen Flächen der Seitenzähne die höchste Kariesanfälligkeit zeigen. Ein stark ausgebildetes und zerklüftetes Relief befindet sich beispielsweise auch an der Innenseite der Frontzähne, so dass auch hier eine kariesprophylaktische Versiegelung sinnvoll sein kann. Zusätzlich fördert ein deutlich ausgeprägtes Fissurenrelief auch die Plaqueanheftung. Plaqueakkumulation wurde auch in engen Fissuren und an steilen Höckerabhängen beobachtet.

Die verschiedenen Fissurentypen (beispielsweise ampullenförmig, I-förmig, U-förmig, V-förmig) können Speisereste aufnehmen und somit auch für Karies verursachende Bakterien einen idealen und geschützten Lebensraum bieten, da mundhygienische Maßnahmen wie das Putzen der Zähne hier nicht greifen: Die Borsten der Zahnbürsten sind im allgemeinen zu breit, um den Boden der Fissur reinigen zu können. Die Morphologie der Fissuren macht daher eine mechanische Reinigung der Grübchen fast unmöglich. Sollte in der Fissur eine Schmelzkaries entstehen, dann wird sie sich mit hoher Wahrscheinlichkeit sehr schnell ins Dentin ausweiten, da die Schmelzdicke im Bereich der Fissur, insbesondere am Fissurengrund, in der Regel sehr dünn ist. Die große Anfälligkeit der Fissuren für einen kariösen Befall wird somit hauptsächlich durch ihre spezielle Morphologie erklärt.

Weiterhin wurde berichtet, dass die Kariesgefahr in den Fissuren gegenüber glatten Zahnflächen deutlich erhöht ist, da in den Fissuren eine Fluoridprophylaxe nicht die gewohnte Wirksamkeit entfalten kann.

Man unterscheidet verschiedene Formen der Fissurenversiegelung:

- Bei der prophylaktischen Fissurenversiegelung werden enge, tiefe Fissuren mit einem geeigneten, dünnfließenden Material versiegelt.

- Bei der Versiegelung mit vorherigem Ausschleifen der Fissur wird die Fissur mit Hilfe eines kleinen Bohrers minimal erweitert, und, wenn keine Karies vorliegt, die Versiegelung mit dem Material durchgeführt.

- Wird beim Ausschleifen der Fissur Karies gefunden, so muss diese entfernt werden. Der mit der Präparation entstandene Defekt wird zunächst mit einer Kompositfüllung versorgt, die dann mit dem Versiegelungsmaterial überschichtet wird.

Durch eine Versiegelung der Fissuren wird das Zahnrelief flacher, der Zahn ist leichter zu reinigen und die Entstehung einer Karies kann so verhindert werden. Die Fissurenversiegelung ist heutzutage als bewährte und empfohlene, effektive prophylaktische Maßnahme anerkannt und findet in der täglichen zahnärztlichen Praxis eine immer breitere Anwendung. Es gilt die Regel, dass alle kariesgefährdeten Fissuren und Grübchen bereits vorsorglich versiegelt werden sollten. Man konnte nachweisen, dass ein solches Vorgehen zu einer deutlichen Abnahme kariogener Mikroorganismen in der Fissur unterhalb der Versiegelung führt.

Versiegelung kariöser Läsionen

Während Fissurenversiegeler flüssige Kunststoffe sind, die nach Anätzen des Schmelzes in das okklusale Fissurenrelief eingebracht werden, um nach dem Aushärten eine mechanische und chemische Barriere für Bakterien und deren kariogene Stoffwechselprodukte zu bilden und so eine Kariesentstehung, bzw. Kariesprogression zu verhindern, können flüssige, niederviskose Versiegelungskunststoffe auch zur Infiltration initialer kariöser Läsionen verwendet werden. Initiale kariöse Läsionen sind Schmelzareale mit erhöhtem Porenvolumen. Diese Poren stellen Diffusionswege für eine fortschreitende Auflösung der Schmelzstruktur dar. Aus diesem Grund wurde vorgeschlagen, durch Infiltration mit härtbaren Materialien sowohl einen Verschluss der Diffusionswege als auch eine Stabilisierung der geschädigten

Schmelzstruktur zu erreichen. Man wies nach, dass niederviskose Kunststoffzusammensetzungen in Läsionen penetrieren und nach Aushärtung eine weitere Demineralisation verhindern können.

Als Standardmaterialien zur Versiegelung von Fissuren, Grübchen und Kariesläsionen werden bislang regelmäßig Kunststoffe auf Basis von Dimethacrylat eingesetzt, die entweder nicht oder, im Vergleich zu Füllungskompositen, geringer gefüllt sind. Diese Materialien zeigen das günstigste Retentionsverhalten. Aufgrund ihrer geringeren Füllstoffbeladung fließen die Versiegeler im Vergleich zu Füllungskompositen besser in die Tiefe der Fissur oder der Läsion. Allerdings sind sie durch den reduzierten Füllstoffanteil weniger abrasions- und biegefest als Füllungsmaterialien.

Man unterscheidet die Materialien beispielsweise nach der Art ihrer Aushärtung, die entweder durch Licht- und/oder Autopolymerisation erfolgen kann. So sind lichthärtbare Fissurenversiegeler als Einkomponentenmaterialien im Vergleich zu den zweikomponentigen autopolymerisierbaren Versiegelungsmaterialien weniger verarbeitungsanfällig, da es keine Blasenbildung während des Anmischens geben kann. Diese Materialien sind somit vom klinischen Standpunkt bevorzugt.

Ein weiteres Unterscheidungsmerkmal betrifft das Erscheinungsbild des Materials. So gibt es transparente und eingefärbte Produkte. Transparente Präparate sind ungefüllt oder mit nanoskaligen Füllstoffteilchen versetzt, deren Größe kleiner ist als die Wellenlänge des sichtbaren Lichts. Diese Materialien erlauben dem Zahnarzt, beispielsweise nach der Versiegelung einer Fissur, ein mögliches Fortschreiten der Karies in der Tiefe der Fissur zu erkennen. Andererseits ermöglicht ein eingefärbtes Material das Erkennen von Defekten der Versiegelung bei Nachkontrollen. Dies wäre bei transparenten Versiegelungen nicht möglich.

Viele Varianten der dentalen Versiegelungsmaterialien werden auch als fluoridabgebende Materialien angeboten.

In der DE 23 01 067 werden Dentalfissurenversiegelungsmittel vorgeschlagen, die stark verbesserte Handhabungseigenschaften und ein vorzügliches Vermögen aufweisen sollen, sich entwickelnde Löcher und Fissuren in Zähnen vollständig zu füllen und zu versiegeln, wobei ihre Zusammensetzungen als hauptsächliche härtbare Monomerkomponente Glykoldimethacrylate, wie beispielsweise Ethylenglycoldimethacrylat, Diethylenglykoldimethacrylat, etc. enthalten. Den Erfindern ist aufgefallen, dass die Verwendung aromatischer Dimethacrylate zum Versiegeln von Fissuren ungeeignet ist, da ihre Viskosität zum Einfließen in die Löcher und Fissuren zu hoch sei und mit ihnen keine vollständige Versiegelung und gute Haftung zu erzielen sei. Glykoldimethacrylate wurden als sogenannte "reaktive Verdünnungsmonomere" seit jeher in dentalen Kompositzusammensetzungen verwendet, um die hohe Viskosität der aromatischen Dimethacrylate anzupassen und zu steuern. Die offenbarten Zusammensetzungen sind zweikomponentig ausgelegt und werden chemisch ausgehärtet.

Es ist zu erwarten, dass Zusammensetzungen, wie sie in der DE 23 01 067 beschrieben sind, aufgrund der relativ großen Menge an reaktivem Verdünnermonomer viel Wasser aufnehmen werden. Die Ethergruppen $-CH_2-CH_2-O-$ zeigen freie, ungehinderte Rotation und bilden hochflexible Molekülketten. Die Packung der Ketten im Polymerisat ist somit nicht starr und fixiert, sondern beweglich. Das polymere Netzwerk wird für einen Eintritt von Wasser geöffnet. Es ist somit sehr wahrscheinlich, dass große Mengen an Wasser ins Polymerisat gelangen, es zusätzlich aufweiten und es dann irreversibel hydrolytisch spalten und abbauen.

In der US 6,573,312 B2 wird eine Zusammensetzung zum Versiegeln/Füllen von Fissuren und Grübchen angegeben, die ein chemisch modifiziertes Bis-GMA (2,2-Bis[4-(2-hydroxy-3-methacryloyl-oxypropoxy)phenyl)propan) enthält. Die Zusammensetzung soll verbesserte physikalische und mechanische Eigenschaften des Polymerisats erzielen und die Umsetzung der Polymerisation soll bei Photohärtung in höheren Raten erfolgen.

Die Modifizierung des Bis-GMA erfolgt durch sukzessive Reaktion der sekundären Hydroxylgruppen des Bis-GMA mit Methacrylsäurechlorid in Gegenwart eines organischen Amins. Im ersten Syntheseschritt wird das Dimethacrylat so zu einem Trimethacrylat (Tri-GMA) und in einem zweiten Schritt zu einem tetrafunktionalisierten Methacrylat (Tetra-GMA) abreagiert.

Die Zusammensetzungen bestehen aus Bis-GMA und Tri-GMA sowie aus Bis-GMA, Tri-GMA und Tetra-GMA, wobei die Zusammensetzungen ferner Füllstoffe, Photoinitiatoren, Additive und Verdünnungsmonomere enthalten. Als Verdünnungsmonomere werden Methylmethacrylat und Glykoldimethacrylate vorgeschlagen.

Die besonderen Eigenschaften dentaler Zusammensetzungen, basierend auf Bis-GMA, beruhen auf der Möglichkeit des Moleküls, neben der Verknüpfung über die Methacrylatgruppen, zusätzliche Struktureinheiten, sogenannte Überstrukturen oder Sekundär/Tertiärstrukturen über Wasserstoffbrückenbindungen, ausgehend von den freien Hydroxylgruppen, auszubilden. Der Aufbau dieser Überstrukturen kann ebenfalls durch Carbamat-, Amid- oder ähnlich zusammengesetzte Gruppen, nicht jedoch durch Estergruppen befördert werden. Weiterhin bedingt durch die starre Konformation des Bisphenol-A-Strukturmotivs weist das Polymerisat enge, dicht gepackte und über mehrere Bindungstypen miteinanderwechselwirkende Molekülverbände auf. Diese strukturellen Voraussetzungen begründen die guten mechanischen und physikalischen Eigenschaften von Bis-GMA Polymerisaten.

Werden diese zusätzlichen Wechselwirkungen durch Funktionalisierung der freien Hydroxylgruppen blockiert, so sollte die Ausbildung der Überstrukturen behindert und ein Abfall der physikalischen und mechanischen Werte der ausgehärteten Zusammensetzungen die Folge sein.

Zudem sind die in US 6,573,312 B2 aufgeführten Werte der physikalischen Eigenschaften lediglich geschätzt. Unterschiede zwischen den Werten der Vergleichsbeispiele und denen der erfindungsgemäßen Beispiele gemäß US 6,573,312 B2 sind nicht ersichtlich.

Die US 2005/0288387 A1 beschreibt dentale Zusammensetzungen, die zur Beschichtung von Zähnen oder als dentale Versiegelungsmaterialien einsetzbar sein sollen. Die Zusammensetzungen enthalten eine Multiacrylatverbindung, einen Initiator und einen Alkohol. Die Multiacrylatverbindung enthält wenigstens 3 Acrylateinheiten pro Molekül, beispielsweise Dipentaerythritolpentaacrylat, Di-trimethylolpropantetraacrylat, Trimethylolpropantriacrylat, etc.. Der Initiator ist bevorzugt ein Photoinitiator.

Die US 2009/0047633 A1 betrifft ebenfalls ein dentales Versiegelungs- und/oder Beschichtungssystem, das an Zahnhartsubstanz selbst haftet. Die Zusammensetzung enthält 10 bis 60 Gew.-% einer polymerisierbaren Verbindung, umfassend Bis-GMA und Urethanverbindungen sowie deren Mischungen, 1 bis 40 Gew.-% einer polymerisierbaren sauren Verbindung, 3 bis 60 Gew.-% eines Kieselsäurefüllstoffs mit einer durchschnittlichen Teilchengröße von 1 bis 100 nm, 1 bis 30 Gew.-% Wasser und 10 bis 60 Gew.-% Lösungsmittel.

Die EP 0 969 789 B1 beschreibt dentale Versiegelungs- und Beschichtungsmaterialien, die mindestens 10 Gew.-% eines polymerisierbaren Materials, 0,01 bis 20 Gew.-% eines Füllstoffs im Nanobereich, der eine primäre Teilchengröße zwischen 1 und 100 nm aufweist, wobei die Oberfläche des Füllstoffs im Nanobereich durch eine chemische Oberflächenbehandlung mit einem Silanisierungsmittel modifiziert ist und mindestens 10 Gew.-% eines organischen Lösungsmittel mit einem Siedepunkt, der unterhalb von Wasser liegt, umfassen.

Auch die US 6,899,948 B2 zielt auf dentale Versiegelungsmaterialien, enthaltend neben härtbaren Monomeren nicht-aggregierte, oberflächenmodifizierte Kieselsäureteilchen mit durchschnittlichen Teilchengrößen von kleiner 200 nm.

Die WO 2007/028159 A2 betrifft klare, transparente und opake dentale Versiegelungszusammensetzungen, bei denen kolloidale Kieselsäureteilchen mit einer durchschnittlichen Teilchengröße von 10 bis 100 nm im Methacrylat(Acrylat)harz dispergiert sind. Die Zusammensetzungen sind photochemisch härtbar und sollen harte, glatte und glänzende Beschichtungen auf dem Zahn ergeben. Die nicht-agglomerierten Kieselsäureteilchen sollen homogen innerhalb der Harzmatrix verteilt sein, so dass die Dispersion eine niedrige Viskosität aufweist. Die Polymerisate sollen gute mechanische Werte wie Abriebfestigkeit aufweisen. Bevorzugt werden die Zusammensetzungen zum Versiegeln von Fissuren und Grübchen auf der Oberfläche eines Zahns oder einer Restauration benutzt.

Unter anderem werden auch in der WO 01/30307 A1 dentale Zusammensetzungen mit einer visuellen Opazität bei einem Wert von kleiner als 0,25 angegeben. Da die reziproke Eigenschaft der Transluzenz die Opazität darstellt, wird hier eine Zusammensetzung beschrieben mit Werten der Transluzenz, die nicht kleiner als 0,75 sind. Diese Zusammensetzungen, die auch als Versiegelungsmaterialien eingesetzt werden können, sollten ebenfalls transparent sein. Sie weisen, neben einer härtbaren Harzzusammensetzung, Kieselsäureteilchen mit einem durchschnittlichen Durchmesser von weniger als 200 nm auf.

Eine temporäre, transparente, dentale Lackzusammensetzung zur Beschichtung von Zahnoberflächen beschreibt die US 2006/0063853 A1. Hier wird die Transparenz durch den Zusatz von Glashohlkugeln zur Zusammensetzung bewirkt, wobei die Glashohlkugeln jedes ankommende Licht zurück zur Lichtquelle umleiten. Dieser Effekt wird durch eine im Inneren der Kugeln stattfindende Totalreflexion bewirkt, die immer dann stattfindet, wenn Licht in eine Glashohlkugel eintritt. Dieses Phänomen wird insbesondere bei Straßenmarkierungen genutzt. Um diesen Effekt hervorbringen zu können, muss der Brechungsindex des Bindemittels geringer sein als der der Glashohlkugeln.

Sowohl US 2007/0166450 A1 als auch US 2010/0016464 A1 beschreiben dentale Beschichtungs- und Versiegelungsmaterialien, die Fluoreszenzmittel enthalten.

Die WO 2005/094757 A1 beansprucht eine dentale Zusammensetzung, die für einen Einsatz als Fissuren- und Grübchenversiegeler geeignet ist und ein mittels Wärme aushärtbares Polymerisationssystem, das einer Stufenwachstumspolymerisation unterliegt, umfasst. Das System kann beispielsweise ein Epoxid-Amin-, ein Epoxid-Thiol, ein Epoxid-Carbonsäure-, ein Epoxid-Phenol-, ein Isocyanat-Amin-, ein Isocyanat-Alkohol-, ein Isocyanat-Thiol-, ein Isothiocyanat-Amin-, ein Isothiocyanat-Alkohol-, ein Isothiocyanat-Thiol-, ein geblocktes Isocyanat-, ein Siloxansystem oder ein ähnlich härtbares System sein.

In der EP 1 307 173 B1 wird ein Fluoridlack und Fissurenversiegelungsmaterial auf Silikonbasis beschrieben.

Die Druckschriften DE 20 2006 020 483 U1, DE 20 2006 020 480 U1, DE 20 2006 020 479 U1, DE 20 2006 020 477 U1, DE 20 2006 020 476 U1, EP 2 023 884 A1, EP 1 854 445 A1, EP 2 145 613 A1 und EP 2 151 229 A2 betreffen Zusammensetzungen zur Infiltration von Zahnschmelz bei der Behandlung oder Prävention von Kariesläsionen.

(Meth)acrylsäureester des Bis(hydroxymethyl)tricyclo[5.2.1.0$^{2,6}$]decans sind als Monomerbausteine zur Herstellung dentaler Versiegelungsmassen für Zahnschmelz-Fissuren bekannt. In der DE 28 16 823 C2 wird der Ester ohne Comonomeren und ohne Füllstoff für eine solche Verwendung eingesetzt. Zusammensetzungen des Esters für Zahnfüllmaterialien wurden gemäß dieser Druckschrift mit den Comonomeren Bis-GMA und Hexandioldimethacrylat abgemischt.

EP 0 254 185 A1 betrifft Urethangruppen enthaltende (Meth)acrylsäureester von Tricyclo[5.2.1.0$^{2,6}$]decanen

und deren Verwendung als Dentalwerkstoffe.

DE 10 2007 034 457 (entsprechend EP 2 016 931 A2) beschreibt Formulierungen mit einer Monomerenmischung umfassend Bis-GMA und TCD-di-HEMA (Bis(methacrylolyoxymethyl)-tricyclo[5.2.1.$0^{2,6}$]decan) bzw. TCD-di-HEA (Bis(acrylolyoxymethyl)tricyclo[5.2.1.$0^{2,6}$]decan) mit verschiedenen Vernetzern. Die darin angegebenen Rezepturen betreffen ausschließlich dentale Kompositmaterialien zum Einsatz als standfeste bzw. stopfbare Füllungswerkstoffe. Entsprechendes gilt für DE 10 2005 021 332 und die entsprechende US 7 601 767 B2.DE 35 22 005 A1 offenbart (Meth-)Acrylsäure-Derivate von Tricyclodecanen und deren Verwendung im Dentalbereich. Entsprechendes gilt für DE 35 22 006 A1.

DE 35 22 005 offenbart (Meth-)Acrylsäure-Derivate von Tricyclodecanen und deren Verwendung im Dentalbereich. Entsprechendes gilt für die DE 35 22 006 A1.

US 6 617 413 B1 offenbart Verbindungen, welche polymerisierbare Doppelbindungen und weitere funktionelle Gruppen umfassen.

Die Anforderungen an Versiegelungsmaterialien zur Versiegelung von Fissuren, Grübchen und Kariesläsionen sind äußerst vielfältig. Zunächst müssen die Zusammensetzungen für lange Zeit auf der Zahnoberfläche bleiben, um den Zahnschmelz vor Kontamination mit Stoffwechselprodukten von Bakterien zu schützen. Im günstigsten Fall sollte das Material so lange auf dem Schmelz bleiben, wie der Zahn im Munde des Patienten ist. Solche extremen Anforderungen sind jedoch kaum zu erfüllen. Es sollte allerdings bis zu dem Zeitraum auf der Zahnoberfläche verbleiben, in dem die größte Kariesaktivität verzeichnet wird. Dies ist die Zeit bis zum 17./18. Lebensjahr.

Zur Erreichung möglichst langfristiger Versiegelungen sind möglichst abrasionsfeste Materialien, die den Kaudrücken gut widerstehen, erforderlich. Bessere Abriebbeständigkeit weisen Produkte mit möglichst hohem Füllstoffgehalt auf. Diese sind jedoch in den teilweise sehr engen und feinen Fissuren nicht mehr oder nur sehr schwer applizierbar. Es muss somit ein Kompromiss zwischen einer möglichst hohen Füllstoffbeladung einerseits und einem sehr gut fließfähigen, möglichst niederviskosen Material gefunden werden.

Eine weitere, ganz wichtige Eigenschaft von Versiegelungsmaterialien zur Versiegelung von Fissuren, Grübchen und Kariesläsionen, sollte eine möglichst geringe Wasseraufnahme des Präparats sein. Bei der radikalischen Vernetzung von Methacrylat/Acrylatzusammensetzungen entsteht ein dreidimensional verknüpftes Netzwerk. Aufgrund der sehr geringen Größe des Wassermoleküls kann Wasser in die Maschen des Polymerisats eindiffundieren, wo es sich an bestimmten Stellen im Netzwerk anlagert und dort Wasserstoffbrücken, bzw. andere schwach polare Bindungen ausbildet. Je mehr polare Anteile in der Polymermatrix vorhanden sind, umso leichter kann eine weitere Wasseraufnahme erfolgen. Durch die Wasseraufnahme weitet sich das Polymerisat auf und es kommt zu einer Vergrößerung der Intermolekularabstände. Diese hygroskopische Expansion kann dann zu einer strukturellen Reorganisation der Polymerketten führen. Die entstehende Druckspannung am Boden der Grübchen und/oder Fissuren kann die Zahnhartsubstanz dabei nachhaltig schädigen. Die Wasseraufnahme erfolgt über einen längeren Zeitraum nach der Aushärtung, so dass es bei einer übermäßigen Wasseraufnahme zu einem Expansionsstress und somit zu einem "Überquellen" des Materials kommt. Darüber hinaus können Wassermoleküle empfindliche Strukturelemente des Polymerisats, wie beispielsweise Estergruppen, angreifen und hydrolytisch spalten. Dieser Abbau kann zur völligen Desintegration des Netzwerks und somit zum Verlust des Produktes führen.

Andererseits ist eine gewisse Polarität der Versiegelungsmaterialien jedoch auch wünschenswert, da die Zahnhartsubstanz hydrophil ist und polare Zusammensetzungen eine gute Adaptation und Benetzbarkeit des Materials am Zahnsubstrat gewährleisten.

Aufgabe der Erfindung war es, ein dentales Material zu finden, welches zur Versiegelung von Fissuren, Grübchen und kariösen Läsionen geeignet ist, das sich durch eine sehr geringe Wasseraufnahme, ein sehr gutes Anfließverhalten an der Zahnhartstruktur sowie durch ausgezeichnete mechanische Eigenschaften auszeichnet.

Insbesondere sollte das zu findende Material vorzugsweise gleichzeitig

- im ausgehärteten Zustand eine Wasseraufnahme besitzen, die kleiner ist als 25 $\mu g/mm^3$,
- ein gutes Anfließverhalten besitzen entsprechend einem Kontaktwinkel auf trockenem Zahnschmelz von kleiner 60° und
- im ausgehärteten Zustand eine Biegefestigkeit aufweisen, die größer ist als 105 MPa.

Dabei sollten die angegebenen Werte der Eigenschaften des zu findenden Materials bei Meßbedingungen gemessen werden, wie sie weiter unten bei den "Meßmethoden" angegeben sind.

Weitere Aufgaben ergeben sich aus der nachfolgenden Beschreibung und den beigefügten Patentansprüchen.

Die primäre Aufgabe wird gelöst durch ein Kompositmaterial bestehend aus oder umfassend:

(a) als Füllstoffkomponente eine Gesamtmenge an Füllstoffen im Bereich von 0,5 bis 60 Gew.-%, bezogen auf die

Gesamtmasse des Kompositmaterials, wobei die Gesamtmenge an Füllstoffen eine Mischung von Füllstoffen ist umfassend

(a1) eine Gesamtmenge im Bereich von 0,5 bis 60 Gew.-% an nicht agglomerierten, organisch oberflächenmodifizierten Nanopartikeln mit einer mittleren Partikelgröße kleiner 200 nm (vorzugsweise kleiner 60 nm) und

(a2) eine Gesamtmenge im Bereich von 0 bis 59,5 Gew.-% an Mikropartikeln mit einer mittleren Partikelgröße von 0,4 $\mu$m bis 10 $\mu$m
sowie

(a3) gegebenenfalls zusätzlichen Füllstoffen,
wobei die Gewichtsprozentangaben für die Komponenten (a1) und (a2) jeweils auf die Gesamtmasse des Kompositmaterials bezogen sind,

(b) als Monomerenkomponente eine Gesamtmenge an polymerisierbaren Monomeren im Bereich von 24 (vorzugsweise 39) bis 98,5 Gew.-%, bezogen auf die Gesamtmasse des Kompositmaterials, wobei die Gesamtmenge an polymerisierbaren Monomeren umfasst

(b1) ein, zwei oder mehr Monomere ausgewählt aus der Gruppe bestehend aus Verbindungen (Monomere) der Struktur $Q(Y_xZ_e)_b$, wobei gilt:

- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten $Y_xZ_e$ substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sind,

- b ist eine natürliche Zahl ausgewählt aus der Gruppe der natürlichen Zahlen 1, 2, 3 und 4,

- jedes Z bedeutet ein Strukturelement, das unabhängig von etwaigen weiteren Strukturelementen Z ausgewählt ist aus der Gruppe bestehend aus

$-O-(C=O)-CH=CH_2$, $-O-(C=O)-C(CH_3)=CH_2$,

$-(C=O)-CH=CH_2$, $-(C=O)-C(CH_3)=CH_2$

$-CH=CH_2$, $-C(CH_3)=CH_2$ und $-O-CH=CH_2$,

- jeder Index e ist eine natürliche Zahl, die unabhängig von etwaigen weiteren Indizes e ausgewählt ist aus der Gruppe der natürlichen Zahlen 1, 2, 3 und 4,

- jeder Index x bedeutet unabhängig von etwaigen weiteren Indizes x 0 oder1,

- jedes Y bedeutet in der Struktur $Q(Y_xZ_e)_b$ bei x = 1 ein Strukturelement, welches das polyalicyclische Strukturelement Q mit e Strukturelementen Z verbindet, wobei jedes Y unabhängig von etwaigen weiteren Strukturelementen Y gewählt ist,

(b2) optional ein, zwei oder mehr weitere radikalisch polymerisierbare Monomere aus der Gruppe bestehend aus Acrylaten und Methacrylaten, vorzugsweise der Gruppe der Methacrylate, wobei das oder die weiteren radikalisch polymerisierbaren Monomere keine Verbindungen (Monomere) der vorstehend definierten Struktur $Q(Y_xZ_e)_b$ sind,

(c) ein oder mehrere Initiatoren und/oder Katalysatoren, vorzugsweise in einer Menge von bis zu 1 Gew.-%, bezogen auf die Gesamtmasse des Kompositmaterials, sowie

(d) gegebenenfalls ein oder mehrere Additive

zur Anwendung in einem therapeutischen dentalen Verfahren als Versiegelungsmaterial zur Versiegelung von Fissuren und/oder Grübchen und/oder kariösen Läsionen.

Mit anderen Worten wird die Aufgabe gelöst durch ein Kompositmaterial bestehend aus oder umfassend:

(a) als Füllstoffkomponente eine Gesamtmenge an Füllstoffen im Bereich von 0,5 bis 60 Gew.-%, bezogen auf die Gesamtmasse des Kompositmaterials, wobei die Gesamtmenge an Füllstoffen eine Mischung von Füllstoffen ist umfassend

(a1) eine Gesamtmenge im Bereich von 0,5 bis 60 Gew.-% an nicht agglomerierten, organisch oberflächenmodifizierten Nanopartikeln mit einer mittleren Partikelgröße kleiner 200 nm
und

(a2) eine Gesamtmenge im Bereich von 0 bis 59,5 Gew.-% an Mikropartikeln mit einer mittleren Partikelgröße von 0,4 $\mu$m bis 10 $\mu$m
sowie

(a3) gegebenenfalls weiteren Füllstoffen,
wobei die Gewichtsprozentangaben für die Komponenten (a1) und (a2) jeweils auf die Gesamtmasse des Kompositmaterials bezogen sind,

(b) als Monomerenkomponente eine Gesamtmenge an polymerisierbaren Monomeren im Bereich von 24 (vorzugsweise 39) bis 98,5 Gew.-%, bezogen auf die Gesamtmasse des Kompositmaterials, wobei die Gesamtmenge an polymerisierbaren Monomeren umfasst

(b1) ein, zwei oder mehr Monomere ausgewählt aus der Gruppe bestehend aus Verbindungen (Monomere) der Struktur $Q(YZ_e)_b$, wobei gilt:

- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten $YZ_e$ substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sind,

- b ist eine natürliche Zahl ausgewählt aus der Gruppe der natürlichen Zahlen 1, 2, 3 und 4,

- jedes Z bedeutet ein Strukturelement, das unabhängig von etwaigen weiteren Strukturelementen Z ausgewählt ist aus der Gruppe bestehend aus

$$-O-(C=O)-CH=CH_2,\ -O-(C=O)-C(CH_3)=CH_2,$$

$$-(C=O)-CH=CH_2,\ -(C=O)-C(CH_3)=CH_2$$

$$-CH=CH_2,\ -C(CH_3)=CH_2 \text{ und } -O-CH=CH_2,$$

- jeder Index e ist eine natürliche Zahl, die unabhängig von etwaigen weiteren Indizes e ausgewählt ist aus der Gruppe der natürlichen Zahlen 1, 2, 3 und 4,

- jedes Y bedeutet ein Strukturelement, welches in der Struktur $Q(YZ_e)_b$ das polyalicyclische Strukturelement Q mit e Strukturelementen Z verbindet, wobei jedes Y unabhängig von etwaigen weiteren Strukturelementen Y gewählt ist oder entfällt,

(b2) optional ein, zwei oder mehr weitere radikalisch polymerisierbare Monomere aus der Gruppe bestehend aus Acrylaten und Methacrylaten, vorzugsweise der Gruppe der Methacrylate, wobei das oder die weiteren radikalisch polymerisierbaren Monomere keine Verbindungen (Monomere) der vorstehend definierten Struktur $Q(YZ_e)_b$ sind,

(c) ein oder mehrere Initiatoren und/oder Katalysatoren, vorzugsweise in einer Menge von bis zu 1 Gew.-%, bezogen

auf die Gesamtmasse des Kompositmaterials,
sowie

(d) gegebenenfalls ein oder mehrere Additive

zur Anwendung in einem therapeutischen dentalen Verfahren als Versiegelungsmaterial zur Versiegelung von Fissuren und/oder Grübchen und/oder kariösen Läsionen.

Sämtliche nachfolgenden Ausführungen betreffend die Verbindungen der Struktur $Q(YZ_e)_b$ (wobei jedes Y unabhängig von etwaigen weiteren Strukturelementen Y gewählt ist oder entfällt) und die im Zusammenhang mit diesen Verbindungen angegebenen bevorzugten bzw. besonders bevorzugten Ausgestaltungen der vorliegenden Erfindung gelten entsprechend für die Verbindungen der Struktur $Q(Y_xZ_e)_b$ (wobei jeder Index x unabhängig von etwaigen weiteren Indizes x 0 oder 1 bedeutet), und umgekehrt.

Eine solche Verbindung der Struktur $Q(Y_xZ_e)_b$ umfasst ein polyalicyclisches Strukturelement Q, das von einem entsprechenden polyalicyclischen Kohlenwasserstoff abgeleitet ist. Dies bedeutet im Rahmen des vorliegenden Textes, dass b Wasserstoffatome des Kohlenwasserstoffs durch Substituenten $Y_xZ_e$ ersetzt sind (wie oben beschrieben), und optional ein, zwei oder mehr der nicht durch Substituenten $Y_xZ_e$ substituierten Wasserstoffatome durch Alkylgruppen, Alkoxygruppen, Halogenatome oder Trifluormethylgruppen substituiert sind. Das polyalicyclische Strukturelement Q wird konstituiert durch Kohlenstoff-Ring-Atome. Kohlenstoffatome außerhalb der Ringe sind Bestandteil von Substituenten.

Unter (Meth)acryl ist im Rahmen des vorliegenden Textes sowohl Acryl als auch Methacryl zu verstehen.

Bei dem "polyalicyclischen" Strukturelement Q handelt es sich um einen bicyclischen, tricyclischen, tetracyclischen, pentacyclischen oder hexacyclischen Kohlenwasserstoffrest, wie oben definiert. Die Bezeichnungen "bicyclisch", "tricyclisch", tetracyclisch", "pentacyclisch" und "hexacyclisch" entsprechen dabei der IUPAC-Nomenklatur.

Q bedeutet mit anderen Worten ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei keines, eines, zwei oder mehr der nicht durch Substituenten $Y_XZ_e$ substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert ist bzw. sind.

Vorzugsweise bedeutet jedes Y ein Strukturelement, welches in der Struktur $Q(Y_xZ_e)_b$ mit x = 1 das polyalicyclische Strukturelement Q mit e Strukturelementen Z verbindet, wobei jedes Y unabhängig von etwaigen weiteren Strukturelementen Y gewählt ist.

Die erfindungsgemäß einzusetzenden bzw. anzuwendenden Kompositmaterialien, vorzugsweise in einer der nachfolgend als bevorzugt bzw. besonders bevorzugt gekennzeichneten Ausgestaltungen, eignen sich insbesondere zur Versiegelung von Fissuren, zur Versiegelung von Grübchen und zur Versiegelung kariöser Läsionen.

In einem weiteren Aspekt betrifft die Erfindung daher ein Kompositmaterial, vorzugsweise in einer der nachfolgend als bevorzugt bzw. besonders bevorzugt gekennzeichneten Ausgestaltungen, als bzw. zur Verwendung bzw. zur Anwendung als dentales Versiegelungsmaterial, vorzugsweise als Versiegelungsmaterial zur Versiegelung von Fissuren, zur Versiegelung von Grübchen oder zur Versiegelung kariöser Läsionen.

Es wurde gefunden, dass die erfindungsgemäß anzuwendenden Kompositmaterialien für die genannten Zwecke sehr gute dentale Versiegelungsmaterialien sind, die im Vergleich zu einem Versiegelungsmaterial aus dem Stand der Technik am getrockneten Zahnschmelz ein sehr viel besseres Anfließverhalten zeigen (insbesondere in Bezug auf den Kontaktwinkel am trockenen Zahnschmelz), sehr viel weniger Wasser aufnehmen und gute mechanische Werte aufweisen (insbesondere Biegefestigkeit und E-Modul).

Das erfindungsgemäß einzusetzende bzw. anzuwendende Kompositmaterial ist vorzugsweise lichthärtbar.

Die Summe der Zahlenwerte des Index b und des Index e beträgt vorzugsweise 3, 4, 5, 6, 7 oder 8.

Im nicht ausgehärteten Zustand zeichnet sich ein erfindungsgemäß einzusetzendes bzw. anzuwendendes Kompositmaterialdurch einen geringen Kontaktwinkel auf trockenem Zahnschmelz aus (vorzugsweise von weniger als 60°, bevorzugt weniger als 50°, besonders bevorzugt weniger als 40°, insbesondere bevorzugt weniger als 35°, gemessen mit einem Kontaktwinkelmessgerät der Firma Krüss (DSA 100)).

Ferner hat sich gezeigt, dass ein erfindungsgemäß einzusetzendes bzw. anzuwendendes Kompositmaterial im ausgehärteten Zustand eine sehr geringe Wasseraufnahme aufweist, vorzugsweise weniger als 15 $\mu g/mm^3$, vorzugsweise weniger als 13 $\mu g/mm^3$ und besonders bevorzugt weniger als 10 $\mu g/mm^3$. Die Wasseraufnahme wurde dabei analog zu ISO 4049 bestimmt.

Bevorzugt ist ein erfindungsgemäß einzusetzendes bzw. anzuwendendes Kompositmaterial, wobei die Füllstoffkomponente (a) umfasst

(a1) eine Gesamtmenge im Bereich von 0,5 bis 59,5 Gew.-% an nicht agglomerierten, organisch oberflächenmodifizierten Nanopartikeln mit einer mittleren Partikelgröße kleiner 60 nm und/oder

(a2) eine Gesamtmenge im Bereich von 0,5 bis 59,5 Gew.-% an Mikropartikeln mit einer mittleren Partikelgröße im Bereich von 0,4 µm bis 10 µm sowie

(a3) gegebenenfalls zusätzlichen Füllstoffen,

wobei die Gewichtsprozentangaben für die Komponenten (a1) und (a2) jeweils auf die Gesamtmasse des Kompositmaterials bezogen sind.

Bevorzugt ist ein erfindungsgemäß einzusetzendes bzw. anzuwendendes Kompositmaterial, wobei die Füllstoffkomponente (a)

(a3) eine Gesamtmenge in Bereich von 0 bis 15 Gew.-%, vorzugsweise 0 bis 10 Gew.-%, an zusätzlichen (weiteren) Füllstoffen umfasst,
wobei die Gewichtsprozentangabe für die Komponente (a3) auf die Gesamtmasse des Kompositmaterials bezogen ist.

Bevorzugt ist ein erfindungsgemäß einzusetzendes bzw. anzuwendendes Kompositmaterial, wobei die Monomerenkomponente (b)

(b1) ein, zwei oder mehr radikalisch polymerisierbare Monomere der Struktur $Q(Y_xZ_e)_b$ wie vorstehend definiert und

(b2) ein, zwei oder mehr zusätzliche radikalisch polymerisierbare Monomere aus der Gruppe bestehend aus Acrylaten und Methacrylaten, vorzugsweise der Gruppe der Methacrylate, wobei das oder die weiteren radikalisch polymerisierbaren Monomere keine Verbindungen (Monomere) der Struktur $Q(Y_xZ_e)_b$ wie vorstehend definiert sind, enthält,

wobei das Verhältnis der Gesamtmasse der Komponente (b1) zur Gesamtmasse der Komponente (b2) vorzugsweise im Bereich von 4 : 1 bis 1 : 3, bevorzugt im Bereich von 3 : 1 bis 1 : 2 liegt.

Ebenfalls bevorzugt ist ein erfindungsgemäß einzusetzendes bzw. anzuwendendes Kompositmaterial, umfassend oder bestehend aus

(a) als Füllstoffkomponente eine Gesamtmenge an Füllstoffen im Bereich von 0,5 bis 60 Gew.-%, bezogen auf die Gesamtmasse des Kompositmaterials, wobei die Gesamtmenge an Füllstoffen eine Mischung von Füllstoffen ist umfassend

(a1) eine Gesamtmenge im Bereich von 0,5 bis 60 Gew.-% an nicht agglomerierten, organisch oberflächenmodifizierten Nanopartikeln mit einer mittleren Partikelgröße kleiner 200 nm (vorzugsweise kleiner 60 nm) und

(a2) eine Gesamtmenge im Bereich von 0 bis 59,5 Gew.-% an Mikropartikeln mit einer mittleren Partikelgröße von 0,4 µm bis 10 µm sowie

(a3) gegebenenfalls zusätzlichen Füllstoffen,
wobei die Gewichtsprozentangaben für die Komponenten (a1) und (a2) jeweils auf die Gesamtmasse des Kompositmaterials bezogen sind,

(b) als Monomerenkomponente eine Gesamtmenge an polymerisierbaren Monomeren im Bereich von 24 (vorzugsweise 39) bis 89 Gew.-%, vorzugsweise 35 (besonders bevorzugt 44) bis 60 Gew.-%, bezogen auf die Gesamtmasse des Kompositmaterials, wobei die Gesamtmenge an polymerisierbaren Monomeren umfasst

(b1) ein, zwei oder mehr Monomere ausgewählt aus der Gruppe bestehend aus Verbindungen (Monomere) der Struktur $Q(Y_xZ_e)_b$, wobei gilt:

- Q bedeutet ein gesättigtes oder olefinisch ungesättigtes polyalicyclisches Strukturelement ausgewählt aus

der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten $Y_xZ_e$ substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sind,

- b ist eine natürliche Zahl ausgewählt aus der Gruppe der natürlichen Zahlen 1, 2, 3 und 4,

- jedes Z bedeutet ein Strukturelement, das unabhängig von etwaigen weiteren Strukturelementen Z ausgewählt ist aus der Gruppe bestehend aus

  $-O-(C=O)-CH=CH_2$, $-O-(C=O)-C(CH_3)=CH_2$,

  $-(C=O)-CH=CH_2$, $-(C=O)-C(CH_3)=CH_2$

  $-CH=CH_2$, $-C(CH_3)=CH_2$ und $-O-CH=CH_2$,

- jeder Index e ist eine natürliche Zahl, die unabhängig von etwaigen weiteren Indizes e ausgewählt ist aus der Gruppe der natürlichen Zahlen 1, 2, 3 und 4,

- jeder Index x bedeutet unabhängig von etwaigen weiteren Indizes x 0 oder 1,

- jedes Y bedeutet in der Struktur $Q(Y_xZ_e)_b$ bei x = 1 ein Strukturelement, welches das polyalicyclische Strukturelement Q mit e Strukturelementen Z verbindet, wobei jedes Y unabhängig von etwaigen weiteren Strukturelementen Y gewählt ist,

(b2) ein, zwei oder mehr weitere radikalisch polymerisierbare Monomere aus der Gruppe bestehend aus Acrylaten und Methacrylaten, vorzugsweise der Gruppe der Methacrylate, wobei das oder die weiteren radikalisch polymerisierbaren Monomere keine Verbindungen (Monomere) der vorstehend definierten Struktur $Q(Y_xZ_e)_b$ sind,
wobei das Verhältnis der Gesamtmasse der Komponente (b1) zur Gesamtmasse der Komponente (b2) im Bereich von 4 : 1 bis 1 : 3 liegt, bevorzugt im Bereich von 3 : 1 bis 1 : 2 liegt,

(c) ein oder mehrere Initiatoren und/oder Katalysatoren, vorzugsweise in einer Menge von bis zu 1 Gew.-%, bezogen auf die Gesamtmasse des Kompositmaterials,
sowie

(d) gegebenenfalls ein oder mehrere Additive

zur Anwendung in einem therapeutischen dentalen Verfahren als Versiegelungsmaterial zur Versiegelung von Fissuren und/oder Grübchen und/oder kariösen Läsionen.

Erfindungsgemäß einzusetzende bzw. anzuwendende Kompositmaterialien sind vorzugsweise so ausgestaltet, dass sie als Dentalmaterial, insbesondere zur Versiegelung von Fissuren, zur Versiegelung von Grübchen und zur Versiegelung kariöser Läsionen eingesetzt werden können. Entsprechende Verwendungen bzw. Anwendungen eines erfindungsgemäßen bzw. erfindungsgemäß einzusetzenden (anzuwendenden) Kompositmaterials sind bevorzugt.

Das erfindungsgemäß einzusetzende bzw. anzuwendende Kompositmaterial umfasst oder besteht aus verschiedenen Bestandteilen, für die das Folgende gilt:

<u>Bestandteil (a): Füllstoffkomponente</u>

Ein erfindungsgemäß einzusetzendes bzw. anzuwendendes Kompositmaterial enthält eine Gesamtmenge an Füllstoffen, wobei diese Gesamtmenge eine Mischung von Füllstoffen ist, umfassend

(a1) eine Gesamtmenge im Bereich von 0,5 bis 60 Gew.-% an nicht agglomerierten, organisch oberflächenmodifizierten Nanopartikeln mit einer mittleren Partikelgröße kleiner 200 nm
und

(a2) eine Gesamtmenge im Bereich von 0 bis 59,5 Gew.-% an Mikropartikeln mit einer mittleren Partikelgröße von

0,4 μm bis 10 μm
sowie

(a3) gegebenenfalls weiteren Füllstoffen.

In erfindungsgemäß bevorzugten Ausgestaltungen enthält ein erfindungsgemäß einzusetzendes bzw. anzuwendendes Kompositmaterial einen Anteil an Füllstoffkomponente (a) im Bereich von 1 bis 60 Gew.-%, bevorzugt 25 bis 60 Gew.-%, weiter bevorzugt im Bereich von 30 bis 60 Gew.-%, besonders bevorzugt im Bereich von 40 bis 60 Gew.-%.

Die mittlere Partikelgröße $d_{50}$ der erfindungsgemäß einzusetzenden bzw. anzuwendenden Füllstoffpartikel der Füllstoffkomponente (a) eines erfindungsgemäßen bzw. erfindungsgemäß anzuwendenden Kompositmaterials wird mittels Lichtstreuung (Laserbeugung) bestimmt, beispielsweise mit einem Partikelgrößenmessgerät Beckman Coulter LS 13320.

Die Füllstoffkomponente (a) umfasst einen Füllstoff (a1) in Form nicht agglomerierter, organisch oberflächenmodifizierter Nanopartikel mit einer mittleren Partikelgröße kleiner 200 nm.

Die Füllstoffkomponente (a) kann zusätzlich einen von der Komponente (a1) verschiedenen Füllstoff (a2) in Form von Mikropartikeln mit einer mittleren Partikelgröße im Bereich von 0,4 μm bis 10 μm umfassen.

Die Füllstoffkomponente (a) kann ferner zusätzlich weitere Füllstoffe als Komponente (a3) umfassen, muss jedoch keinen weiteren Füllstoff umfassen. Der Anteil der Komponente (a3) beträgt vorzugsweise höchstens 15 Gew.-%, bevorzugt höchstens 10 Gew.-%, weiter bevorzugt weniger als 5 Gew.-%, jeweils bezogen auf die Gesamtmasse des Kompositmaterials.

Die Füllstoffkomponente (a) umfasst in einer bevorzugten Ausgestaltung eine Mischung eines ersten Füllstoffs (a1) in Form nicht agglomerierter, organisch oberflächenmodifizierter Nanopartikel mit einer mittleren Partikelgröße kleiner 200 nm und eines zweiten Füllstoffs (a2) in Form von Mikropartikeln mit einer mittleren Partikelgröße im Bereich von 0,4 μm bis 10 μm.

Durch die Kombination von (a1) Nanopartikeln und (a2) Mikropartikeln in einem erfindungsgemäß bevorzugten Kompositmaterial wird eine vollständige und gleichmäßige Volumenfüllung des Kompositmaterials erzielt. Dadurch wird sowohl die Schrumpfung des Kompositmaterials beim Aushärten der Polymermatrix als auch die Empfindlichkeit des Kompositmaterials gegen Abrasion vermindert.

Eine bevorzugte Füllstoffkomponente (a) eines erfindungsgemäß einzusetzenden bzw. anzuwendenden Kompositmaterials besteht aus oder umfasst:

(a1) eine Gesamtmenge im Bereich von 10 bis 60 Gew.-% an nicht agglomerierten, organisch oberflächenmodifizierten Nanopartikeln mit einer mittleren Partikelgröße kleiner 200 nm, vorzugsweise eine Gesamtmenge im Bereich von 0,5 bis 59,5 Gew.-% an nicht agglomerierten, organisch oberflächenmodifizierten Nanopartikeln mit einer mittleren Partikelgröße kleiner 60 nm
und/oder (vorzugsweise und)

(a2) eine Gesamtmenge im Bereich von 0,5 bis 59,5 Gew.-% an Mikropartikeln mit einer mittleren Partikelgröße im Bereich von 0,4 μm bis 10 μm, vorzugsweise eine Gesamtmenge im Bereich von 20 bis 59,5 Gew.-% an Mikropartikeln mit einer mittleren Partikelgröße im Bereich von 0,4 μm bis 10 μm
sowie vorzugsweise

(a3) eine Gesamtmenge im Bereich von 0 bis 15 Gew.-% zusätzlichen Füllstoffe,
wobei die Gewichtsprozentangaben für die Komponenten (a1), (a2) und (a3) auf die Gesamtmasse des Kompositmaterials bezogen sind.

Weiter bevorzugte Füllstoffkomponenten (a) bestehen aus:

(a1) einer Gesamtmenge im Bereich von 20 bis 60 Gew.-%, vorzugsweise 30 bis 55 Gew.-%, an nicht agglomerierten, organisch oberflächenmodifizierten Nanopartikeln mit einer mittleren Partikelgröße kleiner 200 nm und/oder (vorzugsweise und)

(a2) einer Gesamtmenge im Bereich von 25 bis 59,5 Gew.-%, vorzugsweise 30 bis 55 Gew.-%, an Mikropartikeln mit einer mittleren Partikelgröße im Bereich von 0,4 μm bis 10 μm
sowie

(a3) einer Gesamtmenge im Bereich von 0 bis 10 Gew.-%, vorzugsweise 0 bis 5 Gew.-%, bevorzugt 1 bis 4 Gew.-% an zusätzlichen Füllstoffe,

wobei die Gewichtsprozentangaben für die Komponenten (a1), (a2) und (a3) auf die Gesamtmasse des Kompositmaterials bezogen sind.

In einem erfindungsgemäß einzusetzenden bzw. anzuwendenden Kompositmaterial enthaltend Mikropartikel der Komponente (a2), lässt sich die Füllstoffmenge noch weiter unter Beibehaltung der hervorragenden Anfließ- und Floweigenschaften durch Zugabe eines oder mehrerer nanoskaliger Füllstoffe (a1) erhöhen, wodurch sich die mechanischen Eigenschaften des erfindungsgemäß einzusetzenden bzw. anzuwendenden Kompositmaterials weiter verbessern lassen.

Dies gilt insbesondere für Kompositmaterialien, in denen Komponente (a2) eine oder mehrere Glaskeramiken enthält oder aus diesen besteht.

Komponente (a1): nicht agglomerierte, organisch oberflächenmodifizierte Nanopartikel

Innerhalb eines erfindungsgemäß einzusetzenden bzw. anzuwendenden Kompositmaterials besteht die Funktion der Nanopartikel u.a. darin, die Versiegelung zu verbessern (z.B. von Fissuren und Grübchen), und die Härte und Abrasionsbeständigkeit zu erhöhen.

Sofern ein erfindungsgemäß einzusetzendes bzw. anzuwendendes Kompositmaterial zusätzlich Mikropartikel der Komponente (a2) enthält, können die Nanopartikel die Zwischenräume zwischen den Mikropartikeln auffüllen, um so eine gleichmäßige Füllung des Kompositmaterials zu bewirken.

Unter Nanopartikeln werden in Zusammenhang mit der vorliegenden Erfindung Partikel mit einer mittleren Partikelgröße von weniger als 200 nm verstanden. Vorzugsweise ist die mittlere Partikelgröße kleiner als 100 nm und besonders bevorzugt kleiner 60 nm.

Der Anteil organisch oberflächenmodifizierter Nanopartikel mit einer mittleren Partikelgröße kleiner 200 nm ist vorzugsweise größer als 10 Gew.-% (d.h. > 10 Gew.-%), bevorzugt größer als 20 Gew.-% und besonders bevorzugt größer als 25 Gew.-%.

Bei transparenten erfindungsgemäß einzusetzenden bzw. anzuwendenden Kompositmaterialien ist der Anteil der Komponente (a1) vorzugsweise größer als 30 Gew.-%, bevorzugt größer als 35 Gew.-% und besonders bevorzugt größer als 40 Gew.-%.

In eigenen Untersuchungen hat sich gezeigt, dass bei einem Gehalt von 10 Gew.-% oder weniger an nicht agglomerierten, organisch oberflächenmodifizierten Nanopartikeln mit einer mittleren Partikelgröße kleiner 200 nm das Kompositmaterial im Einzelfall nicht mehr ausreichend abriebfest ist. Andererseits hat sich gezeigt, dass bei einem Gehalt von mehr als 60 Gew.-% an nicht agglomerierten, organisch oberflächenmodifizierten Nanopartikeln mit einer mittleren Partikelgröße kleiner 200 nm die Verarbeitbarkeit des Kompositmaterials nicht mehr ausreichend ist; wegen des hohen Feststoffgehalts wird dann seine Ruheviskosität zu hoch, das heißt, das Material wird im Ruhezustand gelartig.

In einer bevorzugten Ausgestaltung besitzt ein erfindungsgemäß anzuwendendes Kompositmaterial eine Viskosität von 100 Pas oder weniger, bevorzugt 50 Pas oder weniger, besonders bevorzugt 10 Pas oder weniger, bestimmt unter den Meßbedingungen, die weiter unten bei den "Meßmethoden" angegeben sind (zusammenfassend: 23 °C, 10 rad/s, 25 mm Platte/Platte-System).

In einer bevorzugten Ausgestaltung umfasst ein erfindungsgemäß einzusetzendes bzw. anzuwendendes Kompositmaterial einen hohen Anteil an Nanopartikeln der Komponente (a1), vorzugsweise in einer Menge von größer als 25 Gew.-%, bevorzugt im Bereich von 30 - 60 Gew.-%, besonders bevorzugt im Bereich von 35 - 55 Gew.-%, jeweils bezogen auf die Gesamtmasse des Kompositmaterials. Ein solches bevorzugtes erfindungsgemäß einzusetzendes bzw. anzuwendendes Kompositmaterial, insbesondere ein bevorzugtes erfindungsgemäß einzusetzendes bzw. anzuwendendes dentales Kompositmaterial, zeichnet sich durch eine geringe Abrasion aus (vorzugsweise weniger als 100 $\mu$m, bevorzugt weniger als 80 $\mu$m, weiter bevorzugt weniger als 70 $\mu$m, bestimmt nach der ACTA-Methode).

Die Materialien für die erfindungsgemäß einzusetzenden Nanopartikel sind vorzugsweise Oxide oder Mischoxide und bevorzugt ausgewählt aus der Gruppe bestehend aus Oxiden und Mischoxiden der Elemente Silizium, Titan, Yttrium, Strontium, Barium, Zirkonium, Hafnium, Niob, Tantal, Wolfram, Wismut, Molybdän, Zinn, Zink, Ytterbium, Lanthan, Cer, Aluminium und deren Mischungen. Die bevorzugten oxidischen Nanopartikel sind dabei wie dargelegt nicht agglomeriert.

In einer bevorzugten Ausgestaltung liegen die nanoskaligen Teilchen in nicht agglomerierter Form vor, beispielsweise dispergiert in einem Medium, vorzugsweise in monodisperser Form.

Um eine gute Einbindung der Nanopartikel in die Polymermatrix eines erfindungsgemäß einzusetzenden bzw. anzuwendenden Kompositmaterials zu ermöglichen, sind die Oberflächen der Nanopartikel (vorzugsweise der bevorzugten oxidischen Nanopartikel) organisch modifiziert, d.h. ihre Oberflächen weisen organische Strukturelemente auf. Beispielhaft genannt sei die Oberflächenbehandlung der Füllstoffe mit einem Silan. Als Haftvermittler eignet sich besonders das Methacryloxypropyltrimethoxysilan.

Wird als Füllstoffkomponente (a) ausschließlich ein nanoskaliger Füllstoff (a1) eingesetzt, so können transparente Versiegelungsmaterialien erhalten werden (siehe WO 01/30307 A1 und WO 2007/028159 A2). Es werden erfindungsgemäß dann sehr gute Werte zur Abrasion erzielt. Die erfindungsgemäß einzusetzenden bzw. anzuwendenden

transparenten dentalen Kompositmaterialien zeichnen sich gegenüber den Produkten aus dem Stand der Technik durch ein stark verbessertes Anfließverhalten am Zahnschmelz, insbesondere dem trockenen Zahnschmelz, sowie durch eine viel geringere Wasseraufnahme aus.

Daher umfasst in einer bevorzugten Ausführungsform die Füllstoffmischung (a) eines erfindungsgemäß einzusetzenden bzw. anzuwendenden Kompositmaterials ausschließlich (a1) nicht agglomerierte, organisch oberflächenmodifizierte Nanopartikel mit einer mittleren Partikelgröße kleiner 200 nm.

Komponente (a2): Mikropartikel mit einer mittleren Partikelgröße im Bereich von 0,4 μm bis 10 μm

Innerhalb eines erfindungsgemäß einzusetzenden bzw. anzuwendenden Kompositmaterials können Mikropartikel der Komponente (a2) eine weitgehende gleichmäßige Füllung des Volumens bewirken, wobei die verbleibenden Hohlräume zwischen den Mikropartikeln vorzugsweise durch die oben beschriebenen Nanopartikel (Komponente (a1)) zumindest teilweise gefüllt werden.

Unter Mikropartikeln werden in Zusammenhang mit der vorliegenden Erfindung Partikel mit einer mittleren Partikelgröße von 400 nm bis 10 μm verstanden. Vorzugsweise ist die mittlere Partikelgröße kleiner als 5 μm. In eigenen Untersuchungen hat sich gezeigt, dass die mit den Mikropartikeln bereits erreichbare Volumenfüllung des Kompositmaterials umso vollständiger und gleichmäßiger ist, je kleiner die Mikropartikel sind.

Die Mikropartikel der Komponente (a2) können eine monomodale oder polymodale, beispielsweise eine bimodale Partikelgrößenverteilung aufweisen. Mikropartikel mit einer bimodalen oder multimodalen Partikelgrößenverteilung sind erfindungsgemäß bevorzugt, da mit ihnen eine vollständigere Volumenfüllung erreichbar ist als bei allgemeiner Verwendung von Mikropartikeln mit monomodaler Partikelgrößenverteilung. Bei Vorliegen einer bi- oder multimodalen Partikelgrößenverteilung bewirken die Partikel der Fraktionen mit der größeren Partikelgröße eine grobe Ausfüllung des Volumens, während die Partikel der Fraktion mit der kleineren Partikelgröße soweit möglich die Hohlräume zwischen den Partikeln der Fraktionen mit der größeren Partikelgröße ausfüllen werden.

Bevorzugt wird somit in einem erfindungsgemäß einzusetzenden bzw. anzuwendenden Kompositmaterial eine Komponente (a2) eingesetzt, welche zwei oder mehr Fraktionen von Mikropartikeln enthält, wobei sich die mittleren Partikelgrößen der Fraktionen unterscheiden.

Vorzugsweise enthält Komponente (a2) zumindest zwei Mikropartikel-Fraktionen, wobei deren mittlere Partikelgrößen um mindestens 0,5 μm, bevorzugt um mindestens 0,7 μm weiter bevorzugt um mindestens 1 μm voneinander abweichen.

Die Mikropartikel verschiedener Fraktionen können aus dem gleichen oder aus verschiedenen Materialien bestehen; es können dabei auch mehrere Fraktionen von Mikropartikeln vorliegen, deren mittlere Partikelgröße annähernd gleich ist oder in einem bestimmten Bereich liegt, wobei die Materialien der Partikel sich zwischen den Fraktionen unterscheiden.

Bevorzugt umfasst ein erfindungsgemäß einzusetzendes bzw. anzuwendendes Kompositmaterial eine Komponente (a2), welche eine oder mehrere erste Mikropartikelfraktionen, die jeweils eine mittlere Partikelgröße im Bereich von 1 μm bis 10 μm, vorzugsweise 1 μm bis 5 μm, besitzen, und eine oder mehrere zweite Mikropartikelfraktionen, die jeweils eine mittlere Partikelgröße im Bereich von > 0,4 μm bis < 1 μm (d.h. größer als 0,4 μm, aber kleiner als 1 μm), vorzugsweise 0,5 μm bis 0,8 μm, besitzen.

Bevorzugt liegt das Verhältnis der Gesamtmasse der ersten Mikropartikelfraktionen zur Gesamtmasse der zweiten Mikropartikelfraktionen im Bereich von 1 : 3 bis 1 : 15, vorzugsweise im Bereich von 1 : 8 bis 1 : 13.

Bevorzugt liegt das Verhältnis der mittleren Korngröße der oder einer ersten Mikropartikelfraktion zur mittleren Korngröße der oder einer zweiten Mikropartikelfraktion der Komponente (a2) im Bereich von 1,5 : 1 bis 12 : 1, vorzugsweise im Bereich von 2 : 1 bis7:1.

In einem besonders bevorzugten erfindungsgemäß einzusetzenden bzw. anzuwendenden Kompositmaterial umfasst die Komponente (a2) eine oder mehrere erste Mikropartikelfraktionen, die jeweils eine mittlere Partikelgröße im Bereich von 1 μm bis 10 μm, vorzugsweise 1 μm bis 5 μm, besitzen, und eine oder mehrere zweite Mikropartikelfraktionen, die jeweils eine mittlere Partikelgröße im Bereich von > 0,4 μm bis < 1 μm, vorzugsweise 0,5 μm bis 0,8 μm, besitzen; wobei das Verhältnis der Gesamtmasse der ersten Mikropartikelfraktionen zur Gesamtmasse der zweiten Mikropartikelfraktionen im Bereich von 1 : 3 bis 1 : 15, vorzugsweise im Bereich von 1 : 8 bis 1 : 13 und/oder das Verhältnis der mittleren Korngröße der oder einer ersten Mikropartikelfraktion zur mittleren Korngröße der oder einer zweiten Mikropartikelfraktion der Komponente (a2) im Bereich von 1,5 : 1 bis 12 : 1, vorzugsweise 2 : 1 bis 7 : 1 liegt.

Die Basismaterialien für die erfindungsgemäß in oberflächenmodifizierter Form einzusetzenden Mikropartikel sind bevorzugt ausgewählt aus der Gruppe bestehend aus Quarz-Glaskeramik oder Glaspulver (insbesondere Dentalglaspulver), Barium- oder Strontiumgläser, Fluoridionen abgebende Gläser, Oxide von Aluminium oder Silicium, Zeolithe, Apatit, Zirkonsilikate, schwerlösliche Metallsalze wie Bariumsulfat oder Calciumfluorid sowie röntgenopake Füllstoffe wie Ytterbiumfluorid.

Zur besseren Einbindung in die Polymermatrix eines erfindungsgemäß einzusetzenden bzw. anzuwendenden

Kompositmaterials sind die Mikropartikel vorzugsweise organisch oberflächenmodifiziert. Beispielhaft genannt sei die Oberflächenbehandlung der Füllstoffe mit einem Silan, die zu silanisierten Mikropartikeln führt. Zur Oberflächenbehandlung (als Haftvermittler) eignet sich besonders Methacryloxypropyltrimethoxysilan.

In einem besonders bevorzugten erfindungsgemäß einzusetzenden Kompositmaterial wird zumindest ein Teil der Mikropartikel der Komponente (a2) durch organisch oberflächenmodifizierte Partikel, vorzugsweise silanisierte Partikel gebildet und/oder es wird zumindest ein Teil der Mikropartikel der Komponente (a2) durch Dentalglas-Partikel gebildet; vorzugsweise sind zumindest ein Teil der Mikropartikel der Komponente (a2) organisch oberflächenmodifizierte Dentalglas-Partikel, vorzugsweise silanisierte Dentalglas-Partikel.

Bevorzugt zeichnet sich in diesen Fällen Komponente (a2) durch eine bi- oder multimodale Partikelgrößenverteilung aus, insbesondere eine bi- oder multimodale Partikelgrößenverteilung mit den oben beschriebenen bevorzugten Merkmalen.

Komponente (a3) - Zusätzliche (weitere) Füllstoffe

Die Füllstoffkomponente (a) kann ferner einen zusätzlichen Füllstoff (a3) umfassen. Dieser Füllstoff (a3) umfasst keine nicht-agglomerierten, organisch oberflächenmodifizierten Nanopartikel mit einer mittleren Partikelgröße kleiner 200 nm (die zur Komponente (a1) zu zählen sind) und umfasst auch keine Mikropartikel mit einer mittleren Partikelgröße von 0,4 $\mu$m bis 10 $\mu$m (die zur Komponente (a2) zu zählen sind).Vorzugsweise umfasst die Füllstoffkomponente (a) zusätzlich zu den Komponenten (a1) und/oder (a2) zusätzlich weitere Füllstoffe als Komponente (a3).

So können z.B. verstärkend wirkende Füllstoff-Materialien, wie Glasfasern, Polyamid- oder Kohlenstofffasern eingesetzt werden. Ein erfindungsgemäß einzusetzendes bzw. anzuwendendes Kompositmaterial kann auch feinteilige Splitter oder Perlpolymerisate enthalten, wobei die Perlpolymerisate Homo- oder Copolymere organischer härtbarer Monomerer sein können.

Bestandteil (b): Monomerenkomponente

Innerhalb eines erfindungsgemäß einzusetzenden bzw. anzuwendenden Kompositmaterials besteht die Funktion der Monomerenkomponente (b) darin, eine Matrix zu bilden, in welche die oben genannten Füllstoffe (a) eingebunden sind. Diese Matrix wird gebildet durch Polymerisation, insbesondere radikalische Polymerisation, von (b1) ein, zwei oder mehr Monomere der Struktur $Q(Y_xZ_e)_b$, gegebenenfalls zusammen mit Komponente (b2) einem, zwei oder mehr weiteren radikalisch polymerisierbaren Monomeren aus der Gruppe bestehend aus Acrylaten und Methacrylaten, wobei das oder die weiteren radikalisch polymerisierbaren Monomere keine Verbindungen (Monomere) der Struktur $Q(Y_xZ_e)_b$ wie oben definiert sind.

Vorzugsweise ist die Monomerenkomponente (b) eine Monomerenmischung, die sowohl Komponente (b1) als auch Komponente (b2) umfasst oder aus dieser besteht.

Bevorzugte erfindungsgemäß einzusetzende bzw. anzuwendende Kompositmaterialien umfassen Monomerenkomponente (b) in einer Menge im Bereich von 24 (vorzugsweise 39) bis 89 Gew.-%, vorzugsweise 30 (bevorzugt 42) bis 65 Gew.-%, bevorzugt im Bereich von 35 (vorzugsweise 44) bis 60 Gew.-%, bezogen auf die Gesamtmasse des Kompositmaterials.

Besonders gute Ergebnisse im Sinne der vorliegenden Erfindung werden erzielt, wenn das Gewichtsverhältnis der Gesamtmenge an Monomerkomponente (b1) zu der Gesamtmenge an Monomerkomponente (b2) im Bereich 4 : 1 bis 1 : 3 liegt, vorzugsweise im Bereich 3 : 1 bis 1 : 2, bevorzugt im Bereich 2 : 1 bis 2 : 3, besonders bevorzugt im Bereich 3 : 2 bis 2 : 3 und ganz besonders bevorzugt im Bereich 4 : 3 bis 3 : 4.

Dies gilt bevorzugt für Monomere der Komponente (b1), in denen das polyalicyclische Strukturelement Q einen Tricyclo[5.2.1.0$^{2,6}$]decan-Rest bedeutet, insbesondere für Monomere der Komponente (b1), die Umsetzungsprodukte des Bis(hydroxymethyl)tricyclo[5.2.1.0$^{2,6}$]decans sind.

Das polyalicyclische Strukturelement Q der Komponente (b1) sorgt dabei für ein sterisch starres und hydrophobes Rückgrat, und die weiteren Monomere der Komponente (b2) sichern eine ausreichende Vernetzung, insbesondere zu den oberflächenmodifizierten Füllstoffen. Eigene Untersuchungen haben gezeigt, dass Pasten mit einem geringeren Anteil an Komponente (b2) bzw. ohne Komponente (b2) eine erhöhte Abrasion aufweisen, wohingegen Pasten mit einem geringeren Anteil an Komponente (b1) bzw. ohne Komponente (b1) eine erhöhte Wasseraufnahme und ungünstigere mechanische Eigenschaften (insbesondere ein verringertes E-Modul) zeigen.

Komponente (b1): ein, zwei oder mehr Monomere der Struktur $Q(Y_xZ_e)_b$ mit mindestens einem polyalicyclischen Strukturelement

Komponente (b1) bilden ein, zwei oder mehr Monomere der oben definierten Struktur $Q(Y_xZ_e)_b$, wobei Z vorzugsweise ein Strukturelement bedeutet, das unabhängig von etwaigen weiteren Strukturelementen Z ausgewählt ist

aus der Gruppe bestehend aus - O-(C=O)-CH=$CH_2$, -O-(C=O)-C($CH_3$)=$CH_2$, -(C=O)-CH=$CH_2$ oder -(C=O)-C($CH_3$)=$CH_2$. Bevorzugt sind Verbindungen der Struktur $Q(Y_xZ_e)_b$, wobei Z ausgewählt ist aus der Gruppe bestehend aus -O-(C=O)-CH=$CH_2$, -O-(C=O)-C($CH_3$)=$CH_2$, d.h. solche Verbindungen der Struktur $Q(Y_xZ_e)_b$, die ein, zwei oder mehr Acrylat- und/oder Methacrylatgruppen aufweisen, vorzugsweise zwei oder mehr Acrylat- und/oder Methacrylatgruppen.

Die mit den erfindungsgemäß zu verwendenden Monomeren der Komponente (b1) erhältlichen Polymere und Kompositmaterialien weisen eine ausgeprägte Hydrophobie auf, die sich u.a. in einer sehr geringen Wasseraufnahme der Polymere und Kompositmaterialien zeigt. Daneben zeichnen sich die unter Verwendung der erfindungsgemäß zu verwendenden Monomere der Komponente (b1) erhältlichen Polymere durch eine hohe mechanische Stabilität aus, die sich u.a. in einer hohen Biegefestigkeit der Polymere zeigt. Die erfindungsgemäß zu verwendenden Monomere der Komponente (b1), insbesondere gemäß den besonders bevorzugten Ausgestaltungen und Ausführungsformen, lassen sich zu Polymeren verarbeiten, die sowohl eine geringe Wasseraufnahme als auch eine hohe Biegefestigkeit aufweisen.

Die Monomere der Komponente (b1) sind mit den weiteren Monomeren der Komponente (b2) copolymerisierbar, wobei die ausgehärteten Polymere bzw. Formstoffe einen geringen Schrumpf, eine gute Haftung auf verschiedenen Untergründen, eine hohe Hydrolysebeständigkeit, eine geringe Wasseraufnahme und eine hohe mechanische Festigkeit aufweisen. Die genannten Eigenschaften sind insbesondere im Bereich der Dentaltechnik wichtig.

Insbesondere die bevorzugten und besonders bevorzugten erfindungsgemäß zu verwendenden Verbindungen der Komponente (b1) ermöglichen einen hohen Vernetzungsgrad und sind ferner vorzugsweise radikalisch vernetzbar. Wegen ihrer hochfunktionalisierten Struktur weisen sie eine hohe Vernetzungs- und Polymerisationswahrscheinlichkeit auf.

Bevorzugte erfindungsgemäß einzusetzende Verbindungen der Komponente (b1) sind solche, wobei Q ein polyalicyclisches Strukturelement bedeutet, vorzugsweise ein gesättigtes polyalicyclisches Strukturelement, das ausgewählt ist aus der Gruppe bestehend aus bicyclischen oder tricyclischen Kohlenwasserstoffresten, wobei vorzugsweise keines der nicht durch Substituenten $Y_xZ_e$ substituierten Wasserstoffatome dieses polyalicyclischen Strukturelements Q substituiert ist.

Besonders bevorzugt sind erfindungsgemäß einzusetzende Monomere $Q(Y_xZ_e)_b$, deren polyalicyclisches Strukturelement Q sich von einem der folgenden tricyclischen Kohlenwasserstoffe ableitet: Tricyclo[5.2.1.$0^{2,6}$]decan (TCD), Tricyclo[5.2.1.$0^{2,6}$]dec-3-en oder Tricyclo[3.3.1.$1^{3,7}$]decan (Adamantan), d.h. bevorzugt sind erfindungsgemäße Verbindungen, die ein TCD - Gerüst, ein Tricyclo[5.2.1.$0^{2,6}$]dec-3-en - Gerüst oder ein Adamantan-Gerüst aufweisen.

Bei den genannten besonders bevorzugten erfindungsgemäß einzusetzenden Verbindungen, in denen das Strukturelement Q einen Tricyclo[5.2.1.$0^{2,6}$]decan-Rest, einen Tricyclo[5.2.1.$0^{2,6}$]dec-3-en-Rest, einen Tricyclo[3.3.1.$1^{3,7}$]decan-Rest oder einen Bicyclo[2.2.1]heptan-Rest bedeutet, handelt es sich vorzugsweise um solche mit einem Tricyclo[5.2.1.$0^{2,6}$]decan-Gerüst, einem Tricyclo[5.2.1.$0^{2,6}$]dec-3-en-Gerüst, einem Tricyclo[3.3.1.$1^{3,7}$]decan-Gerüst bzw. einem Bicyclo[2.2.1]heptan-Gerüst, bei dem jeweils keines der nicht durch Substituenten $Y_xZ_e$ substituierten Wasserstoffatome dieses polyalicyclischen Strukturelements Q substituiert ist.

Besonders bevorzugte erfindungsgemäß einzusetzende Verbindungen $Q(Y_xZ_e)_b$ der Komponente (b1) sind solche, wobei das Strukturelement Q einen Tricyclo[5.2.1.$0^{2,6}$]decan-Rest, einen Tricyclo[5.2.1.$0^{2,6}$]dec-3-en-Rest, einen Tricyclo[3.3.1.$1^{3,7}$]decan-Rest oder einen Bicyclo[2.2.1]heptan-Rest bedeutet, ganz besonders bevorzugt bedeutet das Strukturelement Q einen Tricyclo[5.2.1.$0^{2,6}$]decan-Rest, einen Tricyclo[5.2.1.$0^{2,6}$]dec-3-en-Rest.

Besonders bevorzugte erfindungsgemäß einzusetzende Verbindungen $Q(Y_xZ_e)_b$ der Komponente (b1) sind solche, in denen das Strukturelement Q ein Tricyclo[5.2.1.$0^{2,6}$]-decan- oder Tricyclo[5.2.1.$0^{2,6}$]-decen-Strukturelement ist und Z vorzugsweise ausgewählt ist aus der Gruppe bestehend aus -O-(C=O)-CH=$CH_2$ und -O-(C=O)-C($CH_3$)=$CH_2$, wobei wiederum die Gruppe Z besonders bevorzugt -O-(C=O)-C($CH_3$)=$CH_2$ ist.

Besonders bevorzugte erfindungsgemäße oder erfindungsgemäß zu verwendende bzw. anzuwendende Kompositmaterialien enthalten als Komponente (b1) ein, zwei oder mehr Verbindungen der Struktur $Q(Y_xZ_e)_b$, die jeweils ein Tricyclo[5.2.1.$0^{2,6}$]-decan- oder Tricyclo[5.2.1.$0^{2,6}$]-decen-Strukturelement aufweisen und Z vorzugsweise ausgewählt ist aus der Gruppe bestehend aus -O-(C=O)-CH=$CH_2$ und -O-(C=O)-C($CH_3$)=$CH_2$, wobei wiederum die Gruppe Z besonders bevorzugt -O-(C=O)-C($CH_3$)=$CH_2$ ist.

Bevorzugt eingesetzt werden Methacrylsäure- oder Acrylsäureester mit einem Tricyclo[5.2.1.$0^{2,6}$]-decan- oder Tricyclo[5.2.1.$0^{2,6}$]-decen-Strukturelement, ausgewählt aus der Gruppe bestehend aus

- 8,9-Bis(acryloxymethyl)tricyclo[5.2.1.$0^{2,6}$]decan
- 8,9-Bis(methacryloyloxymethyl)tricyclo[5.2.1.$0^{2,6}$]decan
- 8,9-[Bis(2-vinyloxyethyl)oxymethyl]tricyclo[5.2.1.$0^{2,6}$]dec-3-en
- 8,9-[Bis(2-vinyloxyethyl)oxymethyl]tricyclo[5.2.1.$0^{2,6}$]decan

- 8-Hydroxymethyl-9-(2-vinyloxyethyl)oxymethyl]tricyclo[5.2.1.$0^{2,6}$]dec-3-en
- 9-Hydroxymethyl-8-(2-vinyloxyethyl)oxymethyl]tricyclo[5.2.1.$0^{2,6}$]dec-3-en
- 8,9-Bis(acryloyloxymethyl)tricyclo[5.2.1.$0^{2,6}$]dec-3-en
- 8,9-Bis(methacryloyloxymethyl)tricyclo[5.2.1.$0^{2,6}$]dec-3-en
- Diacrylsäure- oder Dimethacrylsäureester von Verbindungen ausgewählt aus der Gruppe bestehend aus:
  - 3,8-Dihydroxymethyl- tricyclo[5.2.1.$0^{2,6}$]decan
  - 3,9-Dihydroxymethyltricyclo- [5.2.1.$0^{2,6}$]decan
  - 4,8-Dihydroxymethyltricyclo[5.2.1.$0^{2,6}$]decan
  - 3,8-Dihydroxytricyclo[5.2.1.$0^{2,6}$]decan
  - 3,9-Dihydroxytricyclo- [5.2.1.$0^{2,6}$]decan
  - 4,8-Dihydroxytricyclo[5.2.1.$0^{2,6}$]decan
- Methacrylsäure- oder Acrylsäureester von Verbindungen aus der Gruppe bestehend aus:
  - Poly(hydroxymethyl-tricyclo[5.2.1.$0^{2,6}$]decanyl-siloxanen
  - oxyalkyliertes Bishydroxymethyltricyclo[5.2.1.$0^{2,6}$]decan
  - oxyalkyliertes Bishydroxytricyclo[5.2.1.$0^{2,6}$]decan
- Urethan- oder Harnstoffgruppen enthaltende Methacrylsäure- oder Acrylsäureester von Verbindungen ausgewählt aus der Gruppe bestehend aus:
  - 3,8-Dihydroxymethyl- tricyclo[5.2.1.$0^{2,6}$]decan
  - 4,8-Dihydroxymethyl- tricyclo[5.2.1.$0^{2,6}$]decan
  - 3,9-Dihydroxymethyl- tricyclo[5.2.1.$0^{2,6}$]decan
  - 4,9-Dihydroxymethyl- tricyclo[5.2.1.$0^{2,6}$]decan

Hierbei kann in den genannten Verbindungen Wasserstoff am Tricyclo[5.2.1.$0^{2,6}$]-decan- oder Tricyclo[5.2.1.$0^{2,6}$]-decen-Rest durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sein.

Viele der vorstehend aufgelisteten radikalisch polymerisierbaren Methacrylsäure- oder Acrylsäureester mit einem TCD-Strukturelement sind aus dem Stand der Technik bekannt.

In eigenen Untersuchungen hat sich gezeigt, dass mit den oben genannten Monomeren $Q(Y_xZ_e)_b$ der Komponente (b1) mit einem Tricyclo[5.2.1.$0^{2,6}$]-decan-Strukturelement der Komponente (b1) Kompositmaterialien mit geringem Kontaktwinkel auf trockenem Zahnschmelz (vorzugsweise weniger als 40°, bevorzugt weniger als 30°) oder geringer Abrasion (vorzugsweise weniger als 100 $\mu$m, bevorzugt weniger als 80 $\mu$m, weiter bevorzugt weniger als 70 $\mu$m, bestimmt nach der ACTA-Methode) erhältlich sind.

In eigenen Untersuchungen hat sich ferner gezeigt, dass mit den oben genannten Monomeren $Q(Y_xZ_e)_b$ der Komponente (b1) mit einem Tricyclo[5.2.1.$0^{2,6}$]-decan-Strukturelement - im ausgehärteten Zustand - Kompositmaterialien mit geringer Wasseraufnahme (vorzugsweise weniger als 13 $\mu g/mm^3$, bevorzugt weniger als 10 $\mu g/mm^3$) erhältlich sind.

Y ist vorzugsweise ein Strukturelement, welches in der Struktur $Q(Y_xZ_e)_b$ das polyalicyclische Strukturelement Q mit e Strukturelementen Z verknüpft und ein Strukturelement enthält oder aus diesem besteht, das ausgewählt ist aus der Gruppe bestehend aus

$-CH_2-$ , 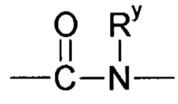 und 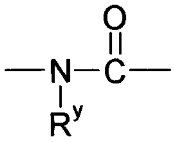

wobei $R^y$ einen sonstigen Rest der Verbindung bedeutet und wobei die jeweils im Formelbild links angeordnete Bindung dem Strukturelement Q und die rechts angeordnete Bindung dem Strukturelement Z näher ist.

Der sonstige Rest $R^y$ einer erfindungsgemäßen bzw. erfindungsgemäß einzusetzenden Verbindung der Struktur $Q(Y_xZ_e)_b$ ist vorzugsweise ausgewählt aus der Gruppe bestehend aus Wasserstoff, linearen, verzweigten oder Ringe umfassenden Strukturelementen mit 1 bis 50 C-Atomen und 0 bis 12 Heteroatomen, wobei die gegebenenfalls vorhandenen Heteroatome vorzugsweise gewählt sind aus der Gruppe bestehend aus N und O.

Der sonstige Rest $R^y$ ist dabei bevorzugt ausgewählt aus der Gruppe bestehend aus Wasserstoff, linearen, verzweigten oder Ringe umfassenden Strukturelementen mit 1 bis 40 C-Atomen und 0 bis 10 Heteroatomen, wobei die gegebenenfalls vorhandenen Heteroatome vorzugsweise gewählt sind aus der Gruppe bestehend aus N und O.

Der sonstige Rest $R^y$ ist dabei besonders bevorzugt ausgewählt aus der Gruppe bestehend aus Wasserstoff, linearen, verzweigten oder Ringe umfassenden Strukturelementen mit 1 bis 35 C-Atomen und 1 bis 10 Heteroatomen, wobei die gegebenenfalls vorhandenen Heteroatome vorzugsweise gewählt sind aus der Gruppe bestehend aus N und O.

Y ist dabei bevorzugt ein Strukturelement, welches ein Strukturelement enthält oder aus diesem besteht, ausgewählt aus der Gruppe bestehend aus

$-CH_2-$ 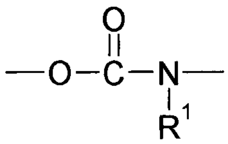 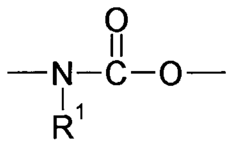 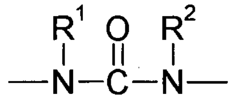

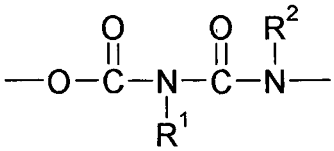 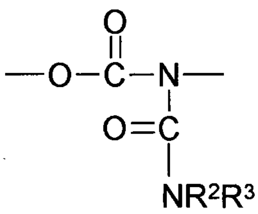 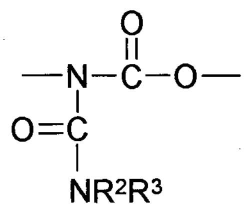

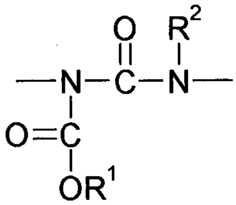 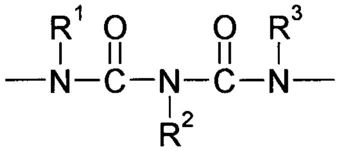 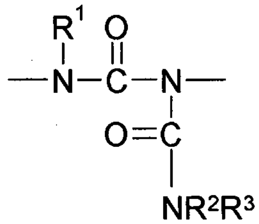

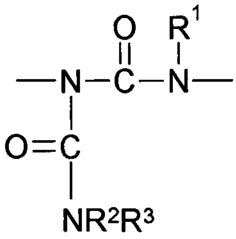

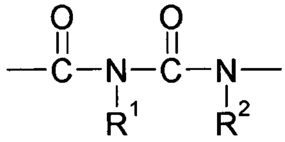

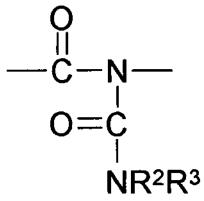

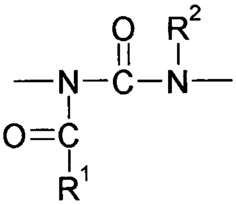

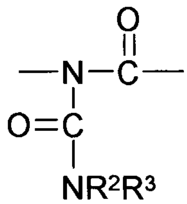

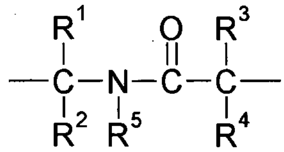

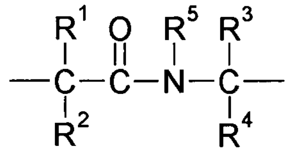

wobei $R^1$, $R^2$, $R^3$, $R^4$ und $R^5$ sonstige Reste der Verbindung bedeuten, wobei die jeweils im Formelbild links angeordnete Bindung dem Strukturelement Q und die rechts angeordnete Bindung dem Strukturelement Z näher ist.

Die oben genannten sonstigen Reste $R^1$, $R^2$, $R^3$, $R^4$ bzw. $R^5$ einer erfindungsgemäßen bzw. erfindungsgemäß einzusetzenden Verbindung der Struktur $Q(Y_xZ_e)_b$ sind, jeweils unabhängig voneinander, vorzugsweise ausgewählt aus der Gruppe bestehend aus Wasserstoff, linearen, verzweigten oder Ringe umfassenden Strukturelementen mit 1 bis 30 C-Atomen und 0 bis 10 Heteroatomen, wobei die gegebenenfalls vorhandenen Heteroatome vorzugsweise gewählt sind aus der Gruppe bestehend aus N und O.

Die sonstigen Reste $R^1$, $R^2$, $R^3$, $R^4$ bzw. $R^5$ sind dabei, jeweils unabhängig voneinander, bevorzugt ausgewählt aus der Gruppe bestehend aus Wasserstoff, linearen, verzweigten oder Ringe umfassenden Strukturelementen mit 1 bis 25 C-Atomen und 0 bis 8 Heteroatomen, wobei die gegebenenfalls vorhandenen Heteroatome vorzugsweise ausgewählt sind aus der Gruppe bestehend aus N und O.

Die sonstigen Reste $R^1$, $R^2$, $R^3$, $R^4$ bzw. $R^5$ sind dabei, jeweils unabhängig voneinander, besonders bevorzugt ausgewählt aus der Gruppe bestehend aus Wasserstoff, linearen, verzweigten oder Ringe umfassenden Strukturelementen mit 1 bis 20 C-Atomen und 0 bis 5 Heteroatomen, wobei die gegebenenfalls vorhandenen Heteroatome ausgewählt sind aus der Gruppe bestehend aus N und O.

In mit vergleichsweise geringem Aufwand synthetisierbaren erfindungsgemäßen bzw. erfindungsgemäß einzusetzenden Verbindungen der Struktur $Q(Y_xZ_e)_b$ ist Y ein Strukturelement, welches ein Strukturelement enthält oder aus diesem besteht, das ausgewählt ist aus der Gruppe bestehend aus

$-CH_2-$

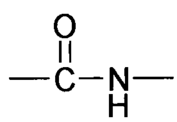

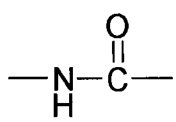

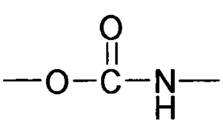

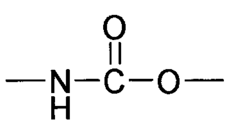

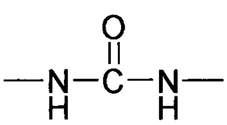

wobei die jeweils im Formelbild links angeordnete Bindung dem Strukturelement Q und die rechts angeordnete Bindung dem Strukturelement Z näher ist.

Die erfindungsgemäßen bzw. erfindungsgemäß einzusetzenden Verbindungen der Struktur $Q(Y_xZ_e)_b$ können gemäß dem Fachmann bekannten Herstellungsverfahren erhalten werden.

Erfindungsgemäße bzw. erfindungsgemäß einzusetzende Verbindungen der Struktur $Q(Y_xZ_e)_b$ mit einem Amid-Strukturelement können beispielsweise durch Umsetzung von (i) einer Edukt-Verbindung mit einer Isocyanatgruppe und (ii) einer Edukt-Verbindung mit einer Carbonsäuregruppe erhalten werden.

Erfindungsgemäße bzw. erfindungsgemäß einzusetzende Verbindungen der Struktur $Q(Y_xZ_e)_b$ mit einem Urethan-Strukturelement können beispielsweise durch Umsetzung von (i) einer Edukt-Verbindung mit einer Isocyanatgruppe und einer Edukt-Verbindung mit einer Alkoholgruppe erhalten werden.

Erfindungsgemäße bzw. erfindungsgemäß einzusetzende Verbindungen der Struktur $Q(Y_xZ_e)_b$ mit einem Harnstoff-Strukturelement können beispielsweise durch Umsetzung von (i) einer Edukt-Verbindung mit einer Isocyanatgruppe und (ii) einer Edukt-Verbindung mit einer Aminogruppe erhalten werden.

Erfindungsgemäße bzw. erfindungsgemäß einzusetzende Verbindungen der Struktur $Q(Y_xZ_e)_b$ mit einem Allophanat-Strukturelement können beispielsweise durch Umsetzung von (i) einer Edukt-Verbindung mit einer Urethangruppe und (ii) einer Edukt-Verbindung mit einer Isocyanatgruppe erhalten werden.

Erfindungsgemäße bzw. erfindungsgemäß einzusetzende Verbindungen der Struktur $Q(Y_xZ_e)_b$ mit einem Biuret-Strukturelement können beispielsweise durch Umsetzung von (i) einer Edukt-Verbindung mit einer Harnstoffgruppe und (ii) einer Edukt-Verbindung mit einer Isocyanatgruppe erhalten werden.

Erfindungsgemäße bzw. erfindungsgemäß einzusetzende Verbindungen der Struktur $Q(Y_xZ_e)_b$ mit einem *N*-Acylharnstoff-Strukturelement können beispielsweise durch Umsetzung von (i) einer Edukt- Verbindung mit einer Amidgruppe und (ii) einer Edukt-Verbindung mit einer Isocyanatgruppe erhalten werden.

In einer bevorzugten Ausgestaltung eines erfindungsgemäß anzuwendenden Kompositmaterials ist Komponente (b1) so gewählt, dass diese umfasst oder besteht aus Bis(methacrylolyoxymethyl)tricyclo[5.2.1.$0^{2,6}$]decan und/oder Bis(acrylolyoxymethyl)tricyclo[5.2.1.$0^{2,6}$]decan.

In erfindungsgemäß anzuwendendem Kompositmaterial sind die Methacrylsäureester wegen ihrer höheren biologischen Verträglichkeit gegenüber den entsprechenden Acrylsäureestern bevorzugt, d.h. dass Z in Verbindungen der Struktur $Q(Y_xZ_e)_b$ bevorzugt $-O-(C=O)-C(CH_3)=CH_2$ bedeutet.

Bevorzugte Verbindungen (Monomere) der Struktur $Q(YZ_e)_b$, der Komponente (b1) sind solche mit ein, zwei, drei, vier oder mehr funktionellen Gruppen ausgewählt aus der Gruppe bestehend aus Urethan, Harnstoff, N-Acylharnstoff, Allophanat, Biuret und Amid, wobei die Amidfunktion nicht unmittelbar mit einem N-Atom, einem O-Atom oder einer Carbonylgruppe verknüpft ist.

Auf dem Gebiet der Dentaltechnik besteht ein ständiger Bedarf an weiteren, vorzugsweise radikalisch, polymerisierbaren Monomeren, mit denen sich dentale Materialien mit bestimmten Eigenschaften herstellen lassen.

Unter (Meth)acryl ist im Rahmen des vorliegenden Textes sowohl Acryl als auch Methacryl zu verstehen.

EP 1 238 993 beschreibt ein Verfahren zur Herstellung von Acylharnstoff-Gruppen enthaltenden Polyisocyanaten sowie Gemische hiervon sowie deren Verwendung als Ausgangskomponente bei der Herstellung von Polyurethan-Kunststoffen.

EP 0 209 700 A2 und DE 35 22 005 beschreiben (Meth)acrylsäure-Derivate bestimmter Tricyclodecane mit zweibindigen Brückengliedern aus der Gruppe der Urethane bzw. Harnstoffe, welche im Dentalbereich eingesetzt werden können.

EP 0 000 194 A1 (entsprechend US 4,160,080) beschreibt Polyisocyanate, die Allophanatgruppen enthalten. Diese Allophanatpolyisocyanate können zur Herstellung von Polyurethan-Schaumstoffen, Elastomeren, Duromeren, Beschichtungen, Verklebungen und Lackierungen eingesetzt werden.

EP 0 682 012 B1 betrifft ein Verfahren zur Herstellung von hellfarbigen lichtechten Allophanatgruppen aufweisenden (cyclo)aliphatischen Polyisocyanaten, durch Umsetzung von Urethangruppen aufweisenden organischen Verbindungen mit organischen Polyisocyanaten mit (cyclo)aliphatisch gebundenen Isocyanatgruppen in Gegenwart von Zinn(II)-salzen. Die in EP 0 682 012 B1 beschriebenen Polyisocyanate können als Aufbaukomponente bei der Herstellung von Polyurethankunststoffen verwendet werden.

EP 1 727 846 B1 offenbart ein Verfahren zur Herstellung von Allophanatgruppen enthaltenden Bindemitteln, die unter Einwirkung aktinischer Strahlung mit ethylenisch ungesättigten Verbindungen unter Polymerisation reagierende Gruppen und gegebenenfalls NCO-reaktive Gruppen aufweisen.

EP 0 712 840 B1 betrifft ein Verfahren zur Herstellung von bestimmten Allophanatgruppen aufweisenden Polyisocyanaten durch Umsetzung unter Allophanatbildung von Urethangruppen aufweisenden Verbindungen. Die Verbindungen gemäß EP 0 712 840 B1 können als Bindemittel oder Bindemittelkomponente in Beschichtungsmitteln verwendet werden.

EP 0 867 457 B1 offenbart ein ethylenisch ungesättigtes Polyurethan, das im Wesentlichen frei von Isocyanatgruppen ist, welches das Reaktionsprodukt eines ethylenisch ungesättigten Polyisocyanats, das Allophanatgruppen

und β,γ-ethylenisch ungesättigte Ethergruppen enthält, mit einer hydroxyfunktionellen, ethylenisch ungesättigten Verbindung ist, wobei das ethylenisch ungesättigte Polyisocyanat durch Allophanatierung des Urethangruppen-enthaltenden Reaktionsprodukts eines organischen Diisocyanats mit einem β,γ-ethylenisch ungesättigten Etheralkohol hergestellt wird, wobei der β ,γ-ethylenisch ungesättigte Etheralkohol eine Verbindung umfasst, die aus der aus Glycerindiallylether, Trimethylolpropandiallylether und Pentaerythtrittriallylether bestehenden Gruppe ausgewählt ist. Die in EP 0 867 457 B1 offenbarten ethylenisch ungesättigten Polyurethane mit Allophanatgruppen können als Bindemittel in Einkomponenten-Beschichtungszusammensetzungen verwendet werden.

DE 10 2007 040 240 A1 und EP 1 645 582 A1 beschreiben jeweils Verfahren zur Herstellung von strahlenhärtenden Allophanaten durch Umsetzung von isocyanatgruppenhaltigen Verbindungen und hydroxyfunktionellen Verbindungen, wobei das Verhältnis von NCO-Gruppen zu den OH-Gruppen 1,45 : 1,0 bis 1,1 : 1,0 beträgt. Gemäß DE 10 2007 040 239 A1 werden unter Einsatz von bestimmten Mischungen enthaltend Hydroxyethylacrylat und Hydroxypropylacrylat als hydroxyfunktionellen Verbindungen entsprechende strahlenhärtenden Allophanate erhalten. Die strahlenhärtenden Allophanate gemäß dieser drei Dokumente können zur Herstellung von Beschichtungen und Lacken sowie Klebstoffen, Druckfarben, Gießharzen, Dentalmassen, Schlichten, Photoresisten, Stereolithographiesystemen, Harzen für Verbundwerkstoffe und Dichtungsmassen verwendet werden.

DE 10 2004 060 285 A1 betrifft strahlungshärtbare Zusammensetzungen auf Basis eines Dicidolgemisches (enthaltend zwei oder drei isomere 3,8-, 4,8- und/oder 5,8-Bis(hydroxymethyl)tricyclo[5.2.1.$0^{2,6}$]decane) mit mindestens einer Verbindung, welche mindestens eine ethylenisch ungesättigte Gruppierung mit gleichzeitig mindestens einer gegenüber Dicidol reaktive Gruppierung aufweist, wobei diese Verbindung ein Umsetzungsprodukt aus Hydroxyalkyl(meth)acrylat und Diisocyanat sein kann. Die Zusammensetzungen gemäß DE 10 2004 060 285 A1 können verwendet werden als strahlungsinduziert härtende Beschichtungsstoffe, Klebstoffe, Laminierungen, Druckfarben und Tinten, Polituren, Lasuren, Pigmentpasten, Spachtelmassen, Kosmetikartikel, Verpackungsmaterialien und/oder Dicht- und/oder Dämmstoffe.

WO 2006/063891 A1 offenbart radikalisch polymerisierbare Verbindungen, im Wesentlichen enthaltend das Umsetzungsprodukt eines Dicidolgemisches und mindestens einer Verbindung, welche mindestens eine ethylenisch ungesättigte Gruppierung mit gleichzeitig mindestens einer gegenüber Dicidol reaktive Gruppierung aufweist. Die Anwendungsgebiete entsprechen den in DE 10 2004 060 285 A1 genannten.

US 6,670,499 B1 beschreibt vom Adamantan abgeleitet Diurethane. Die in US 6,670,499 beschriebenen Verbindungen eignen sich als Intermediate für den Einsatz im dentalen Bereich oder zur Herstellung von optischen Materialien (wie beispielsweise Linsen).

Daher war es eine weitere Aufgabe der vorliegenden Erfindung, neue, vorzugsweise radikalisch polymerisierbare, Monomere bereitzustellen, die in einem erfindungsgemäßen bzw. erfindungsgemäß einzusetzenden bzw. erfindungsgemäß anzuwendenden dentalen Kompositmaterial in bzw. als Bestandteil der Komponente (b1) eingesetzt werden können.

Vorzugsweise sollen die unter Verwendung der Monomere erhältlichen Polymere eine ausgeprägte Hydrophobie aufweisen, die sich u.a. in einer sehr geringen Wasseraufnahme zeigt. Ebenfalls bevorzugt sollen sich die unter Verwendung der Monomere erhältlichen Polymere durch eine hohe mechanische Stabilität auszeichnen, die sich u.a. in einer hohen Biegefestigkeit zeigt. Besonders bevorzugt sollen sich unter Verwendung der Monomere solche Polymere herstellen lassen, die sowohl eine geringe Wasseraufnahme als auch eine hohe Biegefestigkeit als auch ein hervorragendes Anfließverhalten aufweisen.

Diese weitere Aufgabe wird gelöst durch eine Verbindung der Struktur $Q(YZ_e)_b$ (wobei jedes Y unabhängig von etwaigen weiteren Strukturelementen Y gewählt ist) mit ein, zwei, drei, vier oder mehr funktionellen Gruppen ausgewählt aus der Gruppe bestehend aus *N*-Acylharnstoff, Allophanat, Biuret und Amid, wobei die Amidfunktion nicht unmittelbar mit einem N-Atom, einem O-Atom oder einer Carbonylgruppe verknüpft ist, wobei gilt:

- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten $YZ_e$ substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sind,

- b ist eine natürliche Zahl ausgewählt aus der Gruppe der natürlichen Zahlen 2, 3, 4,

- jedes Z bedeutet ein Strukturelement, das unabhängig von etwaigen weiteren Strukturelementen Z ausgewählt ist aus der Gruppe bestehend aus

$$-O-(C=O)-CH=CH_2,\ -O-(C=O)-C(CH_3)=CH_2,$$

$-(C{=}O)-CH{=}CH_2$, $-(C{=}O)-C(CH_3){=}CH_2$,

$-CH{=}CH_2$, $-C(CH_3){=}CH_2$ und $-O-CH{=}CH_2$,

- jeder Index e ist eine natürliche Zahl, die unabhängig von etwaigen weiteren Indizes e ausgewählt ist aus der Gruppe der natürlichen Zahlen 1, 2, 3 und 4,

- jedes Y bedeutet ein Strukturelement, welches in der Struktur $Q(YZ_e)_b$ das polyalicyclische Strukturelement Q mit e Strukturelementen Z verbindet,

wobei die Verbindung ein erstes Umsetzungsprodukt ist aus einer ersten Reaktion von

A) einer Verbindung der Struktur $QG_b$, worin jedes G eine reaktive Gruppe bedeutet, die unabhängig von weiteren Gruppen G ausgewählt ist aus der Gruppe bestehend aus $(-CH_2)_n-NH_2$, $(-CH_2)_n-(OCH_2-CHR)_m-OH$, $(-CH_2)_n-NCO$ und $(-CH_2)_n-COOH$ mit

B) zwei oder mehr gleichen oder verschiedenen Verbindungen $MZ_e$, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, $-NH_2$, -OH, -NCO und -COOH,
wobei gilt:

- R bedeutet jeweils unabhängig von etwaigen weiteren R ein Wasserstoffatom oder einen Alkylrest; bevorzugt bedeutet R ein Wasserstoffatom oder einen linearen oder verzweigten Alkylrest mit 1 bis 6 C-Atomen; weiter bevorzugt bedeutet R ein Wasserstoffatom oder einen Methylrest,

- m ist eine natürliche Zahl ausgewählt aus der Gruppe der natürlichen Zahlen von 0 bis 10,

- jeder Index n ist eine natürliche Zahl, die unabhängig von etwaigen weiteren Indizes n ausgewählt ist aus der Gruppe bestehend aus 0 und 1,

oder
die Verbindung ein zweites Umsetzungsprodukt ist aus einer Reaktion von dem obigen ersten Umsetzungsprodukt mit

C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
oder
die Verbindung ein drittes Umsetzungsprodukt ist aus einer Reaktion von dem obigen zweiten Umsetzungsprodukt mit

D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.

Aus dem oben Gesagten ergibt sich, dass in erfindungsgemäß einzusetzenden Verbindungen, die eine Amidgruppe (wie definiert) enthalten, diese Amidgruppe nicht Bestandteil einer Urethangruppe ist.

Weiter bevorzugt sind erfindungsgemäß einzusetzende Verbindungen der Struktur $Q(YZ_e)_b$ (wobei jedes Y unabhängig von etwaigen weiteren Strukturelementen Y gewählt ist) der Komponente (b1) mit ein, zwei, drei, vier oder mehr funktionellen Gruppen ausgewählt aus der Gruppe bestehend *N*-Acylharnstoff, Allophanat, Biuret und Amid, wobei die Amidfunktion nicht unmittelbar mit einem N-Atom, einem O-Atom oder einer Carbonylgruppe verknüpft ist, wobei das Amid wiederum vorzugsweise (Meth)acrylamid bedeutet.

In bevorzugten erfindungsgemäß einzusetzenden Verbindungen der Struktur $Q(YZ_e)_b$ (wobei jedes Y unabhängig von etwaigen weiteren Strukturelementen Y gewählt ist) erfolgt die Verknüpfung zwischen Q und zumindest einem Strukturelement Z über eine Brücke, die ein zweibindiges Brückenglied enthält oder aus diesem besteht, ausgewählt aus der Gruppe bestehend aus

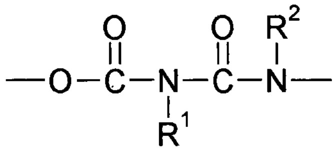
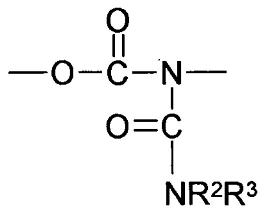
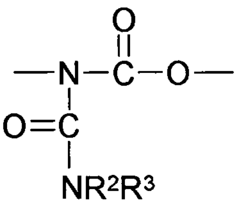
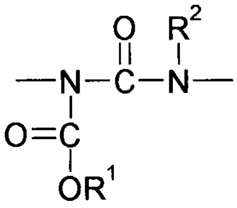
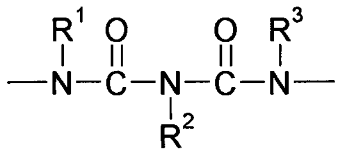
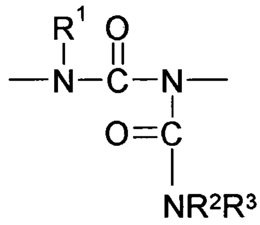
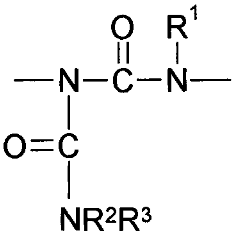
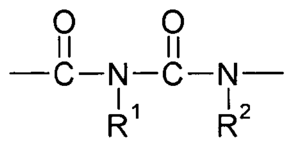
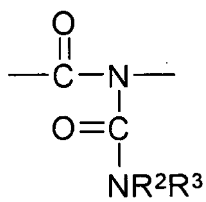
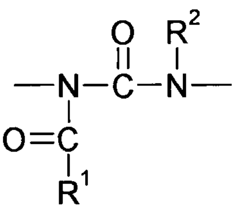
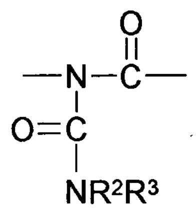
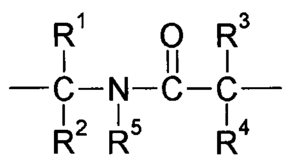
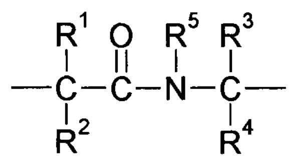

wobei $R^1$, $R^2$, $R^3$ $R^4$, $R^5$ sonstige Reste der Verbindung bedeuten, wobei die jeweils im Formelbild links angeordnete Bindung dem Strukturelement Q und die rechts angeordnete Bindung dem Strukturelement Z näher ist.

Die weitere Aufgabe wird ebenfalls gelöst durch neue Verbindungen der Struktur $Q(Y_xZ_e)_b$ mit x = 1 mit ein, zwei, drei, vier oder mehr funktionellen Gruppen, welche ausgewählt sind aus der Gruppe bestehend aus *N*-Acylharnstoff, Allophanat und Biuret, wobei gilt:

- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei keines, eines, zwei oder mehr der nicht durch Substituenten $YZ_e$ substituierten Wasserstoffatome dieses polyalicyclischen Strukturelements Q durch Alkylgruppen, Alkoxygruppen, Halogenatome oder Trifluormethylgruppen substituiert ist bzw. sind;

- b ist eine natürliche Zahl ausgewählt aus der Gruppe der natürlichen Zahlen 2, 3, 4;

- jedes Z bedeutet ein Strukturelement, das unabhängig von etwaigen weiteren Strukturelementen Z ausgewählt ist aus der Gruppe bestehend aus

$-O-(C=O)-CH=CH_2$, $-O-(C=O)-C(CH_3)=CH_2$,

$-(C=O)-CH=CH_2$, $-(C=O)-C(CH_3)=CH_2$,

$-CH=CH_2$, $-C(CH_3)=CH_2$ und $-O-CH=CH_2$,

- jeder Index e ist eine natürliche Zahl, die unabhängig von etwaigen weiteren Indizes e ausgewählt ist aus der Gruppe der natürlichen Zahlen 1, 2, 3 und 4;

- jedes Y bedeutet ein Strukturelement, welches in der Struktur $Q(Y_xZ_e)_b$ mit x = 1 das polyalicyclische Strukturelement Q mit e Strukturelementen Z verknüpft und ein Strukturelement enthält oder aus diesem besteht, ausgewählt aus der Gruppe bestehend aus

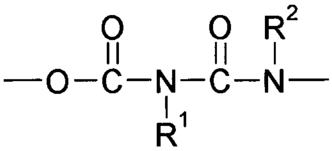
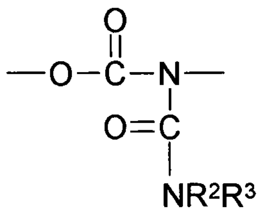
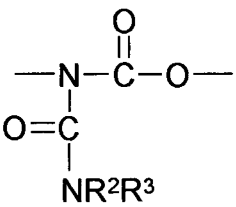

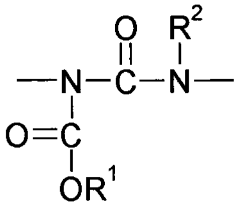
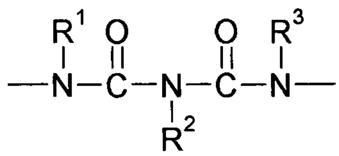
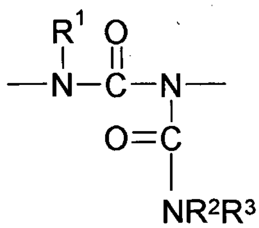

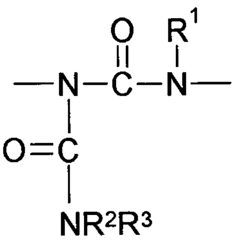
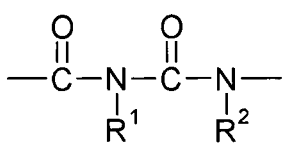
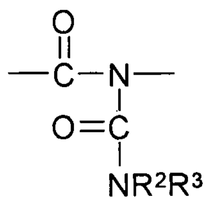

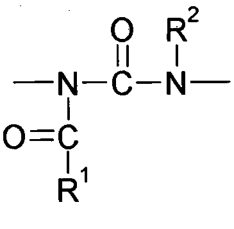
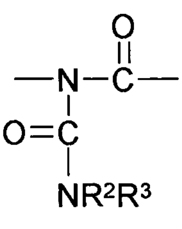

wobei $R^1$, $R^2$ und $R^3$ sonstige Reste der Verbindung bedeuten, wobei die jeweils im Formelbild links angeordnete

Bindung dem Strukturelement Q und die rechts angeordnete Bindung dem Strukturelement Z näher ist. Diese Verbindungen sind in besonderer Weise als Monomere für den Einsatz in erfindungsgemäßen Kompositmaterialien geeignet.

Vorzugsweise umfasst eine solche Verbindung der Struktur $Q(Y_xZ_e)_b$ mit x = 1 zwei, drei, vier oder mehr funktionelle Gruppen ausgewählt aus der Gruppe bestehend aus N-Acylharnstoff, Allophanat und Biuret.

In einer bevorzugten Ausgestaltung bedeutet jeder Index e eine natürliche Zahl, die unabhängig von etwaigen weiteren Indizes e ausgewählt ist aus der Gruppe der natürlichen Zahlen 2, 3 und 4.

Die oben genannten sonstigen Reste $R^1$, $R^2$ bzw. $R^3$ einer erfindungsgemäß einzusetzenden neuen Verbindung sind, jeweils unabhängig voneinander, vorzugsweise ausgewählt aus der Gruppe bestehend aus Wasserstoff, linearen, verzweigten oder Ringe umfassenden Strukturelementen mit 1 bis 30 C-Atomen und 0 bis 10 Heteroatomen, wobei die gegebenenfalls vorhandenen Heteroatome vorzugsweise gewählt sind aus der Gruppe bestehend aus N und O.

Die sonstigen Reste $R^1$, $R^2$ bzw. $R^3$ einer erfindungsgemäß einzusetzenden neuen Verbindung sind, jeweils unabhängig voneinander, bevorzugt ausgewählt aus der Gruppe bestehend aus Wasserstoff, linearen, verzweigten oder Ringe umfassenden Strukturelementen mit 1 bis 25 C-Atomen und 0 bis 8 Heteroatomen, wobei die gegebenenfalls vorhandenen Heteroatome vorzugsweise ausgewählt sind aus der Gruppe bestehend aus N und O.

Die sonstigen Reste $R^1$, $R^2$ bzw. $R^3$ einer erfindungsgemäß einzusetzenden neuen Verbindung sind, jeweils unabhängig voneinander, bevorzugt ausgewählt aus der Gruppe bestehend aus Wasserstoff, linearen, verzweigten oder Ringe umfassenden Strukturelementen mit 1 bis 20 C-Atomen und 0 bis 5 Heteroatomen, wobei die gegebenenfalls vorhandenen Heteroatome ausgewählt sind aus der Gruppe bestehend aus N und O.

Eine in einem erfindungsgemäßen bzw, erfindungsgemäß anzuwendenden Kompositmaterial einzusetzende neue Verbindung $Q(Y_xZ_e)_b$ mit x = 1, vorzugsweise eine Verbindung $Q(Y_xZ_e)_b$ mit x = 1 wie sie vorstehend oder nachfolgend als bevorzugt bezeichnet ist, ist vorzugsweise herstellbar durch Umsetzung eines ersten Umsetzungsproduktes, welches das Umsetzungsprodukt ist aus einer ersten Reaktion von

A) einer Verbindung der Struktur $QG_b$, worin jedes G eine reaktive Gruppe bedeutet, die unabhängig von weiteren Gruppen G ausgewählt ist aus der Gruppe bestehend aus $(-CH_2)_n-NH_2$, $(-CH_2)_n-(OCH_2-CHR)_m-OH$, $(-CH_2)_n-NCO$ und $(-CH_2)_n-COOH$ mit

B) zwei oder mehr gleichen oder verschiedenen Verbindungen $MZ_e$, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, $-NH_2$, -OH, -NCO und -COOH,
wobei gilt:

- R bedeutet jeweils unabhängig von etwaigen weiteren R ein Wasserstoffatom oder einen Alkylrest;
- m ist eine natürliche Zahl ausgewählt aus der Gruppe der natürlichen Zahlen von 0 bis 10,
- jeder Index n ist eine natürliche Zahl, die unabhängig von etwaigen weiteren Indizes n ausgewählt ist aus der Gruppe bestehend aus 0 und 1,

wobei die Verbindung ein zweites Umsetzungsprodukt ist aus einer Reaktion von dem obigen ersten Umsetzungsprodukt mit

C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
oder
die Verbindung ein drittes Umsetzungsprodukt ist aus einer Reaktion von dem obigen zweiten Umsetzungsprodukt mit

D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.

Eine bevorzugt einzusetzende Verbindung gemäß einer bevorzugten Ausgestaltung ist ein zweites Umsetzungsprodukt aus einer Reaktion von dem obigen ersten Umsetzungsprodukt mit

C) einer weiteren Verbindung gemäß A) oder B), wobei jede weitere Verbindung gemäß A) bzw. B) dieselbe Bedeutung hat wie in der ersten Reaktion,
und/oder
wobei die Verbindung ein drittes Umsetzungsprodukt ist aus einer Reaktion von dem obigen zweiten Umsetzungsprodukt mit

D) einer weiteren Verbindung gemäß A) oder B), wobei jede weitere Verbindung gemäß A) bzw. B) dieselbe Bedeutung hat wie in der ersten und/oder der zweiten Reaktion, vorzugsweise wie in der ersten und der zweiten Reaktion.

In einer bevorzugten Ausführungsform ist m = 0. Dies gilt für sämtliche Aspekte der vorliegenden Erfindung.

In bevorzugten erfindungsgemäß einzusetzenden Verbindungen erfolgt die Verknüpfung zwischen Q und zumindest einem Strukturelement Z über eine Brücke, die ein zweibindiges Brückenglied enthält oder aus diesem besteht, ausgewählt aus der Gruppe bestehend aus

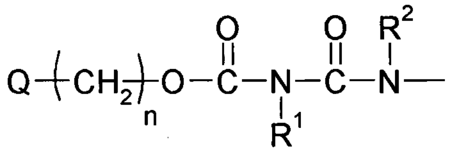

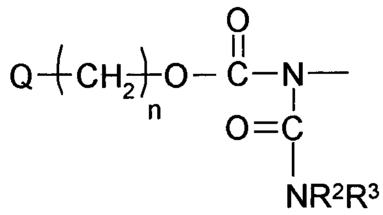

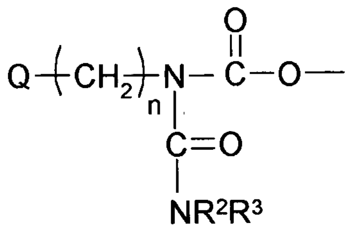

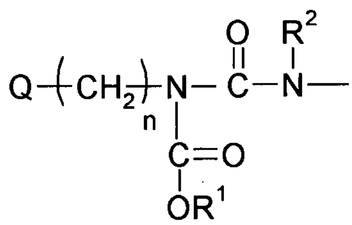

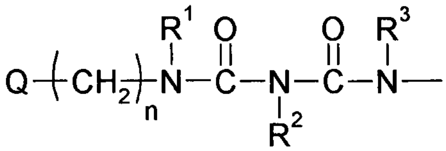

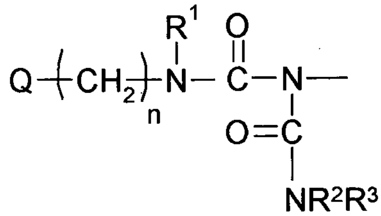

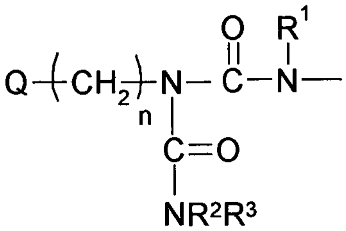

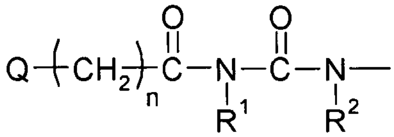

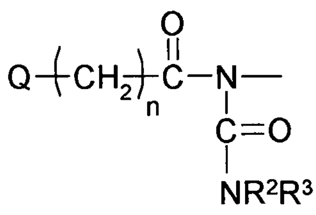
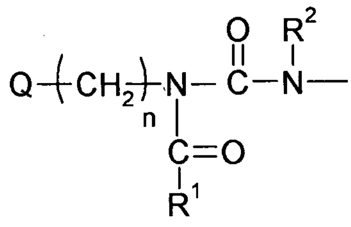
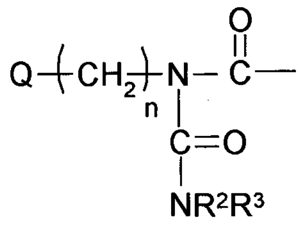

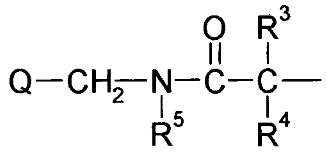
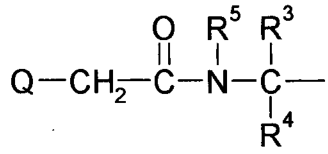

wobei $R^1$, $R^2$, $R^3$ $R^4$, $R^5$ sonstige Reste der Verbindung bedeuten und Q sowie der Index n die oben angegebene Bedeutung haben.

Die jeweils im Formelbild rechts angeordnete Bindung ist dem Strukturelement Z näher.

In einer bevorzugten Ausgestaltung umfasst eine neue erfindungsgemäß einzusetzende Verbindung $Q(Y_xZ_e)_b$ mit x = 1, vorzugsweise eine Verbindung $Q(Y_xZ_e)_b$ mit x = 1 wie sie vorstehend oder nachfolgend als bevorzugt bezeichnet ist, ein oder mehrere Strukturelemente ausgewählt aus der Gruppe bestehend aus

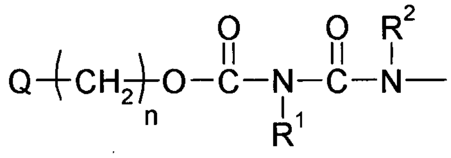
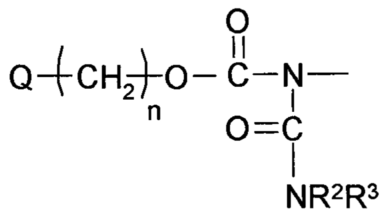

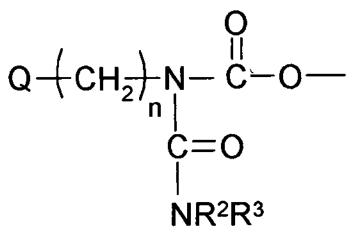
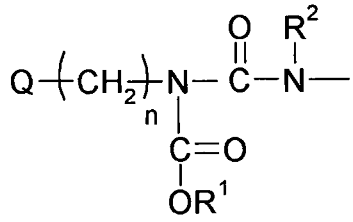

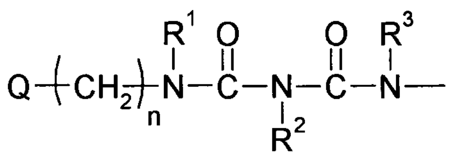
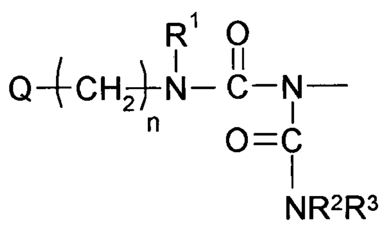

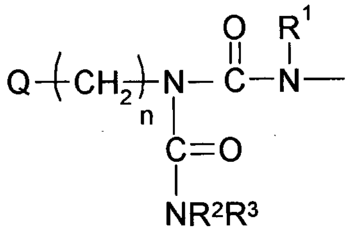

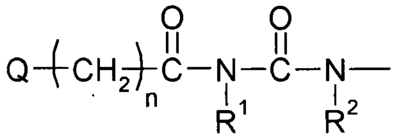

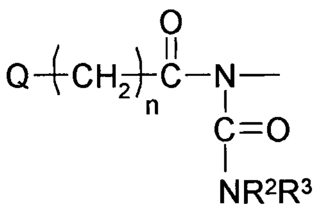

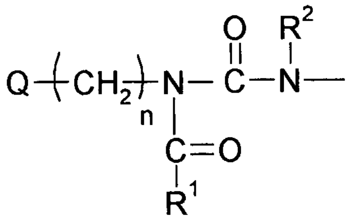

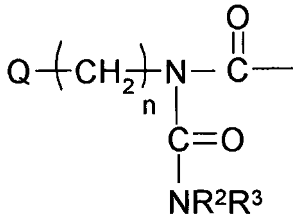

wobei $R^1$, $R^2$ und $R^3$ sonstige Reste der Verbindung bedeuten (und vorzugsweise die oben genannte bevorzugte Bedeutung haben) und Q die oben genannte Bedeutung hat und der Index n ausgewählt ist aus der Gruppe bestehend aus 0 und 1.

Wie oben bereits erwähnt sind bevorzugte erfindungsgemäß einzusetzende neue Verbindungen solche, wobei Q ein gesättigtes polyalicyclisches Strukturelement bedeutet, das ausgewählt ist aus der Gruppe bestehend aus bicyclischen und tricyclischen Kohlenwasserstoffresten, wobei vorzugsweise keines der nicht durch Substituenten $YZ_e$ substituierten Wasserstoffatome dieses polyalicyclischen Strukturelements Q substituiert ist.

Besonders bevorzugte erfindungsgemäß einzusetzende neue Verbindungen sind solche, wobei das Strukturelement Q einen Tricyclo[5.2.1.$0^{2,6}$]decan-Rest, einen Tricyclo[5.2.1.$0^{2,6}$]dec-3-en-Rest, einen Tricyclo[3.3.1.$1^{3,7}$]decan-Rest oder einen Bicyclo[2.2.1]heptan-Rest bedeutet.

Bevorzugte erfindungsgemäß einzusetzende neue Verbindungen der Komponente (b1) sind solche, wobei Q einen tricyclischen Kohlenwasserstoffrest bedeutet, wobei vorzugsweise keines der nicht durch Substituenten $YZ_e$ (wobei jedes Y unabhängig von etwaigen weiteren Strukturelementen Y gewählt ist oder entfällt) substituierten Wasserstoffatome dieses tricyclischen Kohlenwasserstoffrestes substituiert ist.

Besonders bevorzugte erfindungsgemäß einzusetzende neue Verbindungen sind solche, wobei das Strukturelement Q einen Tricyclo[5.2.1.$0^{2,6}$]decan-Rest, einen Tricyclo[5.2.1.$0^{2,6}$]dec-3-en-Rest oder einen Tricyclo[3.3.1.$1^{3,7}$]decan-Rest bedeutet, weiter bevorzugt einen Tricyclo[5.2.1.$0^{2,6}$]decan-Rest oder einen Tricyclo[3.3.1.$1^{3,7}$]decan-Rest.

Bevorzugt sind erfindungsgemäß einzusetzende neue Verbindungen, in denen

(i) das Strukturelement Z = $-O-(C=O)-C(CH_3)=CH_2$ bedeutet, wobei die funktionellen Gruppen Allophanat-, Biuret- oder Acylharnstoff-Gruppen sind, da mit diesen Verbindungen besonders gute Ergebnisse erzielt wurden, und/oder

(ii) das Strukturelement Q einen Tricyclo[5.2.1.$0^{2,6}$]decan-Rest bedeutet.

Weiter bevorzugt sind erfindungsgemäß einzusetzende neue Verbindungen, in denen das Strukturelement Z = $-O-(C=O)-C(CH_3)=CH_2$ bedeutet, wobei die funktionellen Gruppen Allophanat-, Biuret-oder Acylharnstoff-Gruppen sind und das Strukturelement Q einen Tricyclo[5.2.1.$0^{2,6}$]decan-Rest bedeutet.

Bevorzugt sind erfindungsgemäß einzusetzende neue Verbindungen, in denen sämtliche vorhandenen lichthärtbaren Gruppen dem Strukturelement Z entsprechen.

Bevorzugt sind erfindungsgemäß einzusetzende neue Verbindungen, in denen sämtliche vorhandenen endständigen polymerisierbaren Gruppen dem Strukturelement Z entsprechen.

Eine erfindungsgemäß einzusetzende neue Verbindung kann neben lichthärtbaren Gruppen des Strukturelements Z auch weitere polymerisierbare, vorzugsweise endständige polymerisierbare, Gruppen umfassen, die nicht lichthärtbar sind, insbesondere nicht unter den im Dentalbereich üblichen Bedingungen des Lichthärtens. Dies ist regelmäßig jedoch nicht bevorzugt, da solche Gruppen nicht zu den gewünschten Eigenschaften des nach der Polymerisation vorliegenden Produktes beitragen.

Weiter bevorzugte erfindungsgemäß einzusetzende Verbindungen sind solche, in denen mindestens ein Struk-

turelement $YZ_e$ unabhängig von dem oder den weiteren Strukturelementen $YZ_e$ ausgewählt ist, und vorzugsweise sämtliche Strukturelemente $YZ_e$ ausgewählt sind, aus der Gruppe bestehend aus

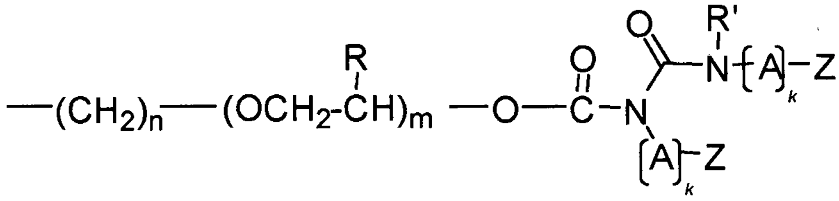

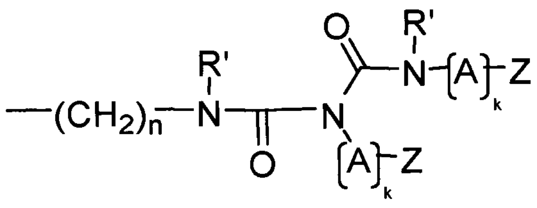

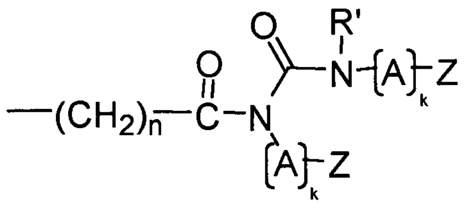

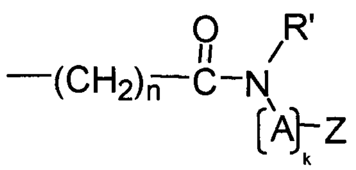

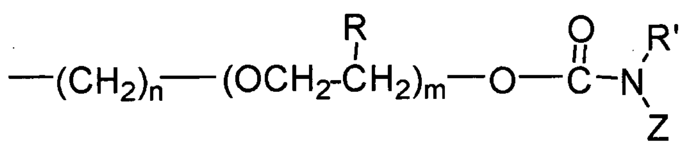

wobei Z, R, m sowie n die obige Bedeutung haben und worin ferner gilt:

- jedes A bedeutet ein zweibindiges organisches Brückenglied,
- jeder Index k ist eine natürliche Zahl, die unabhängig von etwaigen weiteren Indizes k ausgewählt ist aus der Gruppe bestehend aus 0 und 1;
- jedes R' bedeutet ein Strukturelement, das unabhängig von etwaigen weiteren Strukturelementen R' ausgewählt ist aus der Gruppe bestehend aus H und einem Strukturelement $(C{=}O)\text{-}NH\text{-}(A)_k\text{-}Z$, wobei A, Z und k wiederum die obigen Bedeutungen haben.

In einer bevorzugten Ausführungsform ist m = 0.

Ebenfalls bevorzugte erfindungsgemäß einzusetzende Verbindungen sind solche, bei denen mindestens ein Strukturelement $YZ_e$ unabhängig von dem oder den weiteren Strukturelementen $YZ_e$ ausgewählt ist, und vorzugsweise sämtliche Strukturelemente $YZ_e$ ausgewählt sind, aus der Gruppe bestehend aus

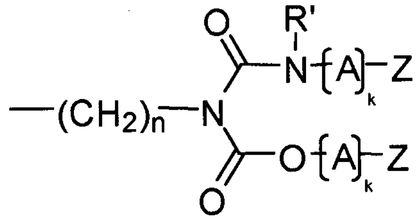

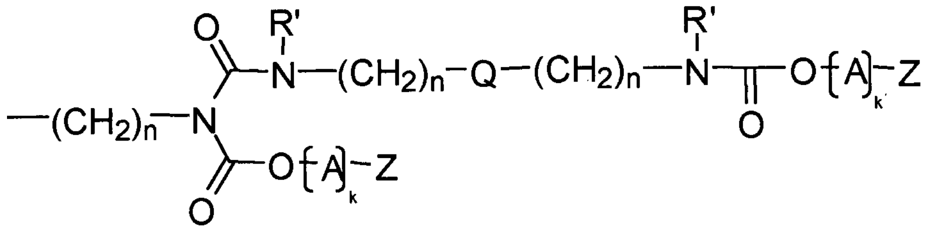

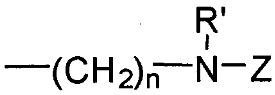

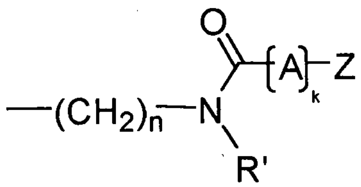

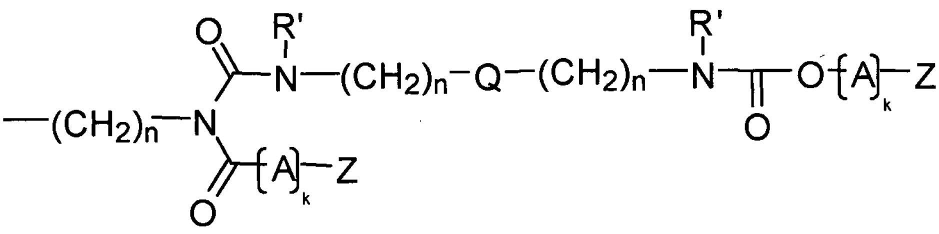

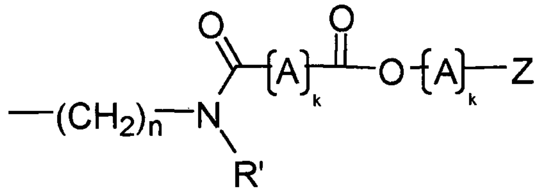

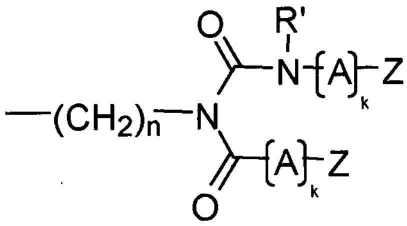

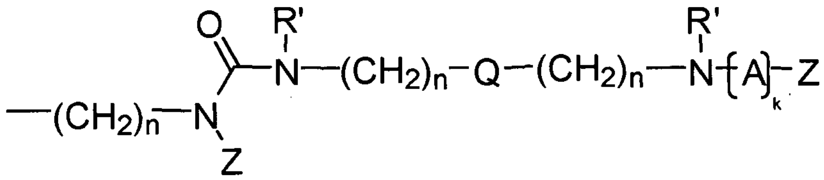

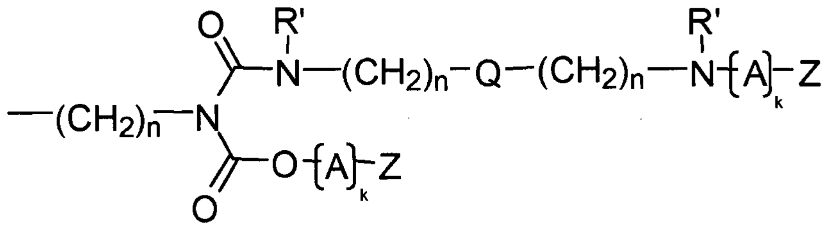

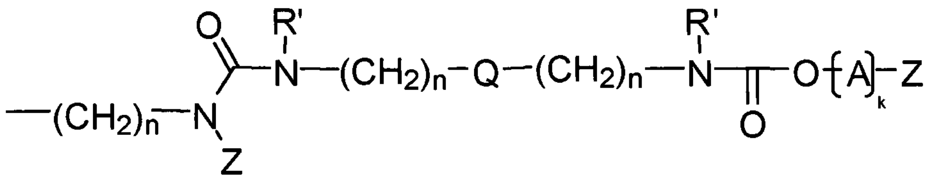

wobei jedes Q unabhängig von etwaigen weiteren Strukturelementen Q die obige Bedeutung hat und wobei Z, A, k und R‘ sowie n die oben genannte Bedeutung haben.

In einer bevorzugten Ausgestaltung betrifft die vorliegende Erfindung eine erfindungsgemäß einzusetzende Verbindung $Q(Y_xZ_e)_b$ mit x = 1, vorzugsweise eine erfindungsgemäß einzusetzende Verbindung $Q(Y_xZ_e)_b$ mit x = 1 wie sie vorstehend oder nachfolgend als bevorzugt bezeichnet ist, wobei mindestens ein Strukturelement $YZ_e$ unabhängig von dem oder den weiteren Strukturelementen $YZ_e$ ausgewählt ist, und vorzugsweise sämtliche Strukturelemente $YZ_e$ ausgewählt sind, aus der Gruppe bestehend aus

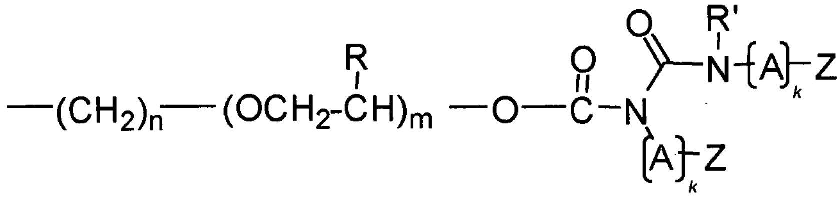

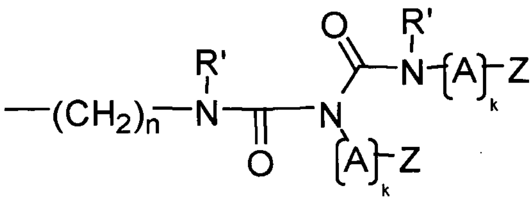

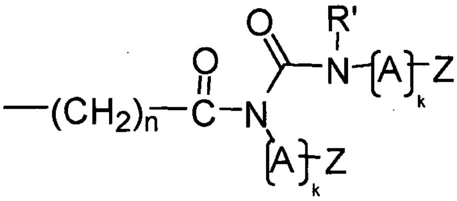

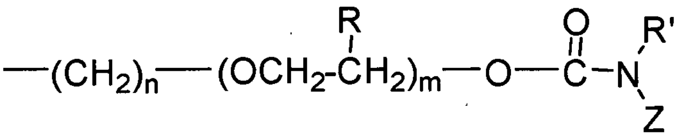

wobei Z, R, m sowie n die obige Bedeutung haben und worin ferner gilt:

- jedes A bedeutet ein organisches Strukturelement,
- jeder Index k ist eine natürliche Zahl, die unabhängig von etwaigen weiteren Indizes k ausgewählt ist aus der Gruppe bestehend aus 0 und 1;
- jedes R' bedeutet ein Strukturelement, das unabhängig von etwaigen weiteren Strukturelementen R' ausgewählt ist aus der Gruppe bestehend aus H und einem Strukturelement (C=O)-NH-$(A)_k$-Z, wobei A, Z und k wiederum die obigen Bedeutungen haben.

In einer bevorzugten Ausgestaltung betrifft die vorliegende Erfindung eine erfindungsgemäß einzusetzende neue Verbindung $Q(Y_xZ_e)_b$ mit x = 1, vorzugsweise eine erfindungsgemäß einzusetzende Verbindung $Q(Y_xZ_e)_b$ mit x = 1 wie sie vorstehend oder nachfolgend als bevorzugt bezeichnet ist, wobei
mindestens ein Strukturelement $YZ_e$ unabhängig von dem oder den weiteren Strukturelementen $YZ_e$ ausgewählt ist, und vorzugsweise sämtliche Strukturelemente $YZ_e$ ausgewählt sind,
aus der Gruppe bestehend aus

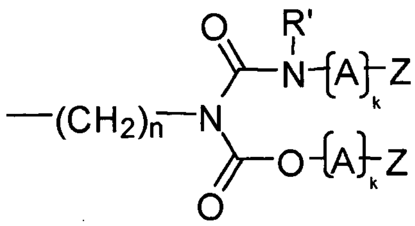

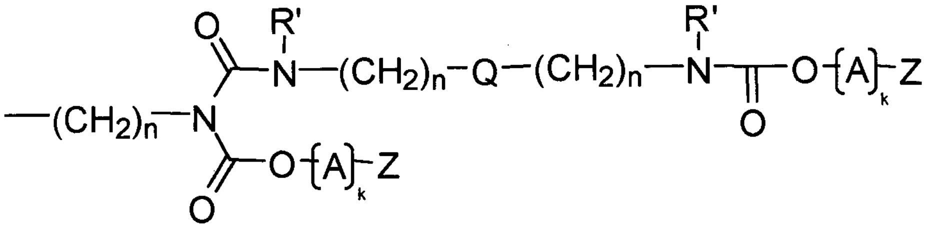

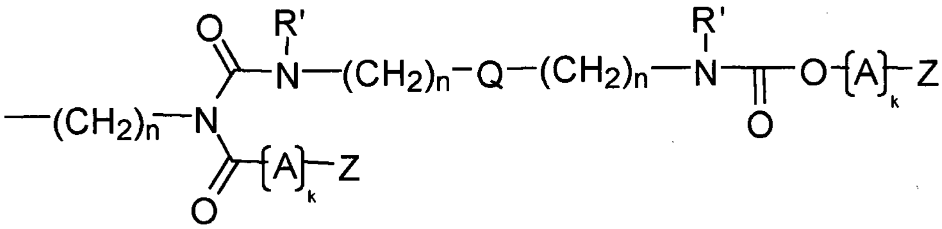

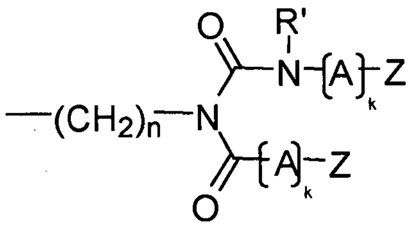

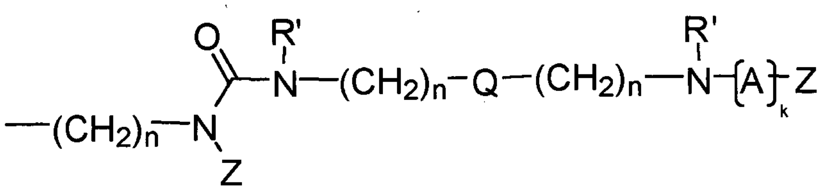

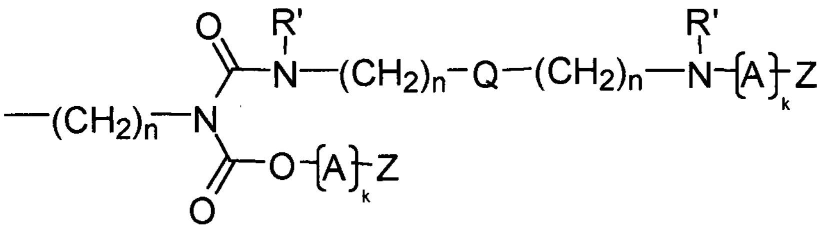

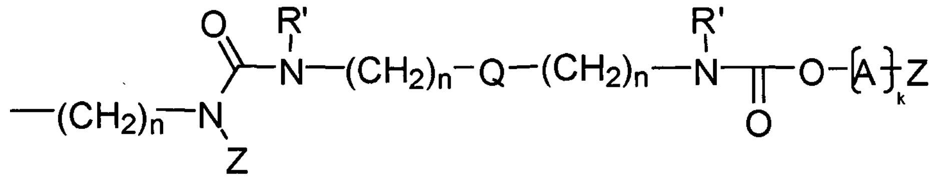

wobei jedes Q unabhängig von etwaigen weiteren Strukturelementen Q die obige Bedeutung hat, und wobei Z und n die oben genannte Bedeutung haben und wobei ferner gilt:

- jedes A bedeutet ein organisches Strukturelement,
- jeder Index k ist eine natürliche Zahl, die unabhängig von etwaigen weiteren Indizes k ausgewählt ist aus der Gruppe bestehend aus 0 und 1;
- jedes R' bedeutet ein Strukturelement, das unabhängig von etwaigen weiteren Strukturelementen R' ausgewählt ist aus der Gruppe bestehend aus H und einem Strukturelement (C=O)-NH-$(A)_k$-Z, wobei A, Z und k wiederum die obigen Bedeutungen haben.

Dabei wiederum bevorzugt sind erfindungsgemäß einzusetzende Verbindungen, in denen jedes Strukturelement A unabhängig von etwaigen weiteren Strukturelementen A ausgewählt ist aus der Gruppe bestehend aus linearen, verzweigten oder Ringe umfassenden zweibindigen organischen Brückengliedern mit 1 bis 25 C-Atomen und gegebenenfalls 1 bis 10, vorzugsweise 1 bis 5 Heteroatomen, wobei die gegebenenfalls vorhandenen Heteroatome vorzugsweise gewählt sind aus der Gruppe bestehend aus N und O.
Dabei wiederum bevorzugt ist eine erfindungsgemäß einzusetzende Verbindung, in der jedes Strukturelement A unabhängig von etwaigen weiteren Strukturelementen A ausgewählt ist aus der Gruppe bestehend aus linearen, verzweigten oder Ringe umfassenden Strukturelementen mit 1 bis 25 C-Atomen und 0 bis 10 Heteroatomen, vorzugsweise mit 1 bis 5 Heteroatomen, wobei die gegebenenfalls vorhandenen Heteroatome vorzugsweise gewählt sind aus der Gruppe bestehend aus N und O.
Weiter bevorzugt sind Verbindungen, in denen jedes Strukturelement A unabhängig von etwaigen weiteren Strukturelementen A ausgewählt ist aus der Gruppe bestehend aus $C_1$-$C_{20}$-Alkylen, $C_1$-$C_{20}$-Heteroalkylen, $C_3$-$C_{20}$-Cycloalkylen, $C_4$-$C_{20}$-Cycloalkylalkylen, $C_2$-$C_{20}$-Alkenylen, $C_3$-$C_{20}$-Cycloalkenylen, $C_4$-$C_{20}$-Cycloalkenylalkylen, $C_4$-$C_{20}$-Cycloalkenylenalkylen, $C_3$-$C_{25}$-Arylen, $C_2$-$C_{25}$-Heteroarylen, $C_4$-$C_{25}$-Arylalkylen, $C_4$-$C_{25}$-Arylenalkylen, $C_4$-$C_{25}$-Arylheteroalkylen, $C_4$-$C_{25}$-Arylenheteroalkylen.
In bevorzugten Ausgestaltungen umfasst Strukturelement A eine oder mehrere der folgenden Atome oder Atomgruppen:

$$\text{-O-, -O-}Ar^1\text{-}CR^6R^7\text{-}Ar^2\text{-O-, -}NR^8\text{-, -N-(C=O)-, -NH-(C=O)-O-, -NH-C(=O)-NH-}$$

wobei gilt:

$Ar^1$ und $Ar^2$ bedeuten unabhängig voneinander einen gegebenenfalls substituierten, dabei vorzugsweise einen ein oder mehrfach mit C1-C4-Alkylresten substituierten, aromatischen Ring, dabei wiederum bevorzugt einen Phenylring,

$R^6$, $R^7$ und $R^8$ bedeuten unabhängig voneinander Wasserstoff oder einen C1-C8-Rest, dabei vorzugsweise einen C1-C4-Alkylrest, dabei wiederum bevorzugt Methyl oder Ethyl.

Zur Herstellung der genannten Verbindungen der Struktur $Q(YZ_e)_b$ bzw. $Q(Y_xZ_e)_b$ können vorzugsweise Hydroxylverbindungen von (Meth)acrylaten eingesetzt werden, wobei auch Gemische von Acrylaten und Methacrylaten verwendet werden können. Bevorzugt sind zum Einsatz als Reaktionspartner gemäß Komponente B), C) oder D):

Alkylenoxidmono(meth)acrylate wie beispielsweise Ethylenglykolmono(meth)acrylat, Diethylenglykolmono(meth)acrylat, Triethylenglykolmono(meth)acrylat, Tetraethylenglykolmono(meth)acrylat, etc., Polyalkylenoxidmono(meth)acrylate wie beispielsweise Polyethylenglykolmono(meth)acrylat, Polypropylenglykolmono(meth)acrylat, Polybutylenglykolmono(meth)acrylat, etc., Hydroxyalkylmono(meth)acrylate wie beispielsweise

Hydroxyethyl(meth)acrylat, Hydroxypropyl(meth)acrylat, 2-Hydroxypropyl(meth)acrylat, Hydroxybutyl(meth)acrylat, 4-Hydroxybutyl(meth)acrylat, Hydroxypentyl(meth)acrylat, 3-Hydroxy-2,2-dimethylpropyl(meth)acrylat, Hydroxyhexyl(meth)acrylat, Hydroxyheptyl(meth)acrylat, Hydroxyoctyl(meth)acrylat, Hydroxynonyl(meth)acrylat, Hydroxydecyl(meth)acrylat, Hydroxyundecyl(meth)acrylat, Hydroxydodecyl(meth)acrylat, etc., Poly(ε-caprolacton)mono(meth)acrylat, Poly(γ-caprolacton)mono(meth)acrylat, etc., die Mono-, Di-, Tetra-, oder Penta(meth)acrylate mehrwertiger Alkohole, wie Glycerin, wie beispielsweise Glycerindi(meth)acrylat (2-Hydroxypropyl-1,3-di(meth)acrylat, 3-Hydroxypropyl-1,2-di(meth)acrylat), wie Trimethylolpropan, wie beispielsweise Trimethylolpropandi(meth)acrylat, wie Pentaerythrit, wie beispielsweise Pentaerythritol-tri(meth)acrylat, wie Dipentaerythrit, wie beispielsweise Dipentaerythritol-penta(meth)acrylat, wie Ditrimethylolpropantri(meth)acrylat, wie Neopentylglykol(meth)acrylat, die (Meth)acrylate von alkoxyliertem oder phenoxyliertem Glycerin, dabei vorzugsweise die (Meth)acrylate von ethoxyliertem, propoxyliertem, etc. Glycerin, Trimethylolpropan, Pentaerythrit, Dipentaerythrit, Ditrimethylolpropan, etc. sowie deren technische Gemische, Bisphenol-A-Glycidyl-(Meth)acrylat (Bis-GMA), Bisphenol-B-Glycidyl-(Meth)acrylat, Bisphenol-C-Glycidyl-(Meth)acrylat, Bisphenol-F-Glycidyl-(Meth)acrylat, alkoxyliertes Bisphenol-A-Glycidyl-(Meth)acrylat (z.B. ethoxyliertes Bisphenol-A-Glycidyl-(Meth)acrylat), etc..

Zur Herstellung der erfindungsgemäß einzusetzenden Verbindungen können als Komponente B) auch Isocyanate eingesetzt werden. Bevorzugt sind dabei Mono- und Diisocyanate.

Bevorzugte Diisocyanate sind gewählt aus der Gruppe bestehend aus Cyclohexandüsocyanat, Methylcyclohexandüsocyanat, Ethylcyclohexandüsocyanat, Propylcyclohexandüsocyanat, Methyldiethylcyclohexandüsocyanat, Phenylendiisocyanat, Toluylendiisocyanat, Bis(isocyanatophenyl)methan, Propandiisocyanat, Butandiisocyanat, Pentandiisocyanat, Hexandiisocyanat, wie Hexamethylendiisocyanat oder 1,5-Diisocyanato-2-methylpentan, Heptandiisocyanat, Octandiisocyanat, Nonandiisocyanat, wie 1,6-DÜsocyanato-2,4,4-trimethylhexan oder 1,6-Diisocyanato-2,2,4-trimethylhexan, Nonantriisocyanat, wie 4-Isocyanatomethyl-1,8-octandiisocyanat, Decandi- und triisocyanat, Undekandi- und -triisocyanat, Dodecandi- und -triisocyanate, isophorondiisocyanat, Dicyclohexylmethan-4,4'-diisocyanat, Isocyanatomethylmethylcyclohexylisocyanat, 1,3-Bis(isocyanatomethyl)cyclohexan oder 1,4-Bis(isocyanatomethyl)cyclohexan.

Bevorzugte Monoisocyanate sind (Meth)acryloylisocyanat und (Meth)acryl-C2-C8-alkylisocyanate (d.h. (Meth)acrylalkylisocyanate mit Alkylspacern, die über 2 bis 8, besonders bevorzugt 2 bis 6 Kohlenstoffatome verfügen), dabei wiederum bevorzugt ist (Meth)acrylethylisocyanat (2-Isocyanatoethyl(meth)acrylat).

Außerdem haben sich als Komponente B) Monoisocyanate vorteilhaft erwiesen, die Umsetzungsprodukte sind aus Amino- bzw. Hydroxyalkyl(meth)acrylaten, deren Alkylspacer über 1 bis 12, bevorzugt 2 bis 8, besonders bevorzugt 2 bis 6 Kohlenstoffatome verfügen, und Diisocyanaten.

Vorzugsweise wird dazu ein vorstehend genanntes Diisocyanat äquimolar mit einer (oben als bevorzugt gekennzeichneten) Amino- oder Hydroxylalkylverbindung eines (Meth)acrylats umgesetzt, wobei die Hydroxylalkylverbindungen wiederum vorzugsweise gewählt sind aus der Gruppe bestehend aus Hydroxyethyl(meth)acrylat, Hydroxypropyl(meth)acrylat, Hydroxybutyl(meth)acrylat, Hydroxypentyl(meth)acrylat, Hydroxyhexyl(meth)acrylat.

Als Beispiele seien die Umsetzungsprodukte im molaren Verhältnis von 1 : 1 von Hydroxyethylmethacrylat mit Isophorondüsocyanat, Dicyclohexylmethan-4,4'-diisocyanat oder Hexamethylendiisocyanat genannt.

Die vorliegende Erfindung betrifft ferner die Verwendung bzw. den Einsatz einer vorstehend genannten Verbindung, vorzugsweise in einer der als bevorzugt bzw. besonders bevorzugt gekennzeichneten Ausgestaltungen, in einem erfindungsgemäßen bzw. erfindungsgemäß für die genannten Zwecke anzuwendenden Kompositmaterial, vorzugsweise in einem dentalen Kompositmaterial.

Die vorliegende Erfindung betrifft auch die Verwendung einer vorstehend genannten Verbindung der Struktur $Q(YZ_e)_b$ bzw. $Q(Y_xZ_e)_b$, vorzugsweise in einer der als bevorzugt bzw. besonders bevorzugt gekennzeichneten Ausgestaltungen, zur Herstellung eines Kompositmaterials, vorzugsweise eines dentalen Kompositmaterials, insbesondere eines dentalen Versiegelungsmaterials zur Versiegelung von Fissuren, zur Versiegelung von Grübchen und zur Versiegelung kariöser Läsionen.

Die vorliegende Erfindung betrifft zudem die genannten Verbindungen der Struktur $Q(YZ_e)_b$ bzw. $Q(Y_xZ_e)_b$, vorzugsweise in einer der als bevorzugt bzw. besonders bevorzugt gekennzeichneten Ausgestaltungen, als bzw. zur Verwendung bzw. zur Anwendung als dentales Versiegelungsmaterial, vorzugsweise als Versiegelungsmaterial zur Versiegelung von Fissuren, zur Versiegelung von Grübchen und zur Versiegelung kariöser Läsionen.

Die vorliegende Erfindung betrifft ferner ein Verfahren zur Herstellung einer Verbindung $Q(YZ_e)_b$ oder einer Mischung, umfassend zumindest eine erfindungsgemäß einzusetzende Verbindung $Q(YZ_e)_b$, die vorteilhaft in bzw. als Bestandteil (b1) eines erfindungsgemäßen bzw. erfindungsgemäß anzuwendenden Kompositmaterials eingesetzt werden kann, mit folgenden Schritten:

In einer ersten Reaktion Umsetzen von

A) einer Verbindung der Struktur $QG_b$, worin jedes G eine reaktive Gruppe bedeutet, die unabhängig von weiteren Gruppen G ausgewählt ist aus der Gruppe bestehend aus $(-CH_2)_n-NH_2$, $(-CH_2)_n-(OCH_2-CHR)_m-OH$, $(-CH_2)_n-NCO$ und $(-CH_2)_n-COOH$, vorzugsweise einer Verbindung der Struktur $QG_b$, worin jedes G eine reaktive Gruppe bedeutet, die unabhängig von weiteren Gruppen G ausgewählt ist aus der Gruppe bestehend aus $-NH_2$, $-CH_2NH_2$, -OH, $-CH_2OH$, -NCO, $-CH_2NCO$, und - COOH,
mit

B) zwei oder mehr gleichen oder verschiedenen Verbindungen $MZ_e$, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, $-NH_2$, -OH, -NCO und -COOH
zu einem ersten Umsetzungsprodukt,
gegebenenfalls in einer zweiten Reaktion Umsetzen des ersten Umsetzungsproduktes mit

C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
zu einem zweiten Umsetzungsprodukt
und gegebenenfalls in einer dritten Reaktion Umsetzen des zweiten Umsetzungsproduktes mit

D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.
wobei Q, b, Y, Z, und e jeweils die oben genannte Bedeutung haben, und wobei gilt:

- R bedeutet jeweils unabhängig von etwaigen weiteren R ein Wasserstoffatom oder einen Alkylrest; bevorzugt bedeutet R ein Wasserstoffatom oder einen linearen oder verzweigten Alkylrest mit 1 bis 6 C-Atomen; weiter bevorzugt bedeutet R ein Wasserstoffatom oder einen Methylrest;

- m ist eine natürliche Zahl ausgewählt aus der Gruppe der natürlichen Zahlen von 0 bis 10,

- jeder Index n ist eine natürliche Zahl, die unabhängig von etwaigen weiteren Indizes n ausgewählt ist aus der Gruppe bestehend aus 0 und 1,

wobei das Verhältnis der Gesamtzahl von NCO-Gruppen zu der Gesamtzahl von $-NH_2$, - OH und -COOH in der Gesamtzahl von Verbindungen gemäß A) und B) in der ersten, gegebenenfalls zweiten und gegebenenfalls dritten Reaktion größer als oder gleich 1 ist, bevorzugt im Bereich von 1,1 : 1 bis 5 : 1 liegt, weiter bevorzugt im Bereich von 1,25 : 1 bis 4 : 1 liegt, besonders bevorzugt im Bereich von 1,5 : 1 bis 3 : 1 liegt, und am meisten bevorzugt im Bereich von 2 : 1 bis 2,5 : 1 liegt.

Dabei liegt das Verhältnis der Gesamtzahl von umgesetzten NCO-Gruppen zu der Gesamtzahl von umgesetzten $-NH_2$, -OH und -COOH in der Gesamtzahl von Verbindungen gemäß A) und B) in der ersten, gegebenenfalls zweiten und gegebenenfalls dritten Reaktion bevorzugt im Bereich von 1,1 : 1 bis 5 : 1, weiter bevorzugt im Bereich von 1,25 : 1 bis 4 : 1, besonders bevorzugt im Bereich von 1,5 : 1 bis 3 : 1, und am meisten bevorzugt im Bereich von 2 : 1 bis 2,5 : 1.

Vorzugsweise erfolgt die Reaktion zu dem ersten Umsetzungsprodukt, zu dem zweiten Umsetzungsprodukt und/oder zu dem dritten Umsetzungsprodukt in Gegenwart eines Katalysators.

Bevorzugte Katalysatoren sind dabei tertiäre Amine oder Lewis Säuren, dabei wiederum bevorzugt Metallsalze höherer Fettsäuren, insbesondere Dibutylzinndilaurat oder Zinn(II)octoat.

Die Menge des Katalysators liegt dabei vorzugsweise im Bereich von 0,01 bis 2 Gew.-%, bevorzugt von 0,08 bis 1 Gew.-%, bezogen auf die Gesamtmenge der Reaktanden gemäß A) und B) sowie gegebenenfalls C) und gegebenenfalls D).

Die Reaktion zu dem ersten Umsetzungsprodukt, zu dem zweiten Umsetzungsprodukt und/oder zu dem dritten Umsetzungsprodukt erfolgt vorzugsweise in einem Temperaturbereich von 0 bis 160°C, bevorzugt im Bereich von 30 bis 140°C und besonders bevorzugt im Bereich von 60 bis 120°C. Die Umsetzung wird vorzugsweise bei Normaldruck (1013 mbar) ausgeführt.

Die vorstehenden bzw. nachfolgenden Ausführungen zu den als bevorzugt und besonders bevorzugt gekennzeichneten erfindungsgemäß einzusetzenden Verbindungen gelten für die bevorzugten und besonders bevorzugten Ausgestaltungen der erfindungsgemäßen Verfahren, Mischungen, Gemische, Erzeugnisse und Verwendungen jeweils entsprechend.

In einem weiteren Aspekt betrifft die vorliegende Erfindung eine Mischung umfassend ein, zwei oder mehr unterschiedliche erfindungsgemäß einzusetzende Verbindungen, herstellbar nach einem erfindungsgemäßen Verfahren.

Im Folgenden wird die Erfindung zunächst für Monomere umfassend tricyclische Strukturelemente Q am Beispiel von Tricyclo[5.2.1.0$^{2,6}$]decan (TCD) - Derivaten im Detail erläutert.

1.) Ausgehend vom Bis(hydroxymethyl)tricyclo[5.2.1.0$^{2,6}$]decan (TCD-Diol)

Bis(hydroxymethyl)tricyclo[5.2.1.0$^{2,6}$]decan ist kommerziell erhältlich, beispielsweise als Dicidolgemisch der isomeren Verbindungen 3,8-Bis(hydroxymethyl)tricyclo[5.2.1.0$^{2,6}$]decan und 4,8-Bis(hydroxymethyl)tricyclo[5.2.1.0$^{2,6}$]decan sowie 3,9-Bis(hydroxymethyl)tricyclo[5.2.1.0$^{2,6}$]decan und 4,9-Bis(hydroxymethyl)tricyclo[5.2.1.0$^{2,6}$]decan.

Die Bis(hydroxymethyl)tricyclo[5.2.1.0$^{2,6}$]decane können, ausgehend von Dicyclopentadien (Tricyclo[5.2.1.0$^{2,6}$]deca-3,8-dien), synthetisiert werden. Dicyclopentadien ist präparativ leicht in einer Diels-Alder Reaktion durch Dimerisierung von Cyclopentadien zugänglich. Hydroformylierung von Dicyclopentadien ergibt dann das Bis(hydroxymethyl)tricyclo[5.2.1.0$^{2,6}$]decan. Je nach Synthesepfad können gezielt an unterschiedlichen Positionen substituierte Bis(hydroxymethyl)tricyclo[5.2.1.0$^{2,6}$]decane erhalten werden. So werden in den Druckschriften JP 7-206740, EP 1 112 995 B1 oder EP 0 049 631 B1 Vorschriften angegeben, wie beispielsweise das 8,9-Bis(hydroxymethyl)tricyclo[5.2.1.0$^{2,6}$]decan herstellbar ist. Die DE 103 52 260 B3 beschreibt dagegen Verfahren zur Herstellung von 3(4), 8(9)-Bis(hydroxymethyl)tricyclo[5.2.1.0$^{2,6}$]decan. Die Notation der Positionen der Hydroxymethylgruppen 3(4), 8(9) bedeutet 3 oder 4, 8 oder 9.

Das im Handel erhältliche und als Ausgangsverbindung zur Herstellung erfindungsgemäß einzusetzender Monomere einsetzbare Bis(hydroxymethyl)tricyclo[5.2.1.0$^{2,6}$]decan enthält somit Hydroxymethylgruppen sowohl an den Positionen 3 oder 4 als auch in den Stellungen 8 oder 9. Es ist nun möglich, durch Anlagerung von Alkylenoxiden, im Allgemeinen in Mengen von 1 bis 10 mol, insbesondere von Ethylenoxid, Propylenoxid, Butylenoxid, etc. in Gegenwart basischer Katalysatoren nach bekannten Verfahren die entsprechenden Polyetherpolyole zu synthetisieren. Die EP 0 023 686 B1 enthält hierzu genaue Herstellvorschriften.

Die Umsetzung der 3(4), 8(9)-Bis(hydroxymethyl)tricyclo[5.2.1.0$^{2,6}$]decane mit Isocyanaten zu den entsprechenden Urethanen ist ebenfalls bekannt. So beschreibt die DE 35 22 006 A1 die Reaktion des 3(4), 8(9)-Bis(hydroxymethyl)tricyclo[5.2.1.0$^{2,6}$]decans mit 2-Isocyanatoethylmethacrylat. 2-Isocyanatoethylmethacrylat ist kommerziell erhältlich oder kann gemäß der Herstellvorschrift aus der DE 33 38 077 A1 durch Phosgenierung von Dihydrooxazinen synthetisiert werden.

Das erhaltene Reaktionsprodukt (Formel (1)) von 2-Isocyanatoethylmethacrylat mit 3(4), 8(9)-Bis(hydroxymethyl)tricyclo[5.2.1.0$^{2,6}$]decan weist in einer Formulierung nach Aushärtung einen niedrigen Reaktionsschrumpf und eine hohe mechanische Festigkeit auf.

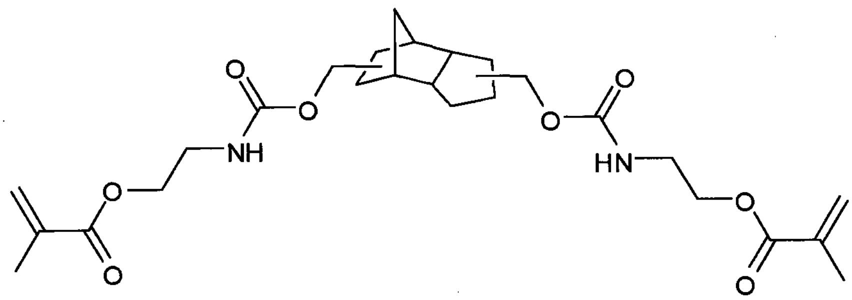

Formel (1)

Das Urethan der Formel (1) besitzt noch zwei reaktionsfähige Wasserstoffatome am Stickstoff, die nun in einer zweiten Umsetzungsstufe mit überschüssigem Isocyanat weiter abreagiert werden unter Bildung einer erfindungsgemäß einzusetzenden Verbindung. Es bildet sich hierbei zunächst das Allophanat der Formel (2) als tetrafunktionalisierte, radikalisch vernetzbare Verbindung. Auch dieses Monomer weist wiederum noch umsetzungsfähige Wasserstoffatome am Stickstoff auf, die erfindungsgemäß bei Reaktion mit weiterem Isocyanat das hexafunktionalisierte, radikalisch härtbare Allophanat der Formel (3) bilden.

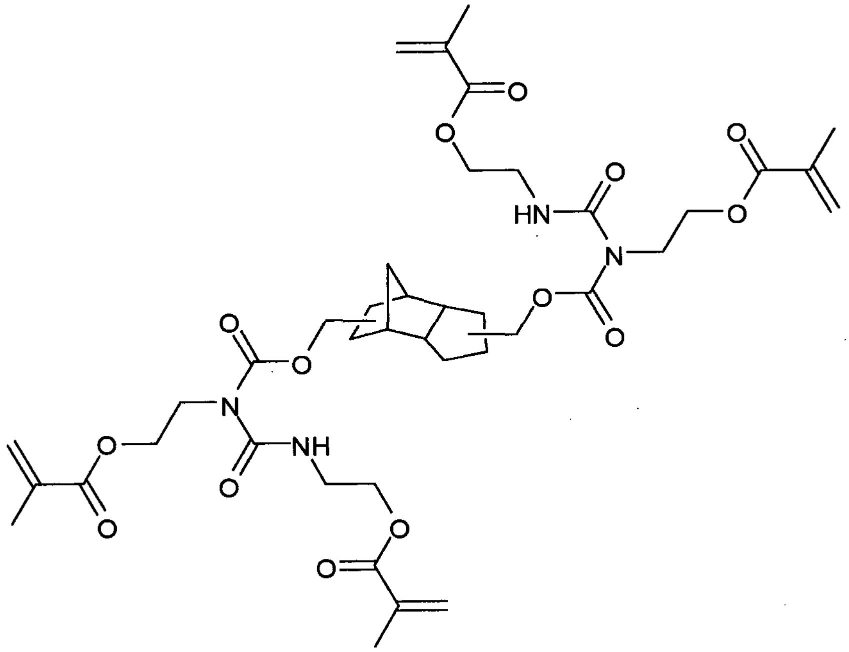

Formel (2)

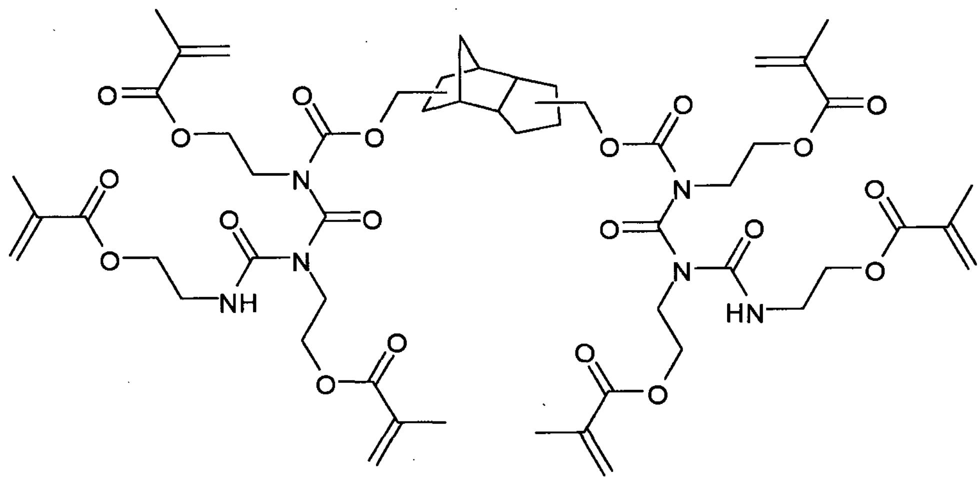

Formel (3)

Alternativ kann das 3(4), 8(9)-Bis(hydroxymethyl)tricyclo[5.2.1.$0^{2,6}$]decan auch mit Methacryloylisocyanat zur Reaktion gebracht werden. Methacryloylisocyanat ist kommerziell oder durch Umsetzung von Methacrylamid mit Oxalylchlorid erhältlich, wie in der EP 0 143 613 B1 beschrieben. Durch Umsetzung von 3(4), 8(9)-Bis(hydroxymethyl)tricyclo[5.2.1.$0^{2,6}$]decan mit Methacryloylisocyanat wird eine Verbindung der Formel (4) erhalten:

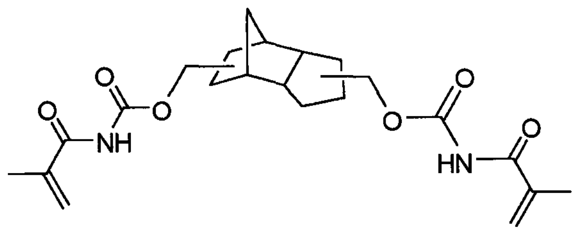

Formel (4)

Die verbleibenden reaktiven Wasserstoffatome am Stickstoff der Verbindung der Formel (4) können anschließend wiederum in Isocyanatreaktionen zu Allophanaten umgesetzt werden. Beispielhaft sei hier das Reaktionsprodukt mit 2-Isocyanatoethylmethacrylat (Formel (5)) gezeigt.

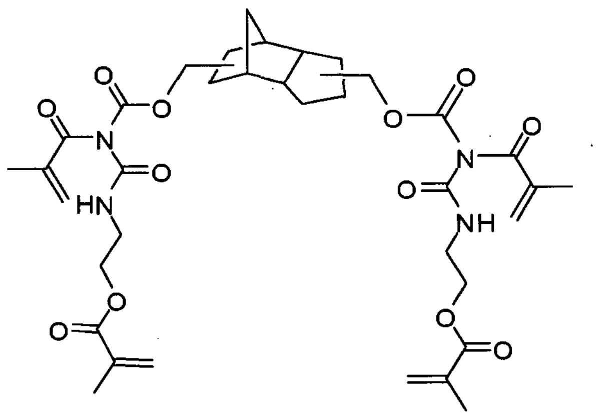

Formel (5)

2.) Ausgehend von 3(4), 8(9)-Bis(Carbonsäure)tricyclo[5.2.1.$0^{2,6}$]decan

Das 3(4), 8(9)-Bis(Carbonsäure)tricyclo[5.2.1.$0^{2,6}$]decan ist durch einfache Oxidation des kommerziell erhältlichen 3(4), 8(9)-Bis(formyl)tricyclo[5.2.1.$0^{2,6}$]decans herstellbar. Umsetzung der Dicarbonsäure mit 2-Isocyanatoethylmethacrylat ergibt das Amid der Formel (8):

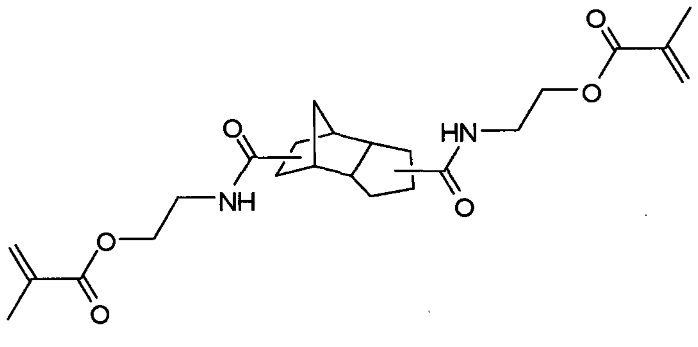

Formel (8)

Weitere Umsetzung der beiden reaktionsfähigen Amid-Wasserstoffatome des Amids der Formel (8) mit 2-Isocyanatoethylmethacrylat ergibt den Acylharnstoff der Formel (9).

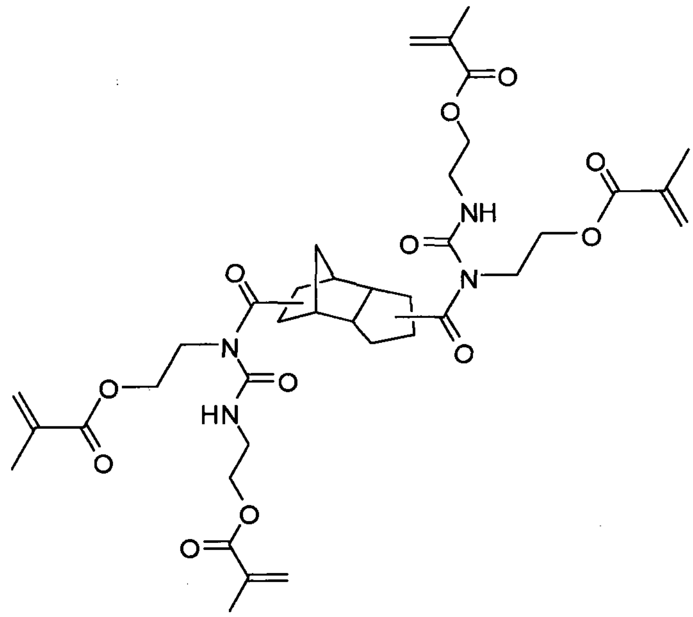

Formel (9)

Wird 3(4), 8(9)-Bis(Carbonsäure)tricyclo[5.2.1.0$^{2,6}$]decan mit Methacryloylisocyanat umgesetzt, ergibt sich das Imid der Formel (10). Die reaktiven Wasserstoffatome am Stickstoff können auch hier weiter in Isocyanatreaktionen umgesetzt werden.

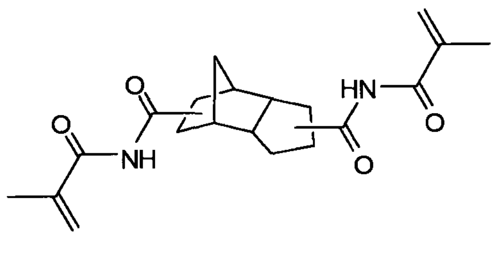

Formel (10)

3.) Ausgehend von 3(4), 8(9)-Bis(isocyanatomethyl)tricyclo[5.2.1.0$^{2,6}$]decan

Das 3(4), 8(9)-Bis(isocyanatomethyl)tricyclo[5.2.1.0$^{2,6}$]decan ist an sich bekannt und zählt zu den herkömmlichen, in industriellen Anwendungen eingesetzten Diisocyanatverbindungen (siehe hierzu die DE 37 03 120 A1 sowie die WO 2009/065873 A2). Die erfindungsgemäße Durchführung der zweiten Umsetzungsstufe der Isocyanat-Alkoholreaktion kann nicht nur ausgehend vom Tricyclodecandiol mit dem

Isocyanatoethylmethacrylat, sondern auch ausgehend vom Tricyclodecandiisocyanat und Hydroxyethylmethacrylat eingeleitet werden. Durch stöchiometrische Umsetzung der beiden Reaktanden erhält man das Urethan der Formel (11).

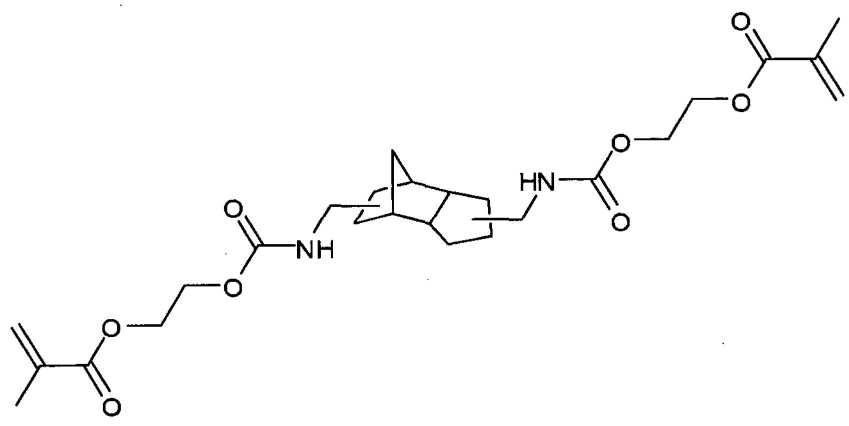

Formel (11)

Auch dieses Carbamat (Formel (11)) weist zwei reaktionsfähige Wasserstoffatome am Stickstoff auf, die mit einem Überschuss an Bis(isocyanatomethyl)tricyclo[5.2.1.0$^{2,6}$]decan zu dem Diisocyanat der Formel (12) weiter umgesetzt werden kann.

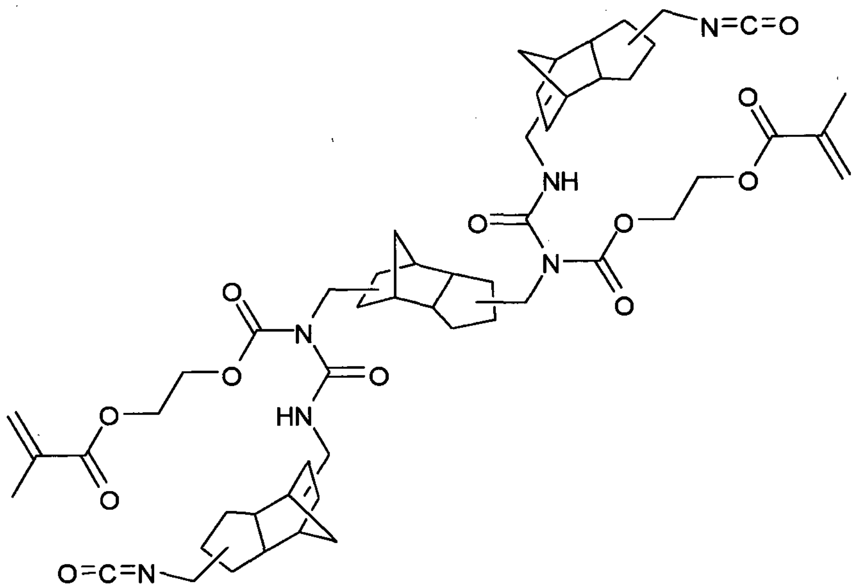

Formel (12)

Umsetzung des Allophanat-Diisocyanats (Formel (12)) mit Methacrylsäure liefert die Verbindung der Formel (13).

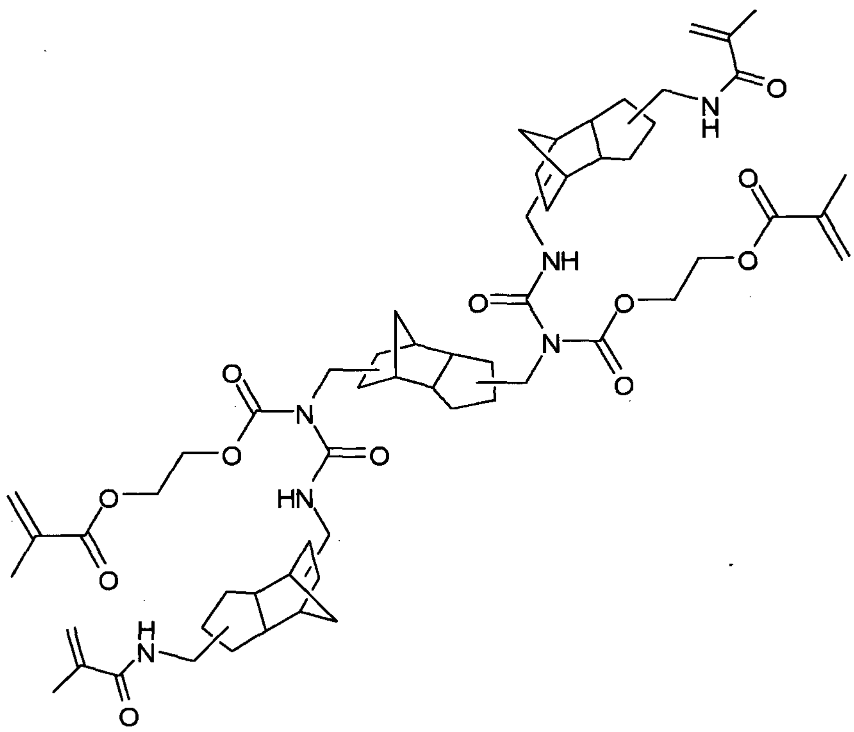

Formel (13)

Anstelle von Hydroxyethylmethacrylat können in den oben exemplarisch dargelegten Umsetzungen auch andere Hydroxylverbindungen von (Meth)acrylaten eingesetzt werden, wobei auch Gemische von Acrylaten und Methacrylaten verwendet werden können. So kann - analog dem obigen Beispiel - 3(4), 8(9)-Bis(isocyanatomethyl)-tricyclo[5.2.1.0$^{2,6}$]decan umgesetzt werden. Dabei bevorzugte Hydroxylverbindungen von (Meth)acrylaten sind:

Alkylenoxidmono(meth)acrylate wie beispielsweise Ethylenglykolmono(meth)acrylat, Diethylenglykolmono(meth)acrylat, Triethylenglykolmono(meth)acrylat, Tetraethylenglykolmono(meth)acrylat, etc., Polyalkylenoxidmono(meth)acrylate wie beispielsweise Polyethylenglykolmono(meth)acrylat, Polypropylenglykolmono(meth)acrylat, Polybutylenglykolmono(meth)acrylat, etc., Hydroxyalkylmono(meth)acrylate wie beispielsweise Hydroxyethyl(meth)acrylat, Hydroxypropyl(meth)acrylat, 2-Hydroxypropyl(meth)acrylat, Hydroxybutyl(meth)acrylat, 4-Hydroxybutyl(meth)acrylat, Hydroxypentyl(meth)acrylat, 3-Hydroxy-2,2-dimethylpropyl(meth)acrylat, Hydroxyhexyl(meth)acrylat, Hydroxyheptyl(meth)acrylat, Hydroxyoctyl(meth)acrylat, Hydroxynonyl(meth)acrylat, Hydroxydecyl(meth)acrylat, Hydroxyundecyl(meth)acrylat, Hydroxydodecyl(meth)acrylat, etc., Poly(ε-caprolacton)mono(meth)acrylat, Poly(γ-caprolacton)mono(meth)acrylat, etc., die Mono-, Di-, Tetra-, oder Penta(meth)acrylate mehrwertiger Alkohole, wie Glycerin, wie beispielsweise Glycerindi(meth)acrylat (2-Hydroxypropyl-1,3-di(meth)acrylat, 3-Hydroxypropyl-1,2-di(meth)acrylat), wie Trimethylolpropan, wie beispielsweise Trimethylolpropandi(meth)acrylat, wie Pentaerythrit, wie beispielsweise Pentaerythritol-tri(meth)acrylat, wie Dipentaerythrit, wie beispielsweise Dipentaerythritol-penta(meth)acrylat, wie Ditrimethylolpropantri(meth)acrylat, wie Neopentylglykol(meth)acrylat, die (Meth)acrylate von alkoxyliertem oder phenoxyliertem Glycerin, dabei vorzugsweise die (Meth)acrylate von ethoxyliertem, propoxyliertem, etc. Glycerin, Trimethylolpropan, Pentaerythrit, Dipentaerythrit, Ditrimethylolpropan, etc. sowie deren technische Gemische, Bisphenol-A-Glycidyl-(Meth)acrylat (Bis-GMA), Bisphenol-B-Glycidyl-(Meth)acrylat, Bisphenol-C-Glycidyl-(Meth)acrylat, Bisphenol-F-Glycidyl-(Meth)acrylat, alkoxyliertes Bisphenol-A-Glycidyl-(Meth)acrylat (z.B. ethoxyliertes Bisphenol-A-Glycidyl-(Meth)acrylat), etc..

Alle diese Verbindungen weisen sowohl (Meth)acrylatgruppen als auch Hydroxygruppen auf. Letztere können mit Isocyanatgruppen in der oben für die Reaktion zwischen Hydroxyethylmethacrylat und 3(4), 8(9)-Bis(isocyanatomethyl)-tricyclo[5.2.1.0$^{2,6}$]decan beschriebenen Weise reagieren. So lässt sich in einem einzigen Reaktionsschritt ein hoher Funktionalisierungsgrad erreichen.
3(4), 8(9)-Bis(isocyanatomethyl)tricyclo[5.2.1.0$^{2,6}$]decan kann mit 2-Carbonsäureethylmethacrylat zum entsprechenden Amid der Formel (16) umgesetzt werden.

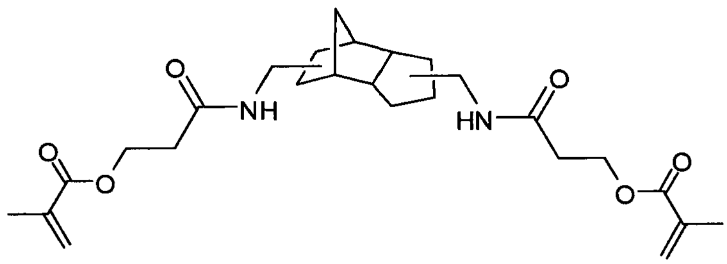

Formel (16)

Umsetzung des Amids der Formel (16) mit 2-Isocyanatoethylmethacrylat liefert den Acylharnstoff der Formel (17).

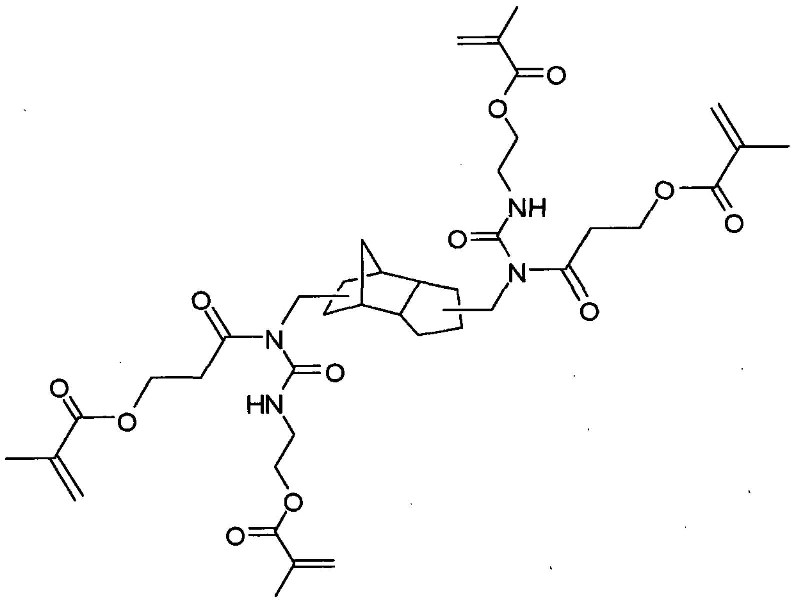

Formel (17)

Das Amid der Formel (16) kann auch mit einem Überschuss an 3(4), 8(9)-Bis(isocyanatomethyl)tricyclo[5.2.1.$0^{2,6}$]decan zum entsprechenden Isocyanat umgesetzt werden, wobei das gebildete Isocyanat mit Hydroxyethylmethacrylat weiter zum vernetzbaren Monomer der Formel (18) weiter umgesetzt wird.

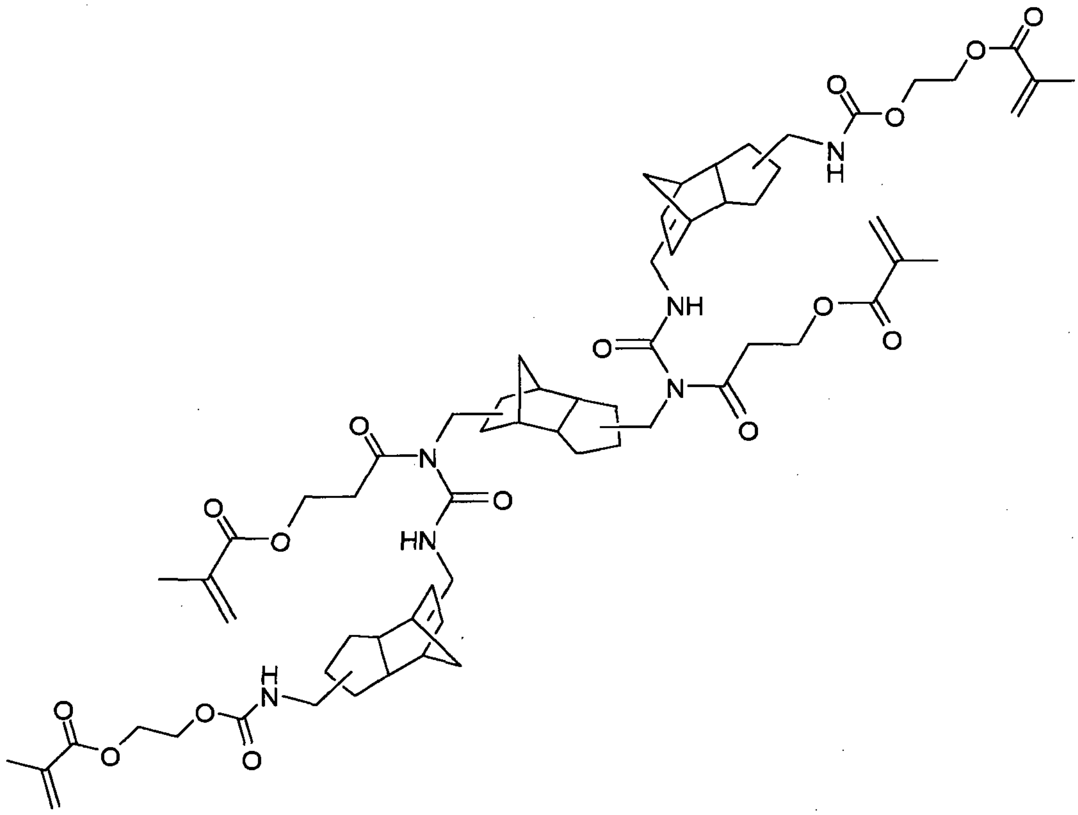

Formel (18)

Wird 3(4), 8(9)-Bis(isocyanatomethyl)tricyclo[5.2.1.0$^{2,6}$]decan mit 2-Methacryloyloxyethylhydrogensuccinat umgesetzt, so erhält man das Amid der Formel (19), das weiter mit 2-Isocyanatoethylmethacrylat zum Acylharnstoff der Formel (20) abreagiert wird.

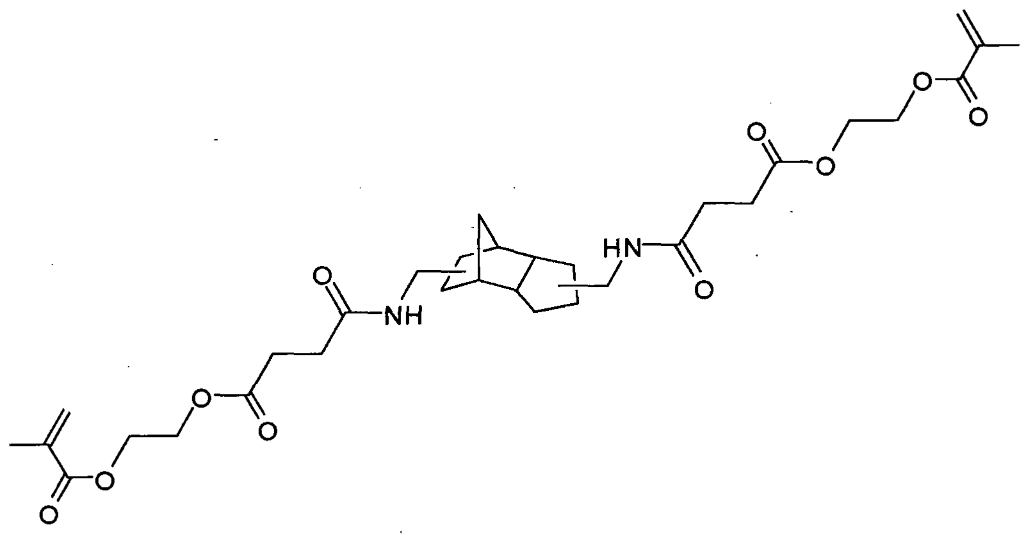

Formel (19)

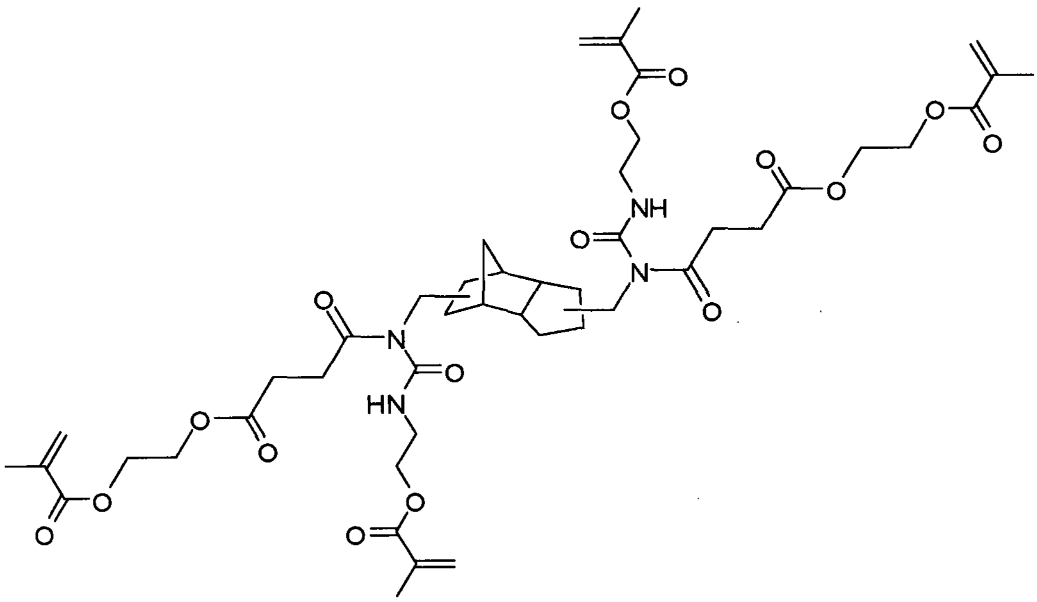

Formel (20)

Weitere geeignete Carbonsäuremethacrylate können durch Reaktionen von Di- oder Tetracarbonsäuremono- oder dianhydrid mit geeigneten OH-funktionalisierten, härtbaren Verbindungen wie beispielsweise 2-Hydroxyethylmethacrylat erhalten werden.

4.) Ausgehend von 3(4), 8(9)-Bis(Aminomethyl)tricyclo[5.2.1.$0^{2,6}$]decan

Das 3(4), 8(9)-Bis(Aminomethyl)tricyclo[5.2.1.$0^{2,6}$]decan ist an sich bekannt oder kann beispielsweise durch Umsetzung der entsprechenden Tosylate mit Ammoniak hergestellt werden. Reaktion des 3(4), 8(9)-Bis(Aminomethyl)tricyclo[5.2.1.$0^{2,6}$]decans mit 2-Isocyanatoethylmethacrylat ergibt die aus der EP 0 209 700 A2 bekannte Harnstoffverbindung der Formel (26).

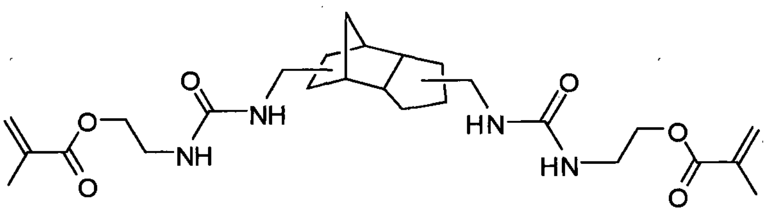

Formel (26)

Auch hier befinden sich noch aktive, reaktionsfähige Wasserstoffatome am Stickstoff, die mit einem Überschuss an Isocyanat beispielsweise zum Biuret der Formel (27) reagieren.

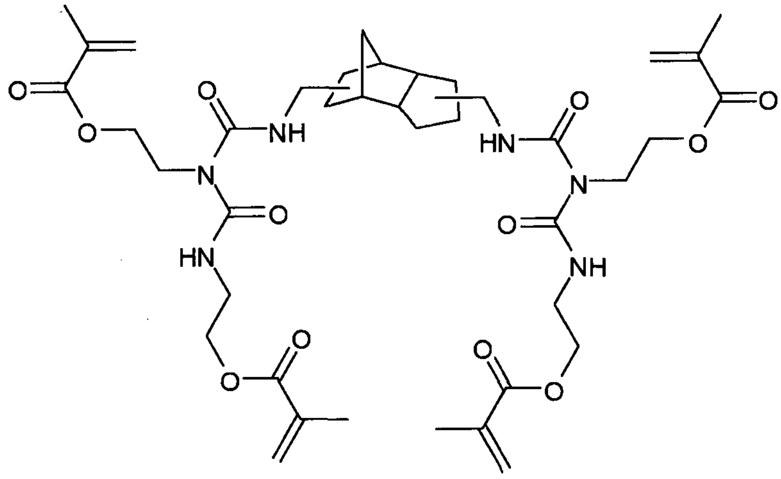

Formel (27)

Das 3(4), 8(9)-Bis(Aminomethyl)tricyclo[5.2.1.0$^{2,6}$]decan kann auch mit Methacryloylisocyanat zum entsprechenden Acylharnstoff umgesetzt werden. Die weitere Reaktion der verbleibenden reaktiven am Stickstoff befindlichen Wasserstoffatome mit Methacryloylisocyanat liefert das Biuret der Formel (28).

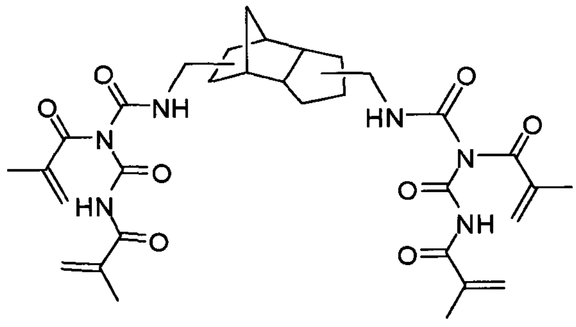

Formel (28)

Analog zu den vorstehend beschriebenen Monomeren, welche ein vom Tricyclo[5.2.1.0$^{2,6}$]decan abgeleitetes polyalicyclisches Strukturelement Q umfassen, können auch Monomere hergestellt werden, welche ein vom Tricyclo[3.3.1.1$^{3,7}$]decan (Adamantan) abgeleitetes polyalicyclisches Strukturelement Q umfassen. Als Beispiele seien folgende Reaktionsprodukte gezeigt:

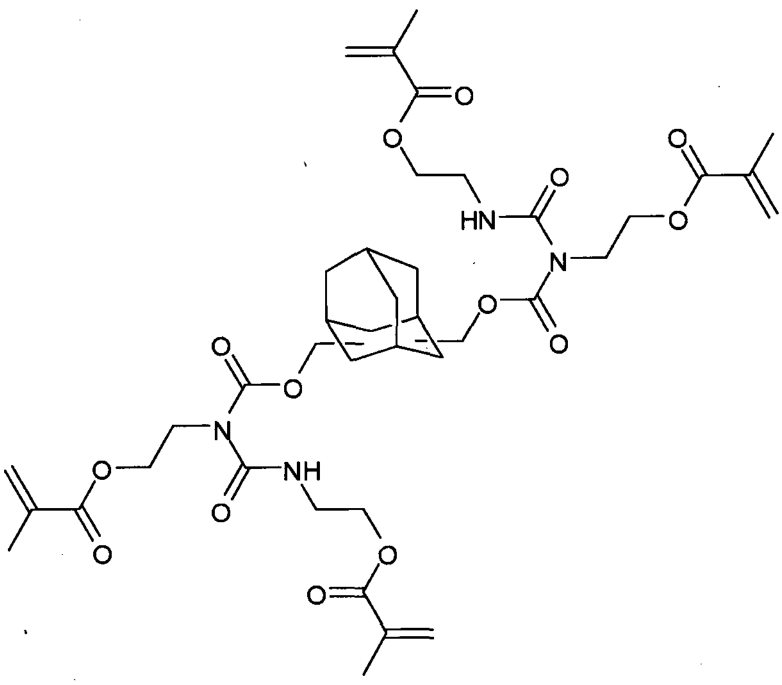

Formel (29)

Die Umsetzung der Verbindung der Formel (11) mit Diisocyanatoadamantan [(Bis(isocyanatomethyl)tricyclo[3.3.1.1$^{3,7}$]decan] liefert ein erfindungsgemäß einzusetzendes Monomer, dessen Molekül zwei voneinander verschiedene polyalicyclische Strukturelemente Q umfasst, wie die nachfolgende Strukturformel der Verbindung der Formel (69) zeigt.

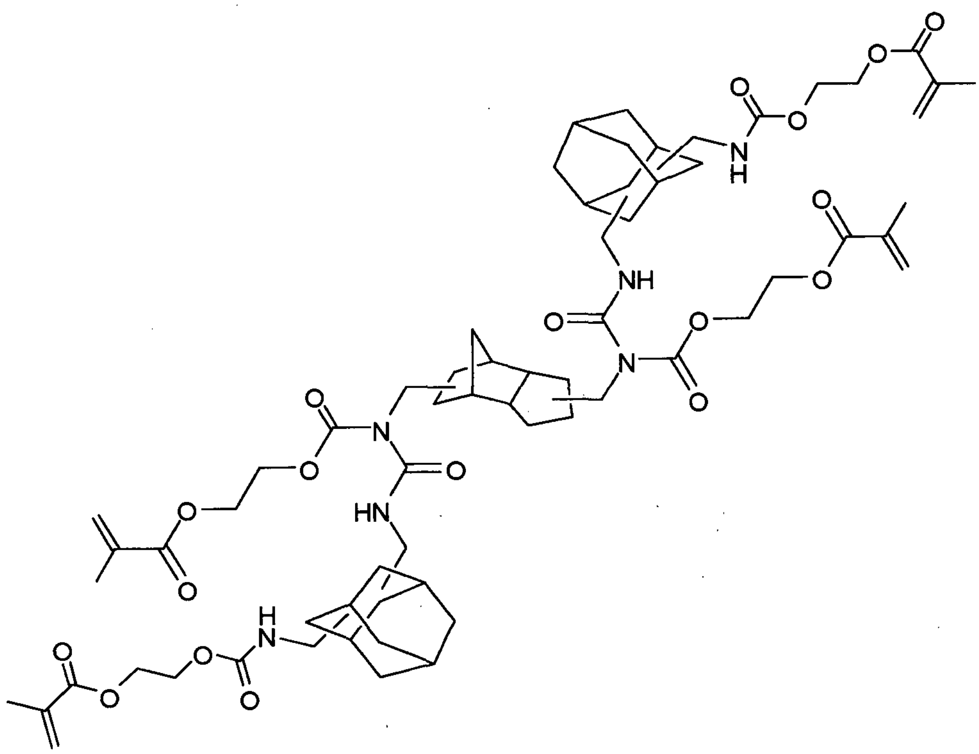

Formel (69)

Komponente (b2): ein, zwei oder mehr weitere radikalisch polymerisierbare Monomere aus der Gruppe bestehend aus Acrylaten und Methacrylaten, vorzugsweise der Gruppe der Methacrylate

Den optionalen zweiten, nicht zu Monomerkomponente (b1) zählbaren Bestandteil der matrixbildenden Monomerenkomponente bilden radikalisch polymerisierbare Monomere ausgewählt aus der Gruppe bestehend aus Acrylaten und Methacrylaten. Ihre Funktion innerhalb des erfindungsgemäßen bzw. erfindungsgemäß einzusetzenden Kompositmaterials besteht im Wesentlichen im Einstellen der Viskosität.

Erfindungsgemäß sind die Methacrylsäureester bzw. -diester wegen ihrer höheren biologischen Verträglichkeit gegenüber den entsprechenden Acrylsäureestern bzw. -diestern bevorzugt.

Die radikalisch polymerisierbaren Monomere der Komponente (b2) weisen vorzugsweise mindestens zwei ethylenische Gruppen auf.

In der Patentliteratur ist eine Vielzahl von Diacrylat- und Dimethacrylat-Monomeren genannt (beispielsweise auch in der DE 39 41 629 A1, insbesondere die Offenbarung im Bereich von Spalte 6, Zeile 15 bis Spalte 8, Zeile 10), die sich zum Einsatz in einem erfindungsgemäßen bzw. erfindungsgemäß anzuwendenden Kompositmaterial eignen.

In einem bevorzugten erfindungsgemäßen bzw. erfindungsgemäß einzusetzenden (anzuwendenden) Kompositmaterial enthält Komponente (b2) ein oder mehrere Dimethacrylat-Monomere gewählt aus der Gruppe bestehend aus Ethylenglykoldimethacrylat (EGDMA), 1,6-Hexandioldimethacrylat (HEDMA), Triethylenglykoldimethacrylat (TEDMA), 1,12-Dodecandioldimethacrylat (DODMA), ethoxyliertes Bisphenol-A-dimethacrylat, Polyethylenglykoldimethacrylat (PEGDMA), 7,7,9-Trimethyl-4,13-dioxo-5,12-diazahexadecan-1,16-dioxydimethacrylat (UDMA), Butandioldimethacrylat, Tetraethylenglykoldimethacrylat, Neopentylglykoldimethacrylat, 2-Hydroxypropyl-1,3-dimethacrylat, 3-Hydroxypropyl-1,2-dimethacrylat, Pentaerythritoldimethacrylat und Glycerindimethacrylat.

Erfindungsgemäß bevorzugt ist ein Kompositmaterial, wobei das oder die weiteren radikalisch polymerisierbaren Monomere (b2) ausgewählt sind aus der Gruppe bestehend aus Triethylenglykoldimethacrylat (TEDMA), Urethandimethacrylat (7,7,9-Trimethyl-4,13-dioxo-3,14-dioxa-5,12-diazahexadecan-1,16-dioxydimethacrylat, UDMA) und deren Mischungen. Mit diesen radikalisch polymerisierbaren Monomeren (b2) wurden in Kombination mit den Monomeren (b1), insbesondere mit den als bevorzugt bzw. besonders bevorzugt gekennzeichneten Monomeren (b1), besonders gute Effekte im Sinne der vorliegenden Erfindung erzielt. Die Anwesenheit von UDMA in einem erfindungsgemäßen

bzw. erfindungsgemäß anzuwendenden Kompositmaterial ist besonders bevorzugt.

Wird als Monomerkomponente (b2) eine Mischung enthaltend oder bestehend aus Triethylenglykoldimethacrylat (TEDMA) und Urethandimethacrylat (7,7,9-Trimethyl-4,13-dioxo-3,14-dioxa-5,12-diazahexadecan-1,16-dioxydimethacrylat, UDMA) eingesetzt, so verbessern sich die mechanischen Werte wie die Biegefestigkeit und insbesondere das E-Modul, während gleichzeitig die Wasseraufnahme und das Anfließverhalten (bestimmt mittels Kontaktwinkelmessung) immer noch deutlich unter den Werten eines Vergleichsmaterials aus dem Stand der Technik liegt. Dies gilt insbesondere, wenn als Monomerkomponente (b1) (Meth)acrylsäureester des Dihydroxymethyltricyclo[5.2.1.0$^{2,6}$]decans eingesetzt werden.

In eigenen Untersuchungen wurde ein Versiegelungsmaterial aus dem Stand der Technik eingesetzt mit einer Harzmatrix zusammengesetzt aus herkömmlichen dentalen Monomeren (UDMA 30%, TEDMA 37%, Bis-GMA 16% und ethoxyliertes Bis-GMA 17%, jeweils in Gew.-% bezogen auf die Monomermischung) sowie einem Füllstoffgemisch aus Glaskeramik in einer Gesamtmenge von 52% (in Gew.-% bezogen auf die Gesamtzusammensetzung) (siehe Vergleichsbeispiel im experimentellen Teil).

Wird die Polymermatrix eines erfindungsgemäßen bzw. erfindungsgemäß einzusetzenden (anzuwendenden) Kompositmaterials vollständig, d.h. ausschließlich, aus einem oder mehreren (Meth)acrylsäureestern von Bis(hydroxymethyl)tricyclo[5.2.1.0$^{2,6}$]decan gebildet (siehe untenstehendes Beispiel 2), sind überraschenderweise sowohl die Wasseraufnahme als auch der Kontaktwinkel am getrockneten Zahnschmelz erheblich geringer als bei einem Kompositmaterial aus dem Stand der Technik, wobei die mechanischen Werte (Biegefestigkeit und E-Modul) zwar etwas reduziert werden, jedoch immer noch akzeptable Werte aufweisen.

Wird die Polymermatrix eines erfindungsgemäßen bzw. erfindungsgemäß einzusetzenden (anzuwendenden) Kompositmaterials aus (i) einem oder mehreren (Meth)acrylsäureestern des Bis(hydroxymethyl)tricyclo[5.2.1.0$^{2,6}$]decans und (ii) Triethylenglykoldimethacrylat (TEDMA) und/oder Urethandimethacrylat (7,7,9-Trimethyl-4,13-dioxo-3,14-dioxa-5,12-diazahexadecan-1,16-dioxydimethacrylat, UDMA) gebildet, so verbessern sich die mechanischen Werte wie die Biegefestigkeit und insbesondere das E-Modul, während die Wasseraufnahme und das Anfließverhalten (wiedergegeben durch die Kontaktwinkelmessung) akzeptabel sind und immer noch deutlich unter den Werten des Vergleichsmaterials aus dem Stand der Technik liegen.

Sehr gute Ergebnisse werden erzielt, wenn als Monomerkomponente (b2) eine Mischung von TEDMA und UDMA eingesetzt wird, wobei das Gewichtsverhältnis von TEDMA zu UDMA im Bereich 4 : 1 bis 1 : 4 liegt, vorzugsweise im Bereich 3 : 1 bis 1 : 3, bevorzugt im Bereich 2 : 1 bis 1 : 2, besonders bevorzugt im Bereich 3 : 2 bis 2 : 3 und ganz besonders bevorzugt im Bereich 4 : 3 bis 3 : 4.

Besonders gute Ergebnisse werden erzielt, wenn das Gewichtsverhältnis der Gesamtmenge an (Meth)acrylsäureestern des Bis(hydroxymethyl)tricyclo[5.2.1.0$^{2,6}$]decans (Monomerkomponente (b1)) zu der Gesamtmenge der Mischung von TEDMA und UDMA (Monomerkomponente (b2)) im Bereich 4 : 1 bis 1 : 3 liegt, vorzugsweise im Bereich 3 : 1 bis 1 : 2, bevorzugt im Bereich 2 : 1 bis 2 : 3, besonders bevorzugt im Bereich 3 : 2 bis 2 : 3 und ganz besonders bevorzugt im Bereich 4 : 3 bis 3 : 4, wobei in der Monomerkomponente (b2) wiederum vorzugsweise die oben genannten Gewichtsverhältnisse von TEDMA zu UDMA eingestellt sind.

Bisphenol-A-Glycidyl-Methacrylat (Bis-GMA) kann zwar eingesetzt werden, vorzugsweise enthält ein erfindungsgemäß einzusetzendes bzw. anzuwendendes dentales Kompositmaterial, jedoch nicht die Verbindung Bis-GMA. Vorzugsweise ist ein erfindungsgemäß einzusetzendes bzw. anzuwendendes dentales Kompositmaterial, frei von sämtlichen Verbindungen mit einem Bisphenol-A-Strukturelement.

Die radikalisch polymerisierbaren Monomere der Komponente (b2), die somit nicht zu Komponente (b1) zählen, können auch Hydroxylverbindungen sein. Dabei können alle in der Dentalchemie üblicherweise eingesetzten Hydroxylverbindungen von Acrylaten oder Methacrylaten eingesetzt werden. Bevorzugt sind Hydroxylverbindungen von Methacrylaten, dabei wiederum bevorzugt 2-Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat, 2,2-bis[4-[3-methacryloyloxy-2-hydroxypropoxy]phenyl]propan.

Als weiterer Bestandteil können auch lichthärtbare Acrylat- oder Methacrylat-Monomere auf Basis von Polysiloxanen verwendet werden, wie sie beispielsweise in der DE 199 03 177 oder in der DE 44 16 857 beschrieben sind.

Ein erfindungsgemäß einzusetzendes bzw. anzuwendendes dentales Kompositmaterial, kann ferner in Komponente (b2) ein oder mehrere säuregruppenhaltige Acrylate- und/oder Methacrylat-Monomere enthalten. Solche säuregruppenhaltigen Monomere können vorzugsweise eine Carbonsäure-, eine Phosphorsäure-, eine Phosphonsäure-, eine Sulfonsäure- und/oder eine Thiophosphorsäurefunktion aufweisen. Das Monomer kann, eine oder eine Vielzahl von Säurefunktionen in einem Molekül enthalten.

Geeignete eine Phosphorsäuregruppe enthaltende Monomere sind beispielsweise 2-(Meth)acryloyloxyethyldihydrogenphosphat, Bis[2-(meth)acryloyloxyethyl]hydrogenphosphat, 2-(Meth)acryloyloxyethylphenylhydrogenphosphat, 6-(Meth)-acryloyloxyhexyldihydrogenphosphat, 10-(Meth)acryloyloxydecyldihydrogenphosphat (MDP), 6-(Meth)-acryloyloxyhexylphenylhydrogenphosphat, 1 0-(Meth)acryloyloxydecyldihydrogenphosphat, 1,3-Di-(meth)acryloyloxypropan-2-dihydrogenphosphat, 1,3-Di-(meth)acryloyloxypropan-2-phenylhydrogenphosphat und Bis[5-(2-(meth)acryloyloxyethoxycarbonyl)-heptyl]hydrogenphosphat.

Geeignete eine Carbonsäuregruppe enthaltende Monomere sind beispielsweise 4-(Meth)acryloxyethyltrimellithsäure (4-MET), 4-(Meth)acryloxyethyltrimellithsäureanhydrid (4-META), 4-(Meth)acryloxydecyltrimellithsäure, 4-(Meth)acryloxydecyltrimellithsäureanhydrid, 11-(Meth)acryloyloxy-1,1-undecandicarbonsäure, 1,4-Di(meth)acryloyloxypyromellithsäure, 2-(Meth)acryloyloxyethylmaleinsäure, 2-(Meth)acryloyloxyethylphthalsäure und 2-(Meth)acryloyloxyethylhexahydrophthalsäure.

Weitere geeignete säuregruppentragende Monomere sind beispielsweise in der EP 0 980 682 A1 (insbesondere Absätze [0059] bis [0065]) oder der EP 0 948 955 A1 (insbesondere Absätze [0031] bis [0034]) genannt.

Ferner können auch die Phosphorsäureester mit Glycerindimethacrylat oder mit Hydroxyethylmethacrylat oder mit Hydroxypropylmethacrylat verwendet werden.

Die genannten Monomere können einzeln oder in Gemischen eingesetzt werden.

Bestandteil (c): Initiatoren und/oder Katalysatoren

Bevorzugte erfindungsgemäß einzusetzende bzw. anzuwendende Kompositmaterialien sind lichthärtbar (photohärtbar) und umfassen Lichthärtungsinitiatoren. Beispiele für einen Lichthärtungsinitiator schließen Substanzen ein, die nur photosensibilisierend wirken sowie Kombinationen aus Sensibilisator und Beschleuniger.

Beispiele für Photosensibilisatoren sind alpha-Diketone, Benzoinalkylether, Thioxanthone, Benzophenone, Acylphosphinoxide, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1 oder in der DE 39 41 629 C2.

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2.

Weitere geeignete Initiatoren sowie Initiatorkombinationen sind in der DE 601 16 142 beschrieben.

Die im Rahmen der vorliegenden Erfindung verwendbaren Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung eines erfindungsgemäßen bzw. erfindungsgemäß anzuwendenden bzw. einzusetzenden Kompositmaterials, insbesondere ein erfindungsgemäßes bzw. erfindungsgemäß anzuwendenden bzw. einzusetzenden dentalen Kompositmaterials, bewirken können.

Das Absorptionsmaximum von Campherchinon (CC) liegt bei ca. 470 nm und somit im Bereich des blauen Lichts. Campherchinon (CC) zählt zu den $PI_2$-Initiatoren und wird regelmäßig zusammen mit einem Coinitiator eingesetzt.

Vorzugsweise enthält ein erfindungsgemäß anzuwendendes bzw. einzusetzendes Kompositmaterial die Kombination eines alpha-Diketons und eines aromatischen tertiären Amins, bevorzugt ist die Kombination von Campherchinon (CC) und Ethyl-p-N,N-dimethylaminobenzoat (DABE).

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyldiphenylphosphinoxid. Bezüglich der Strukturen geeigneter Phosphinoxide zum Einsatz in einem erfindungsgemäßen bzw. erfindungsgemäß anzuwendenden oder einzusetzenden Kompositmaterial wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen.

Die in diesen Druckschriften angegebenen Phosphinoxide eigenen sich besonders allein oder in Kombination mit dem System "alpha-Diketon/Amin" als Photopolymerisationsinitiatorsystem in einem erfindungsgemäßen bzw. erfindungsgemäß einzusetzenden oder anzuwendenden Kompositmaterial.

Alternativ können auch Boratsalze, wie sie beispielsweise in US 4,772,530, US 4,954,414, US 4,874,450, US 5,055,372 und US 5,057,393 beschrieben sind, als Photoinitiatoren Verwendung finden.

Weitere geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. II, Elsevier Applied Science, London, New York 1993 beschrieben.

Dem Fachmann sind diverse Initiatoren für eine chemische Aushärtung bekannt. Es sei insoweit exemplarisch auf die EP 1 720 506 verwiesen.

Bevorzugte Initiatoren zur chemischen Härtung sind Benzoylperoxid, Lauroylperoxid insbesondere Dibenzoylperoxid in Kombination mit Aminen wie N,N-Dimethyl-p-toluidin, N,N-Dihydroxyethyl-p-toluidin sowie strukturverwandten Aminen.

Die Peroxide und die Amine werden dabei auf zwei unterschiedliche Komponenten des Dentalmaterials aufgeteilt. Beim Mischen der aminhaltigen Komponente (sogenannte Basispaste) mit der peroxidhaltigen Komponente (sogenannte Initiator- oder Katalysatorpaste) wird durch die Reaktion von Amin und Peroxid (Redoxreaktion) die radikalische Reaktion initiiert.

Dualhärtende Systeme umfassen eine Kombination aus Photoinitiatoren und Initiatoren zur chemischen Aushärtung.

Beispielsweise kann die Basispaste zusätzlich einen Photoinitiator enthalten, so dass die Basispaste entweder allein als lichthärtender oder zusammen mit der Initiatorpaste als licht- und selbsthärtender Dentalwerkstoff eingesetzt werden kann.

Neben den oxidativ wirksamen organischen Peroxidverbindungen können als Redoxsysteme auch Barbitursäuren bzw. Barbitursäurederivate sowie Malonylsulfamide verwendet werden.

Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in der EP 1 839 640 sowie in DE 1495520, WO 02/092021 oder in WO 02/092023.

Geeignete Malonylsulfamide sind in der EP 0 059 451 beschrieben. Bevorzugte Verbindungen sind dabei 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Diocytyl-4-isobutylmalonylsulfamid.

Ferner können Schwefelverbindungen in der Oxidationsstufe +2 oder +4 wie Natriumbenzolsulfinat oder Natriumparatoluolsulfinat eingesetzt werden.

Zur Beschleunigung der Aushärtung kann die Polymerisation in Gegenwart von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfernaphthenat.

Bestandteil (d): optionale weitere Additive

Ein erfindungsgemäß einzusetzendes bzw. anzuwendendes Kompositmaterial umfasst in manchen Fällen ein oder mehrere weitere Additive.

Diese Additive können verschiedene Funktionen haben. Übliche Additive für den Einsatz in dentalen Kompositmaterialien sind dem Fachmann bekannt, je nach gewünschter Funktion wird er das oder die geeigneten Additive auswählen. Beispielhaft sind im folgenden typische Additive und ihre Funktionen beschrieben.

Lichthärtbare dentale Kompositmaterialien, wie sie erfindungsgemäß bevorzugt sind, enthalten vorzugsweise einen oder mehrere Inhibitoren, auch Stabilisatoren genannt. Diese werden üblicherweise zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität der lichthärtbaren dentalen Zusammensetzung. Gängige Inhibitoren sind Phenolderivate wie Hydrochinonmonomethylether (HQME) oder 2,6-Di-tert.butyl-4-methylphenol (BHT). Weitere Inhibitoren wie und tert.-Butylhydroxyanisol (BHA), 2,2 Diphenyl-1-picrylhydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Derivate dieser Verbindung werden in der EP 0 783 880 B1 beschrieben. Alternative Inhibitoren sind in der DE 101 19 831 A1 oder in der EP 1 563 821 A1 angegeben.

Ein erfindungsgemäß bevorzugtes dentales Kompositmaterial umfasst somit als Additiv ein oder mehrere Polymerisationsinhibitoren zur Erhöhung der Lagerstabilität des Kompositmaterials, vorzugsweise ausgewählt aus der Gruppe bestehend aus Hydrochinonmonomethylether (HQME), Phenolen, dabei vorzugsweise 2,6-Di-tert.butyl-4-methylphenol (BHT) und tert.-Butylhydroxyanisol (BHA), 2,2-Diphenyl-1-picrylhydrazyl-Radikalen, Galvinoxyl-Radikalen, Triphenylmethyl-Radikalen, 2,3,6,6,-Tetramethylpiperidinyl-1-oxylradikal (TEMPO) sowie dessen Derivaten und Phenothiazin sowie dessen Derivaten.

Ein erfindungsgemäß bevorzugtes dentales Kompositmaterial, das zur Anwendung in einem therapeutischen dentalen Verfahren als Versiegelungsmaterial zur Versiegelung von Fissuren und/oder Grübchen und/oder kariösen Läsionen besonders geeignet ist, umfasst als Additiv eine oder mehrere fluoridabgebende Substanzen, dabei vorzugsweise Natriumfluorid und/oder Aminfluoride.

UV-Absorber, die beispielsweise durch ihre konjugierten Doppelbindungssytemen und aromatischen Ringe befähigt sind, UV Strahlung zu absorbieren, sind in manchen Fällen Bestandteil eines erfindungsgemäß anzuwendenden dentalen Kompositmaterials. Beispiele an UV-Absorbern sind 2-Hydroxy-4-methoxybenzophenon, Salicylsäurephenylester 3-(2'-Hydroxy-5'-methylphenyl)-benzotriazol oder Diethyl-2,5-dihydroxy-terephthalat.

Da die Zähne möglichst naturgetreu wiederherzustellen sind, ist es notwendig, erfindungsgemäß anzuwendende dentale Kompositmaterialien in den unterschiedlichsten Farbtönen bereitzustellen. Man benutzt zu diesem Zweck in der Regel anorganische Farbstoffe sowie organische Pigmente in sehr geringen Mengen, die somit in bevorzugten

Ausgestaltungen als Additiv eingesetzt werden.

Weitere optionale Additive sind Aromastoffe.

Bevorzugte erfindungsgemäße bzw. erfindungsgemäß anzuwendende Kompositmaterialien sind solche, die vorstehend als bevorzugt bezeichnete Bestandteile umfassen. Hierbei sind eine Vielzahl von Kombinationen vorteilhaft. Besonders bevorzugt sind neue Kompositmaterialien, welche in bzw. als Komponente (b1) bevorzugte Verbindungen der vorstehend diskutierten Struktur $Q(Y_xZ_e)_b$ mit x = 1 umfassen.

Bevorzugt ist also ein Kompositmaterial bestehend aus oder umfassend:

(a) als Füllstoffkomponente eine Gesamtmenge an Füllstoffen im Bereich von 0,5 bis 60 Gew.-%, bezogen auf die Gesamtmasse des Kompositmaterials, wobei die Gesamtmenge an Füllstoffen eine Mischung von Füllstoffen ist umfassend

(a1) eine Gesamtmenge im Bereich von 0,5 bis 60 Gew.-% an nicht agglomerierten, organisch oberflächenmodifizierten Nanopartikeln mit einer mittleren Partikelgröße kleiner 200 nm (vorzugsweise kleiner 60 nm) und

(a2) eine Gesamtmenge im Bereich von 0 bis 59,5 Gew.-% an Mikropartikeln mit einer mittleren Partikelgröße von 0,4 μm bis 10 μm sowie

(a3) gegebenenfalls weiteren Füllstoffen,
wobei die Gewichtsprozentangaben für die Komponenten (a1) und (a2) jeweils auf die Gesamtmasse des Kompositmaterials bezogen sind,

(b) als Monomerenkomponente eine Gesamtmenge an polymerisierbaren Monomeren im Bereich von 39 bis 98,5 Gew.-%, bezogen auf die Gesamtmasse des Kompositmaterials, wobei die Gesamtmenge an polymerisierbaren Monomeren umfasst

(b1) ein, zwei oder mehr Monomere ausgewählt aus der Gruppe bestehend aus Verbindungen (Monomere) der Struktur $Q(Y_xZ_e)_b$, wobei gilt:

- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten $Y_xZ_e$ substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sind,

- b ist eine natürliche Zahl ausgewählt aus der Gruppe der natürlichen Zahlen 1, 2, 3 und 4,

- jedes Z bedeutet ein Strukturelement, das unabhängig von etwaigen weiteren Strukturelementen Z ausgewählt ist aus der Gruppe bestehend aus

$-O-(C=O)-CH=CH_2$, $-O-(C=O)-C(CH_3)=CH_2$,

$-(C=O)-CH=CH_2$, $-(C=0)-C(CH_3)=CH_2$

$-CH=CH_2$, $-C(CH_3)=CH_2$ und $-O-CH=CH_2$,

- jeder Index e ist eine natürliche Zahl, die unabhängig von etwaigen weiteren Indizes e ausgewählt ist aus der Gruppe der natürlichen Zahlen 1, 2, 3 und 4,

- jeder Index x bedeutet unabhängig von etwaigen weiteren Indizes x 0 oder 1,

- jedes Y bedeutet in der Struktur $Q(Y_xZ_e)_b$ bei x = 1 ein Strukturelement, welches das polyalicyclische Strukturelement Q mit e Strukturelementen Z verbindet, wobei jedes Y unabhängig von etwaigen weiteren Strukturelementen Y gewählt ist,

(b2) optional ein, zwei oder mehr weitere radikalisch polymerisierbare Monomere aus der Gruppe bestehend aus Acrylaten und Methacrylaten, vorzugsweise der Gruppe der Methacrylate, wobei das oder die weiteren radikalisch polymerisierbaren Monomere keine Verbindungen (Monomere) der vorstehend definierten Struktur $Q(Y_xZ_e)_b$ sind,

(c) ein oder mehrere Initiatoren und/oder Katalysatoren, vorzugsweise in einer Menge von bis zu 1 Gew.-%, bezogen auf die Gesamtmasse des Kompositmaterials, sowie

(d) gegebenenfalls ein oder mehrere Additive zur Anwendung in einem therapeutischen dentalen Verfahren als Versiegelungsmaterial zur Versiegelung von Fissuren und/oder Grübchen und/oder kariösen Läsionen wobei das, eines, zwei oder mehr der Monomere der Komponente (b1) ausgewählt ist bzw. sind aus der Gruppe bestehend aus Verbindungen (Monomere) der Struktur $Q(Y_xZ_e)_b$ mit x = 1 mit ein, zwei, drei, vier oder mehr funktionellen Gruppen, welche ausgewählt sind aus der Gruppe bestehend aus N-Acylharnstoff, Allophanat und Biuret, wobei gilt:

- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei keines, eines, zwei oder mehr der nicht durch Substituenten $YZ_e$ substituierten Wasserstoffatome dieses polyalicyclischen Strukturelements Q durch Alkylgruppen, Alkoxygruppen, Halogenatome oder Trifluormethylgruppen substituiert ist bzw. sind;

- b ist eine natürliche Zahl ausgewählt aus der Gruppe der natürlichen Zahlen 2, 3, 4;

- jedes Z bedeutet ein Strukturelement, das unabhängig von etwaigen weiteren Strukturelementen Z ausgewählt ist aus der Gruppe bestehend aus

$$-O-(C=O)-CH=CH_2,\ -O-(C=O)-C(CH_3)=CH_2,$$

$$-(C=O)-CH=CH_2,\ -(C=O)-C(CH_3)=CH_2,$$

$$-CH=CH_2,\ -C(CH_3)=CH_2 \text{ und } -O-CH=CH_2,$$

- jeder Index e ist eine natürliche Zahl, die unabhängig von etwaigen weiteren Indizes e ausgewählt ist aus der Gruppe der natürlichen Zahlen 1, 2, 3 und 4;

- jedes Y bedeutet ein Strukturelement, welches in der Struktur $Q(Y_xZ_e)_b$ mit x = 1 das polyalicyclische Strukturelement Q mit e Strukturelementen Z verknüpft und ein Strukturelement enthält oder aus diesem besteht, ausgewählt aus der Gruppe bestehend aus

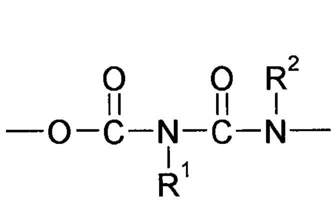
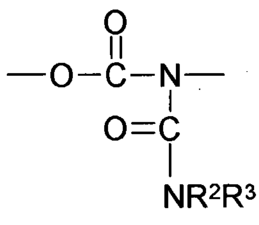
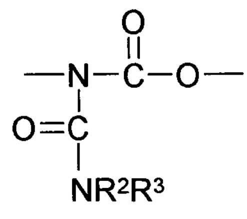

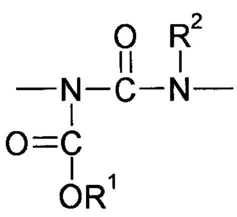
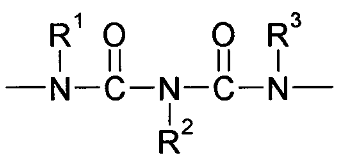
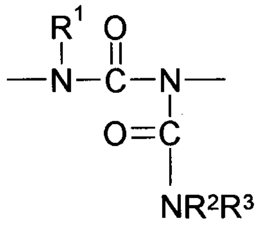

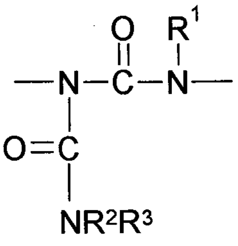

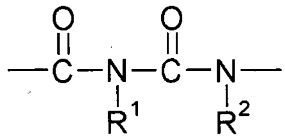

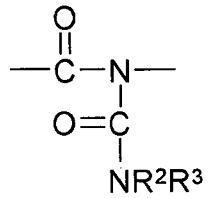

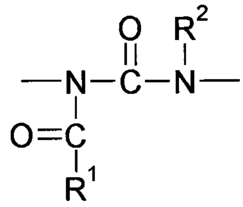

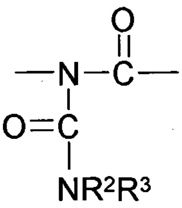

wobei $R^1$, $R^2$ und $R^3$ sonstige Reste der Verbindung bedeuten, wobei die jeweils im Formelbild links angeordnete Bindung dem Strukturelement Q und die rechts angeordnete Bindung dem Strukturelement Z näher ist.

Hinsichtlich bevorzugter Ausgestaltungen solcher bevorzugten Kompositmaterialien gelten die vorstehenden Ausführungen zu bevorzugten Bestandteilen.

Die vorliegende Erfindung betrifft auch ein Verfahren zum Behandeln oder zur Prophylaxe einer Zahnerkrankung, dadurch gekennzeichnet, dass ein erfindungsgemäß anzuwendendes Kompositmaterial, vorzugsweise in einer der als bevorzugt angegebenen Ausgestaltungen, als Versiegelungsmaterial zur Versiegelung von Fissuren und/oder Grübchen und/oder kariösen Läsionen eingesetzt wird.

Ausführungsbeispiele

Anhand der folgenden Beispiele wird die Erfindung weiter erläutert. Sofern nicht anders angegeben, beziehen sich alle Angaben auf das Gewicht.

Verwendete Abkürzungen und Materialien

TEDMA = Triethylenglykoldimethacrylat

UDMA = 7,7,9-Trimethyl-4,13-dioxo-5,12-diazahexadecan-1,16-dioxydimethacrylat

DABE = N,N-Dimethyl-p-aminobenzoesäure

CC = Campherchinon

BHT = 2,6-Di-tert-butyl-4-methylphenol (Stabilisator)

Herstellung von Kompositmaterialien

Erfindungsgemäß einzusetzende bzw. anzuwendende Kompositmaterialien sowie ein nicht erfindungsgemäßes Vergleichsmaterial wurden jeweils wie folgt hergestellt:

Das Monomer (b1) sowie gegebenenfalls das oder die Monomer(e) (b2), Initiatoren (c) und Additive (d) werden zunächst in einem Kunststoffbehälter mittels eines KPG-Rührers homogenisiert. Anschließend werden der oder die Füllstoffe der Komponente (a) zugegeben und durch inniges Vermischen mit einem Doppelplaneten-Mischer eine homogene Paste hergestellt. Gegebenenfalls kann die Homogenisierung noch durch anschließendes Walzen auf einem 3-Walzenstuhl erfolgen.

Messmethoden:

Bestimmung der Abrasion (ACTA)

Die Drei-Medien Acta Abrasion wurde gemäß J. Dent. Suppl. 1, 1994, 22, 21-27 nach 200000 Zyklen ermittelt.

Biegefestigkeit

Die Biegefestigkeit wurde analog zu ISO 4049 bestimmt. Dazu wurden das jeweilige zu untersuchende Material luftblasenfrei in entsprechende Teflonformen eingefüllt, mit Folie und Glasplatte bedeckt und mit einer Schraubzwinge wurden die Überschüsse herausgepresst. Die Probekörper wurden 24 Stunden bei 37°C im Wasserbad ausgehärtet.

Die Maße des Probekörpers (2 mm x 2 mm) wurden in der Mitte mit einer Messgenauigkeit von 0,01 mm gemessen. Anschließend wurden die Probekörper in einer Universalprüfmaschine Zwick Z005 (Zwick GmbH, Ulm, Deutschland) mit einer Vorschubgeschwindigkeit von 0,75 mm/min bis zum Bruch belastet.

Anfließverhalten: Kontaktwinkel auf trockenem Zahnschmelz

Das Anfließverhalten wurde ermittelt durch Messung des Kontaktwinkels auf trockenem Zahnschmelz.

Für die Kontaktwinkelmessungen (KW, in Grad [°]) auf trockenem Zahnschmelz wurde ein extrahierter humaner Molar verwendet. Dieser wurde vor der Messung mit einem Zellstofftuch durch Abwischen getrocknet. Anschließend wurde ein Tropfen des zu untersuchenden Materials auf den Schmelzbereich des Zahns aufgetragen. Anschließend wurde der Kontaktwinkel über einen Zeitraum von 30 Sekunden mit einem Kontaktwinkelmessgerät (DSA 100, Firma Krüss) bestimmt.

Wasseraufnahme

Die Wasseraufnahme wurde analog zu ISO 4049 bestimmt. Dazu wurden die Kompositmaterialien luftblasenfrei in entsprechende Teflonformen eingefüllt, mit Folien und Glasplatten bedeckt und mit einer Schraubzwinge wurden die Überschüsse herausgepresst. Die Probekörper mit einem Durchmesser von 15,0 ± 0,1 mm und einer Höhe von 1,0 ± 0,1 mm wurden segmentweise lichtgehärtet. Anschließend wurden die Probekörper in einen Exsikkator bei 37°C aufbewahrt. Nach 22 Stunden wurden die Probekörper entnommen, für 2 Stunden in einen zweiten Exsikkator auf 23°C gebracht und dann auf 0,1 mg gewogen. Dieser Zyklus wurde so lange wiederholt, bis eine konstante Masse, $m_1$, erreicht war.

Nach dem vollständigen Trocknen wurde der Durchmesser zweimal rechtwinklig zueinander mit einer Messgenauigkeit von 0,01 mm gemessen und daraus der mittlere Durchmesser berechnet. Die Dicke des Probekörpers wurde in der Mitte und an vier in gleichem Abstand liegenden Stellen des Randes auf 0,01 mm gemessen. Aus dem mittleren Durchmesser und der mittleren Dicke wurde das Volumen, V, berechnet.

Anschließend wurden die Probekörper für 7 Tage in Wasser bei 37°C gelagert Danach wurden die Probekörper herausgenommen, mit Wasser abgespült und abgetupft, bis auf der Oberfläche keine Feuchtigkeit mehr sichtbar war. Die Probekörper wurden 15 s in der Luft hin- und hergeschwenkt und 1 min nach dem Herausnehmen aus dem Wasser gewogen. Diese Masse wird als $m_2$ angegeben.

Anschließend wurden die Probekörper erneut in einen Exsikkator bei 37°C aufbewahrt. Nach 22 Stunden wurden die Probekörper entnommen, für 2 Stunden in einen zweiten Exsikkator auf 23°C gebracht und dann auf 0,1 mg gewogen. Dieser Zyklus wurde so lange wiederholt, bis eine konstante Masse, $m_3$, erreicht war.

Die Wasseraufnahme, $W_{sp}$, wurde nach folgender Gleichung berechnet:

$$W_{\text{sp}} = \frac{m_2 - m_3}{V}$$

Dabei ist

$m_2$ die Masse des Probekörpers nach Wasserlagerung für 7 Tage in µg;

$m_3$ die Masse des wieder getrockneten Probekörpers in µg;

$V$ das Volumen des Probekörpers in $mm^3$

Viskosität

Die Viskosität wurde mit einem Rheometer Physica MCR 301 (Anton Paar GmbH, Graz, Österreich) bestimmt.

Dazu wurde das zu untersuchende Material auf die Messplatte eines Platte/Platte-Systems aufgebracht (D = 25 mm, Spalt = 1 mm) und bei 23°C und einer Scherrate von 10/s die Viskosität bestimmt.

Bei dem nachfolgend eingesetzten Nanofüllstoff (silanisiert) handelte es sich um silanisierte Kieselsäureteilchen mit einer mittleren Partikelgröße von 40 nm.

Weitere Definitionen:

Bei der nachfolgend eingesetzten Verbindung "TCD-Monomer" handelte es sich um Bis(methacrylolyoxymethyl)-tricyclo[5.2.1.$0^{2,6}$]decan.

Bei dem nachfolgend eingesetzten Füllstoff "pyrogene Kieselsäure (organisch oberflächenmodifiziert)" handelt es sich um eine hydrophobe Kieselsäure mit einer BET-Oberfläche (nach DIN EN ISO 9277) von $150m^2/g$, einer Stampfdichte (nach DIN EN ISO 787-11) von ca. 200 g/L und einer (vermutlichen) Primärpartikelgröße von 12 nm. Die Oberfläche der Kieselsäure ist mit -OSi(CH3)3- Gruppen belegt.

Tabelle 1:

| Beispiel | 1 | 2 | 3 | 4 | Vergleich |
|---|---|---|---|---|---|
| | | | | | |
| TCD-Monomer | 0,00 | 47,12 | 31,56 | 15,72 | 0,00 |
| Monomer der Formel (2) | 21,38 | 0,00 | 0,00 | 0,00 | 0,00 |
| UDMA | 17,35 | 0,00 | 0,00 | 7,07 | 14,14 |
| TEDMA | 12,44 | 0,00 | 0,00 | 8,71 | 17,43 |
| | | | | | |
| Bis-GMA | 0,00 | 0,00 | 7,54 | 7,55 | 7,54 |
| Ethoxyliertes Bis-GMA | 0,00 | 0,00 | 8,02 | 8,07 | 8,01 |
| | | | | | |
| UV-Stabilisator | 0,25 | 0,24 | 0,24 | 0,24 | 0,24 |
| DABE | 0,22 | 0,21 | 0,21 | 0,21 | 0,21 |
| Katalysator (CC) | 0,15 | 0,14 | 0,14 | 0,14 | 0,14 |
| BHT | 0,09 | 0,09 | 0,09 | 0,09 | 0,09 |
| Glaskeramik 0,7 μm (silanisiert) | 26,85 | 45,94 | 45,94 | 45,94 | 45,94 |
| Reaktives Aluminiumsilikatglas (<10 μm) | 4,13 | 4,07 | 4,07 | 4,07 | 4,07 |
| pyrogene Kieselsäure (organisch oberflächenmodifiziert) | 1,65 | 1,59 | 1,59 | 1,59 | 1,59 |
| Titandioxid (silanisiert) | 0,62 | 0,60 | 0,60 | 0,60 | 0,60 |
| Nanofüllstoff (silanisiert) | 14,87 | 0,00 | 0,00 | 0,00 | 0,00 |
| | | | | | |
| Biegefestigkeit [MPa] | 138 | 109 | 126 | 128 | 104 |
| E-Modul [MPa] | 6319 | 3684 | 4603 | 4675 | 4446 |
| KW [°] Schmelz trocken | 29,5 | 29,0 | 51,90 | 45,10 | 67,1 |
| Wasseraufnahme [$\mu g/mm^3$] | 11,91 | 5,12 | | | 25,14 |
| Viskosität [Pas] | | 3,00 | 8,30 | 4,60 | 2,80 |
| Acta-Abrasion | 75 μm | | | | 145 μm |
| Summe Füllstoffe | 48,12 | 52,20 | 52,20 | 52,20 | 52,20 |

Tabelle 2:

| Beispiel | 5 | 6 | 7 | 8 | 9 | 10 |
|---|---|---|---|---|---|---|
| | | | | | | |
| TCD-Monomer | 23,64 | 28,37 | 18,91 | 21,38 | 17,91 | 21,38 |
| UDMA | 11,82 | 11,82 | 14,19 | 17,35 | 12,31 | 16,91 |
| TEDMA | 11,47 | 6,74 | 13,83 | 12,44 | 10,44 | 13,75 |
| | | | | | | |
| UV-Stabilisator | 0,24 | 0,24 | 0,24 | 0,25 | 0,25 | 0,25 |
| DABE | 0,21 | 0,21 | 0,21 | 0,22 | 0,22 | 0,22 |
| Katalysator (CC) | 0,14 | 0,14 | 0,14 | 0,15 | 0,15 | 0,15 |
| BHT | 0,09 | 0,09 | 0,09 | 0,09 | 0,09 | 0,09 |
| Glaskeramik 0,7 μm (silanisiert) | 46,11 | 46,11 | 46,11 | 26,85 | 27,15 | 0,00 |
| Reaktives Aluminiumsilikatglas (<10 μm) | 4,08 | 4,08 | 4,08 | 4,13 | 4,13 | 0,00 |
| pyrogene Kieselsäure (organisch oberflächenmodifiziert) | 1,60 | 1,60 | 1,60 | 1,65 | 1,65 | 0,00 |
| Titandioxid (silanisiert) | 0,60 | 0,60 | 0,60 | 0,62 | 0,62 | 0,00 |
| Nanofüllstoff (silanisiert) | 0,00 | 0,00 | 0,00 | 14,87 | 25,08 | 47,25 |
| | | | | | | |
| Biegefestigkeit [MPa] | 137 | 127 | 130 | 135 | 131 | |
| E-Modul [MPa] | 6106 | 6070 | 5334 | 6214 | 6279 | |
| KW [°] Schmelz trocken | 29,3 | 46,7 | 30,4 | 31,5 | 36,3 | 33,7 |
| Wasseraufnahme [μg/mm$^3$] | 7,61 | 6,53 | 11,72 | 13,45 | 12,61 | 12,89 |
| Acta-Abrasion | | | | | | 62 μm |
| Summe Füllstoffe | 52,39 | 52,39 | 52,39 | 48,12 | 58,63 | 47,25 |

Synthese der Verbindung der Formel (2)

0,95 g (4,84 mmol) 3(4), 8(9)-Bis(hydroxymethyl)tricyclo[5.2.1.0$^{2,6}$]decan wurden in 10 mL Toluol gelöst und mit 0,04 g BHT und 0,103 g der Katalysatorlösung versetzt. Unter Rühren wurden 3,00 g (19,34 mmol, 4 Äquivalente) Isocyanatoethylmethacrylat, gelöst in 10 mL Toluol, zugetropft. Nach beendeter Zugabe wurde der Tropftrichter durch einen Rückflusskühler ausgetauscht und das Reaktionsgemisch auf 120 °C erwärmt und der Fortschritt der Reaktion mittels IR-Spektroskopie verfolgt. Nach 72 Stunden wurden weitere 0,102 g Katalysatorlösung zugesetzt und weiter erhitzt, bis keine Isocyanatbande mehr detektiert wurde. Das Lösungsmittel wurde am Rotationsverdampfer entfernt. Das Allophanat der Formel (2) wurde in einer Ausbeute von 3,83 g (4,69 mmol, 97%) als leicht gelbliches Öl erhalten.

Die Beispiele 2 bis 10 wurden unter Austausch des dort jeweils eingesetzten TCD-Monomeren gegen die Verbindung der Formel (2) wiederholt; diese weiteren Beispiele werden als Beispiele S2 bis S10 0 bezeichnet. Es wurden sämtliche in den Beispielen 2 bis 10 bestimmten Parameter auch für die Beispiele S2 bis S10 bestimmt. Die bestimmten Parameterwerte für die Beispiele S2 bis S10 sind ähnlich zu denen aus den Beispielen 2 bis 10 und übertreffen diese teilweise. Dies zeigt neben dem erfindungsgemäßen Beispiel 1, dass die Verbindung der Formel (2), welche repräsentativ für die erfindungsgemäßen Verbindungen steht, hervorragend für den Einsatz in erfindungsgemäßen Kompositmaterialien geeignet ist.

**Patentansprüche**

1. Kompositmaterial bestehend aus oder umfassend:

   (a) als Füllstoffkomponente eine Gesamtmenge an Füllstoffen im Bereich von 0,5 bis 60 Gew.-%, bezogen auf

die Gesamtmasse des Kompositmaterials, wobei die Gesamtmenge an Füllstoffen eine Mischung von Füllstoffen ist umfassend
(a1) eine Gesamtmenge im Bereich von 0,5 bis 60 Gew.-% an nicht agglomerierten, organisch oberflächenmodifizierten Nanopartikeln mit einer mittleren Partikelgröße kleiner 200 nm
und
(a2) eine Gesamtmenge im Bereich von 0 bis 59,5 Gew.-% an Mikropartikeln mit einer mittleren Partikelgröße von 0,4 μm bis 10 μm
sowie
(a3) gegebenenfalls zusätzliche Füllstoffe,
wobei die Gewichtsprozentangaben für die Komponenten (a1) und (a2) jeweils auf die Gesamtmasse des Kompositmaterials bezogen sind,
(b) als Monomerenkomponente eine Gesamtmenge an polymerisierbaren Monomeren im Bereich von 24 bis 98,5 Gew.-%, bezogen auf die Gesamtmasse des Kompositmaterials, wobei die Gesamtmenge an polymerisierbaren Monomeren umfasst
(b1) ein, zwei oder mehr Monomere ausgewählt aus der Gruppe bestehend aus Verbindungen (Monomere) der Struktur $Q(Y_xZ_e)_b$, wobei gilt:

- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten $Y_xZ_e$ substituierten Wasserstoffatome dieses polyalicyclischen Strukturelements Q durch Alkylgruppen, Alkoxygruppen, Halogenatome oder Trifluormethylgruppen substituiert sind,
- b ist eine natürliche Zahl ausgewählt aus der Gruppe der natürlichen Zahlen 1, 2, 3 und 4,
- jedes Z bedeutet ein Strukturelement, das unabhängig von etwaigen weiteren Strukturelementen Z ausgewählt ist aus der Gruppe bestehend aus

$-O-(C{=}O)-CH{=}CH_2$, $-O-(C{=}O)-C(CH_3){=}CH_2$,

$-(C{=}O)-CH{=}CH_2$, $-(C{=}O)-C(CH_3){=}CH_2$

$-CH{=}CH_2$, $-C(CH_3){=}CH_2$ und $-O-CH{=}CH_2$,

- jeder Index e ist eine natürliche Zahl, die unabhängig von etwaigen weiteren Indizes e ausgewählt ist aus der Gruppe der natürlichen Zahlen 1, 2, 3 und 4,
- jeder Index x bedeutet unabhängig von etwaigen weiteren Indizes x 0 oder 1,
- jedes Y bedeutet in der Struktur $Q(Y_xZ_e)_b$ bei x = 1 ein Strukturelement, welches das polyalicyclische Strukturelement Q mit e Strukturelementen Z verbindet, wobei jedes Y unabhängig von etwaigen weiteren Strukturelementen Y gewählt ist,

(b2) optional ein, zwei oder mehr zusätzliche radikalisch polymerisierbare Monomere aus der Gruppe bestehend aus Acrylaten und Methacrylaten, vorzugsweise der Gruppe der Methacrylate, wobei das oder die weiteren radikalisch polymerisierbaren Monomere keine Verbindungen (Monomere) der vorstehend definierten Struktur $Q(Y_xZ_e)_b$ sind,
(c) ein oder mehrere Initiatoren und/oder Katalysatoren, vorzugsweise in einer Menge von bis zu 1 Gew.-%, bezogen auf die Gesamtmasse des Kompositmaterials,
sowie
(d) zusätzlich gegebenenfalls ein oder mehrere sonstige Additive zur Anwendung in einem therapeutischen dentalen Verfahren als Versiegelungsmaterial zur Versiegelung von Fissuren und/oder Grübchen und/oder kariösen Läsionen.

**2.** Kompositmaterial zur Anwendung nach Anspruch 1, wobei die Füllstoffkomponente

(a) umfasst
(a1) eine Gesamtmenge im Bereich von 0,5 bis 59,5 Gew.-% an nicht agglomerierten, organisch oberflächenmodifizierten Nanopartikeln mit einer mittleren Partikelgröße kleiner 60 nm
und/oder
(a2) eine Gesamtmenge im Bereich von 0,5 bis 59,5 Gew.-% an Mikropartikeln mit einer mittleren Partikelgröße im Bereich von 0,4 μm bis 10 μm

sowie
(a3) gegebenenfalls zusätzlichen Füllstoffen,
wobei die Gewichtsprozentangaben für die Komponenten (a1) und (a2) jeweils auf die Gesamtmasse des Kompositmaterials bezogen sind.

**3.** Kompositmaterial zur Anwendung nach Anspruch 1 oder 2, wobei die Füllstoffkomponente (a)

(a3) eine Gesamtmenge in Bereich von 0 bis 15 Gew.-%, vorzugsweise 0 bis 10 Gew.-%, an zusätzlichen Füllstoffen umfasst,
wobei die Gewichtsprozentangabe für die Komponente (a3) auf die Gesamtmasse des Kompositmaterials bezogen ist.

**4.** Kompositmaterial zur Anwendung nach einem der vorangehenden Ansprüche, wobei die Monomerenkomponente (b)

(b1) ein, zwei oder mehr radikalisch polymerisierbare Monomere der Struktur $Q(Y_xZ_e)_b$ wie in Anspruch 1 definiert und
(b2) ein, zwei oder mehr zusätzliche radikalisch polymerisierbare Monomere aus der Gruppe bestehend aus Acrylaten und Methacrylaten, vorzugsweise der Gruppe der Methacrylate, wobei das oder die weiteren radikalisch polymerisierbaren Monomere keine Verbindungen (Monomere) der Struktur $Q(Y_xZ_e)_b$ wie in Anspruch 1 definiert sind,
enthält,
wobei das Verhältnis der Gesamtmasse der Komponente (b1) zur Gesamtmasse der Komponente (b2) vorzugsweise im Bereich von 4 : 1 bis 1 : 3, bevorzugt im Bereich von 3 : 1 bis 1 : 2 liegt.

**5.** Kompositmaterial zur Anwendung nach einem der vorangehenden Ansprüche, **dadurch gekennzeichnet, dass** das Strukturelement Q der Verbindungen der Struktur $Q(Y_xZ_e)_b$ der Komponente (b1) einen Tricyclo[5.2.1.0$^{2,6}$]decan-Rest, einen Tricyclo[5.2.1.0$^{2,6}$]dec-3-en-Rest, einen Tricyclo[3.3.1.1$^{3,7}$]decan-Rest oder einen Bicyclo[2.2.1]heptan-Rest bedeutet.

**6.** Kompositmaterial zur Anwendung nach einem der vorangehenden Ansprüche, **dadurch gekennzeichnet, dass** ein, zwei oder mehr Verbindungen der Struktur $Q(Y_xZ_e)_b$ ein Tricyclo[5.2.1.0$^{2,6}$]-decan- oder Tricyclo[5.2.1.0$^{2,6}$]-decen-Strukturelement aufweisen und Z vorzugsweise ausgewählt ist aus der Gruppe bestehend aus $-O-(C=O)-CH=CH_2$ und $-O-(C=O)-C(CH_3)=CH_2$, bevorzugt bedeutet Z die Gruppe $-O-(C=O)-C(CH_3)=CH_2$.

**7.** Kompositmaterial zur Anwendung nach einem der vorangehenden Ansprüche, **dadurch gekennzeichnet, dass** Komponente (b1) umfasst oder besteht aus Bis(methacrylolyoxymethyl)tricyclo[5.2.1.0$^{2,6}$]decan und/oder Bis(acrylolyoxymethyl)tricyclo[5.2.1.0$^{2,6}$]decan.

**8.** Kompositmaterial zur Anwendung nach einem der vorangehenden Ansprüche, wobei die Monomerenkomponente (b2) Monomere umfasst oder aus diesen besteht, die ausgewählt sind aus der Gruppe bestehend aus Triethylenglykoldimethacrylat (TEDMA), Urethandimethacrylat (7,7,9-Trimethyl-4,13-dioxo-3,14-dioxa-5,12-diazahexadecan-1,16-dioxydimethacrylat, UDMA) und deren Mischungen.

**9.** Kompositmaterial zur Anwendung nach einem der vorangehenden Ansprüche, **dadurch gekennzeichnet, dass** das Kompositmaterial kein Bis-GMA enthält, vorzugsweise keine Verbindung mit einem Bisphenol-A-Strukturelement enthält.

**10.** Kompositmaterial zur Anwendung nach einem der vorangehenden Ansprüche, wobei zumindest ein Teil der Mikropartikel der Komponente (a2) organisch oberflächenmodifizierte Partikel, vorzugsweise silanisierte Partikel, sind und/oder
zumindest ein Teil der Mikropartikel der Komponente (a2) Dentalglas-Partikel sind,
wobei vorzugsweise zumindest ein Teil der Mikropartikel der Komponente (a2) organisch oberflächenmodifizierte Dentalglas-Partikel, vorzugsweise silanisierte Dentalglas-Partikel, sind.

**11.** Kompositmaterial zur Anwendung nach einem der vorangehenden Ansprüche, wobei Komponente (a1) nicht agglomerierte, organisch oberflächenmodifizierte Nanopartikel ausgewählt aus der Gruppe bestehend aus Oxiden und Mischoxiden, vorzugsweise ausgewählt aus der Gruppe bestehend aus Oxiden und Mischoxiden der Elemente

Silizium, Titan, Yttrium, Strontium, Barium, Zirkon, Hafnium, Niob, Tantal, Wolfram, Wismut, Molybdän, Zinn, Zink, Ytterbium, Lanthan, Cer, Aluminium und deren Mischungen umfasst.

**12.** Kompositmaterial zur Anwendung nach einem der vorangehenden Ansprüche, **dadurch gekennzeichnet, dass** die Füllstoffmischung (a) ausschließlich (a1) nicht agglomerierte, organisch oberflächenmodifizierte Nanopartikel mit einer mittleren Partikelgröße kleiner 200 nm umfasst.

**13.** Kompositmaterial zur Anwendung nach einem der vorangehenden Ansprüche, **dadurch gekennzeichnet, dass** der Kontaktwinkel des Kompositmaterials auf trockenem Zahnschmelz, gemessen mit dem Kontaktwinkelmessgerät DSA100 der Firma Krüss, weniger als 60°, bevorzugt weniger als 50°, besonders bevorzugt weniger als 40° beträgt.

**14.** Kompositmaterial zur Anwendung nach einem der vorangehenden Ansprüche, **dadurch gekennzeichnet, dass** die Wasseraufnahme des Kompositmaterials weniger als 15 $\mu g/mm^3$ beträgt, bevorzugt weniger als 13 $\mu g/mm^3$, besonders bevorzugt weniger als 10 $\mu g/mm^3$.

**15.** Kompositmaterial zur Anwendung nach einem der vorangehenden Ansprüche, umfassend eine Komponente (a2), welche

- eine oder mehrere erste Mikropartikelfraktionen, die jeweils eine mittlere Partikelgröße im Bereich von 1 $\mu m$ bis 10 $\mu m$ besitzen,
und
- eine oder mehrere zweite Mikropartikelfraktionen, die jeweils eine mittlere Partikelgröße im Bereich von > 0,4 $\mu m$ bis < 1 $\mu m$ besitzen,
aufweist.

## Claims

**1.** A composite material consisting of or comprising:

(a) as filler component a total quantity of fillers in the range of 0.5 to 60 wt.%, based on the total mass of the composite material, wherein the total quantity of fillers is a mixture of fillers comprising
(a1) a total quantity in the range of 0.5 to 60 wt.% of non-agglomerated, organically surface-modified nanoparticles with an average particle size of less than 200 nm
and
(a2) a total quantity in the range of 0 to 59.5 wt.% of microparticles with an average particle size of 0.4 $\mu m$ to 10 $\mu m$, and
(a3) optionally additional fillers,
wherein the weight percentages given for components (a1) and (a2) are based in each case on the total mass of the composite material,
(b) as monomer component a total quantity of polymerisable monomers in the range of 24 to 98.5 wt.%, based on the total mass of the composite material, wherein the total quantity of polymerisable monomers comprises
(b1) one, two or more monomers selected from the group consisting of compounds (monomers) with the structure $Q(Y_xZ_e)_b$, wherein the following applies:

- Q represents a saturated or olefinically unsaturated polyalicyclic structural element selected from the group consisting of bicyclic, tricyclic, tetracyclic, pentacyclic and hexacyclic hydrocarbon residues, wherein optionally one, two or more of the hydrogen atoms of this polyalicyclic structural element Q not substituted by substituents $Y_xZ_e$ are substituted by alkyl groups, alkoxy groups, halogen atoms or trifluoromethyl groups;
- b is a natural number selected from the group of natural numbers 1, 2, 3 and 4,
- each Z represents a structural element, which independently of any other structural elements Z is selected from the group consisting of

$-O-(C=O)-CH=CH_2$, $-O-(C=O)-C(CH_3)=CH_2$,

$-(C=O)-CH=CH_2$, $-(C=O)-C(CH_3)=CH_2$

$-CH=CH_2$, $-C(CH_3)=CH_2$ and $-O-CH=CH_2$,

- each index e is a natural number, which independently of any other indices e is selected from the group of natural numbers 1, 2, 3 and 4,
- each index x independently of any other indices x represents 0 or 1,
- each Y in the structure $Q(Y_xZ_e)_b$ where x = 1 represents a structural element, which links the polyalicyclic structural element Q with e structural elements Z, wherein each Y is selected independently of any other structural elements Y,

(b2) optionally one, two or more additional monomers capable of free radical polymerisation from the group consisting of acrylates and methacrylates, preferably the group of the methacrylates, wherein the further monomer(s) capable of free radical polymerisation are not compounds (monomers) of the structure $Q(Y_xZ_e)_b$ defined above,
(c) one or more initiators and/or catalysts, preferably in a quantity of up to 1 wt.%, based on the total mass of the composite material,
and
(d) in addition, optionally one or more other additives
for use in a therapeutic dental process as a sealing material for sealing fissures and/or pits and/or carious lesions.

**2.** The composite material for the use according to claim 1, wherein the filler component (a) comprises

(a1) a total quantity in the range of 0.5 to 59.5 wt.% of non-agglomerated, organically surface-modified nanoparticles with an average particle size of less than 60 nm
and/or
(a2) a total quantity in the range of 0.5 to 59.5 wt.% of microparticles with an average particle size in the range of 0.4 μm to 10 μm,
and
(a3) optionally additional fillers,
wherein the weight percentages given for components (a1) and (a2) are based in each case on the total mass of the composite material.

**3.** The composite material for the use according to claim 1 or 2, wherein the filler component (a) comprises

(a3) a total quantity in the range of 0 to 15 wt.%, preferably 0 to 10 wt.%, of additional fillers,
wherein the weight percentage given for component (a3) is based on the total mass of the composite material.

**4.** The composite material for the use according to one of the preceding claims, wherein the monomer component (b) contains

(b1) one, two or more monomers capable of free radical polymerisation of the structure $Q(Y_xZ_e)_b$ as defined in claim 1 and
(b2) one, two or more additional monomers capable of free radical polymerisation from the group consisting of acrylates and methacrylates, preferably the group of the methacrylates, wherein the further monomer(s) capable of free radical polymerisation are not compounds (monomers) of the structure $Q(Y_xZ_e)_b$ as defined in claim 1,

wherein the ratio of the total mass of component (b1) to the total mass of component (b2) is preferably in the range of 4:1 to 1:3, preferably in the range of 3:1 to 1:2.

**5.** The composite material for the use according to one of the preceding claims, **characterised in that** the structural element Q of the compounds of the structure $Q(Y_xZ_e)_b$ of component (b1) represents a tricyclo[5.2.1.0$^{2,6}$]decane residue, a tricyclo[5.2.1.0$^{2,6}$]dec-3-ene residue, a tricyclo[3.3.1.1$^{3,7}$]decane residue or a bicyclo[2.2.1]heptane residue.

**6.** The composite material for the use according to one of the preceding claims, **characterised in that** one, two or more compounds of the structure $Q(Y_xZ_e)_b$ have a tricyclo[5.2.1.0$^{2,6}$]decane or tricyclo[5.2.1.0$^{2,6}$]decene structural element and Z is preferably selected from the group consisting of $-O-(C=O)-CH=CH_2$ and $-O-(C=O)-C(CH_3)=CH_2$; preferably, Z represents the group $-O-(C=O)-C(CH_3)=CH_2$.

**7.** The composite material for the use according to one of the preceding claims, **characterised in that** component

(b1) comprises or consists of bis(methacryloyloxymethyl)tricyclo[5.2.1.0$^{2,6}$]decane and/or bis(acryloyloxy-methyl)tricyclo[5.2.1.0$^{2,6}$]decane.

**8.** The composite material for the use according to one of the preceding claims, wherein the monomer component (b2) comprises or consists of monomers selected from the group consisting of triethylene glycol dimethacrylate (TEDMA), urethane dimethacrylate (7,7,9-trimethyl-4,13-dioxo-3,14-dioxa-5,12-diazahexadecane-1,16-dioxydimethacrylate, UDMA) and mixtures thereof.

**9.** The composite material for the use according to one of the preceding claims, **characterised in that** the composite material does not contain any bis-GMA and preferably does not contain any compound with a bisphenol A structural element.

**10.** The composite material for the use according to one of the preceding claims, wherein at least a portion of the microparticles of component (a2) are organically surface-modified particles, preferably silanised particles
and/or
at least a portion of the microparticles of component (a2) are dental glass particles,
wherein preferably at least a portion of the microparticles of component (a2) are organically surface-modified dental glass particles, preferably silanised dental glass particles.

**11.** The composite material for the use according to one of the preceding claims, wherein component (a1) comprises non-agglomerated, organically surface-modified nanoparticles selected from the group consisting of oxides and mixed oxides, preferably selected from the group consisting of oxides and mixed oxides of the elements silicon, titanium, yttrium, strontium, barium, zirconium, hafnium, niobium, tantalum, tungsten, bismuth, molybdenum, tin, zinc, ytterbium, lanthanum, cerium, aluminium and mixtures thereof.

**12.** The composite material for the use according to one of the preceding claims, **characterised in that** the filler mixture (a) comprises exclusively (a1) non-agglomerated, organically surface-modified nanoparticles with an average particle size of less than 200 nm.

**13.** The composite material for the use according to one of the preceding claims, **characterised in that** the contact angle of the composite material on dry tooth enamel, measured with the contact angle measuring instrument DSA 100 from Krüss, is less than 60°, preferably less than 50°, particularly preferably less than 40°.

**14.** The composite material for the use according to one of the preceding claims, **characterised in that** the water absorption of the composite material is less than 15 $\mu$g/mm$^3$, preferably less than 13 $\mu$g/mm$^3$, particularly preferably less than 10 $\mu$g/mm$^3$.

**15.** The composite material for the use according to one of the preceding claims, comprising a component (a2) having

- one or more first microparticle fractions, which in each case possess an average particle size in the range of 1 $\mu$m to 10 $\mu$m,
and
- one or more second microparticle fractions, which in each case possess an average particle size in the range of > 0.4 $\mu$m to < 1 $\mu$m.

## Revendications

**1.** Matériau composite consistant en ou comprenant :

(a) comme composant de substance de charge, une quantité totale de substances de charge comprise entre 0,5 et 60 % en poids par rapport à la masse totale du matériau composite, dans lequel la quantité totale de substances de charge est un mélange de substances de charge comprenant
(a1) une quantité totale de nanoparticules organiquement modifiées en surface, non agglomérées, présentant une taille moyenne de particules inférieure à 200 nm comprise entre 0,5 et 60 % en poids.
et
(a2) une quantité totale de microparticules présentant une taille moyenne de particules de 0,4 $\mu$m à 10 $\mu$m comprise entre 0 et 59,5 % en poids

et

(a3) éventuellement des substances de charge supplémentaires,

dans lequel les pourcentages en poids pour les composants (a1) et (a2) se rapportent respectivement à la masse totale du matériau composite,

(b) comme composants monomères, une quantité totale de monomères polymérisables comprise entre 24 et 98,5 % en poids par rapport à la masse totale du matériau composite, dans lequel la quantité totale de monomères polymérisables comprend

(b1) un, deux ou plusieurs monomères choisis dans le groupe constitué de composés (monomères) de structure $Q(Y_xZ_e)_b$, dans laquelle :

- Q désigne un élément structurel poly-alicyclique saturé ou oléfiniquement insaturé choisi dans le groupe constitué par les radicaux hydrocarbures bicycliques, tricycliques, tétracycliques, pentacycliques et hexacycliques, dans lequel optionnellement un, deux ou plusieurs des atomes d'hydrogène non substitués par des substituants $Y_xZ_e$ de cet élément structurel poly-alicyclique Q sont substitués par des groupes alkyle, alcoxy, des halogénoatomes ou des groupes trifluorométhyle,
- b est un nombre naturel choisi dans le groupe des nombres naturels 1, 2, 3 et 4,
- chaque Z désigne un élément structurel qui est choisi indépendamment d'autres éléments structurels Z éventuels dans le groupe consistant en

$$-O-(C{=}O)-CH{=}CH_2,\ -O-(C{=}O)-C(CH_3)\ {=}CH_2,$$

$$-(C{=}O)-CH{=}CH_2,\ -(C{=}O)-C(CH_3){=}CH_2$$

$$-CH{=}CH_2,\ -C(CH_3){=}CH_2 \text{ et } -O-CH-CH_2,$$

- chaque indice e est un nombre naturel qui est choisi indépendamment d'autres indices e éventuels dans le groupe des nombres naturels 1, 2, 3 et 4,
- chaque indice x désigne indépendamment d'autres indices x éventuels 0 ou 1,
- chaque Y désigne dans la structure $Q(Y_xZ_e)_b$, dans laquelle x = 1, un élément structurel qui lie l'élément structurel poly-alicyclique Q avec e éléments structurels Z, dans lequel chaque Y est choisi indépendamment d'autres éléments structurels Y éventuels,

(b2) optionnellement un, deux ou plusieurs monomères supplémentaires, polymérisables de manière radicalaire, du groupe constitué par les acrylates et les méthacrylates, de préférence du groupe des méthacrylates, dans lequel le ou les autres monomères polymérisables de manière radicalaire ne sont pas des composés (monomères) de structure $Q(Y_xZ_e)_b$ définie précédemment,

(c) un ou plusieurs initiateurs et/ou catalyseurs, de préférence dans une quantité allant jusqu'à 1 % en poids, par rapport à la masse totale du matériau composite,

et

(d) en outre éventuellement un ou plusieurs autres additifs

pour une utilisation dans un procédé thérapeutique dentaire comme matériau de scellement pour combler les fissures et/ou des petits trous et/ou des lésions carieuses.

**2.** Matériau composite pour son utilisation selon la revendication 1, dans lequel le composant de substance de charge (a) comprend

(a1) une quantité totale de nanoparticules non agglomérées, organiquement modifiées en surface, présentant une taille moyenne de particules inférieure à 60 nm comprise entre 0,5 et 59,5 % en poids

et/ou

(a2) une quantité totale de microparticules présentant une taille moyenne de particules dans la plage de 0,4 $\mu$m à 10 $\mu$m comprise entre de 0,5 et 59,5 % en poids

et

(a3) éventuellement des substances de charge supplémentaires,

dans lequel les pourcentages en poids pour les composants (a1) et (a2) se rapportent respectivement à la masse totale du matériau composite.

**3.** Matériau composite pour son utilisation selon la revendication 1 ou 2, dans lequel le composant de substance de charge (a)

(a3) comprend une quantité totale, de substances de charge supplémentaires, comprise entre 0 et 15 % en poids de préférence entre 0 et 10 % en poids
dans lequel les pourcentages en poids pour le composant (a3) se rapportent à la masse totale du matériau composite.

**4.** Matériau composite pour son utilisation selon l'une des revendications précédentes, dans lequel les composants monomères (b) contiennent

(b1) un, deux ou plusieurs monomères polymérisables de manière radicalaire de structure $Q(Y_xZ_e)_b$ telle que définie dans la revendication 1 et
(b2) un, deux ou plusieurs monomères supplémentaires polymérisables de manière radicalaire du groupe constitué par les acrylates et les méthacrylates, de préférence du groupe des méthacrylates, dans lequel le ou les autres monomères polymérisables de manière radicalaire ne sont pas des composés (monomères) de structure $Q(Y_xZ_e)_b$ telle que définie dans la revendication 1,
dans lequel le rapport de la masse totale du composant (b1) à la masse totale du composant (b2) se situe de préférence entre 4:1 et 1:3, de manière encore préféré entre 3:1 et 1:2.

**5.** Matériau composite pour son utilisation selon l'une des revendications précédentes, **caractérisé en ce que** l'élément structurel Q des composés de structure $Q(Y_xZ_e)_b$ du composant (b1) désigne un radical tricyclo[5.2.1.0$^{2,6}$]décane, un radical tricyclo [5.2.1.0$^{2,6}$]déc-3-ène, un radical tricyclo[3.3.1.1$^{3,7}$] décane ou un radical bicyclo[2.2.1]heptane.

**6.** Matériau composite pour son utilisation selon l'une des revendications précédentes, **caractérisé en ce qu'**un, deux ou plusieurs composés de structure $Q(Y_xZ_e)_b$ présentent un élément structurel tricyclo[5.2.1.0$^{2,6}$] décane ou tricyclo[5.2.1.0$^{2,6}$]décène et Z est choisi de préférence dans le groupe constitué par $-O-(C=O)-CH=CH_2$ et $-O-(C-O)-C(CH_3)=CH_2$, de manière encore préféré
Z désigne le groupe $-O-(C-O)-C(CH_3)-CH_2$.

**7.** Matériau composite pour son utilisation selon l'une des revendications précédentes, **caractérisé en ce que** le composant (b1) comprend ou consiste en du bis(méthacrylolyoxyméthyl)tricyclo[5.2.1.0$^{2,6}$]décane et/ou bis(acrylolyoxyméthyl)tricyclo[5.2.1.0$^{2,6}$] décane.

**8.** Matériau composite pour son utilisation selon l'une des revendications précédentes, dans lequel le composant monomère (b2) comprend ou consiste en des monomères qui sont choisis dans le groupe constitué par le triéthylèneglycoldiméthacrylate (TEDMA), l'uréthane-diméthacrylate (7,7,9-triméthyl-4,13-dioxo-3,14-dioxa-5,12-diazahexadécane-1,16-dioxydiméthacrylate (UDMA) et leurs mélanges.

**9.** Matériau composite pour son utilisation selon l'une des revendications précédentes, **caractérisé en ce que** le matériau composite ne contient pas de bis-GMA, et de préférence aucun composé présentant un élément structurel de bisphénol A.

**10.** Matériau composite pour son utilisation selon l'une des revendications précédentes, dans lequel au moins une partie des microparticules du composant (a2) sont des particules organiquement modifiées en surface, de préférence des particules silanisées,
et/ou
au moins une partie des microparticules du composant (a2) sont des particules de verre dentaire,
dans lequel de préférence au moins une partie des microparticules du composant (a2) sont des particules de verre dentaire organiquement modifiées en surface, de préférence des particules de verre dentaire silanisées.

**11.** Matériau composite pour son utilisation selon l'une des revendications précédentes, dans lequel le composant (a1) comprend des nanoparticules non agglomérées, organiquement modifiées en surface, choisies dans le groupe consistant en oxydes et oxydes mixtes, de préférence choisis dans le groupe consistant en oxydes et oxydes mixtes des éléments silicium, titane, yttrium, strontium, baryum, zircone, hafnium, niobium, tantale, tungstène, bismuth, molybdène, étain, zinc, ytterbium, lanthane, cérium, aluminium et leurs mélanges.

**12.** Matériau composite pour son utilisation selon l'une des revendications précédentes, **caractérisé en ce que** le mélange de substance de charge (a) comprend exclusivement (a1) des nanoparticules non agglomérées, organiquement modifiées en surface, présentant une taille moyenne de particules inférieure à 200 nm.

**13.** Matériau composite pour son utilisation selon l'une des revendications précédentes, **caractérisé en ce que** l'angle de contact du matériau composite sur l'émail dentaire sec, mesuré avec l'appareil de mesure d'angle de contact DSA 100 de la société Krüss, est inférieur à 60°, de préférence inférieur à 50°, de manière particulièrement préférée inférieur à 40°.

**14.** Matériau composite pour son utilisation selon l'une des revendications précédentes, **caractérisé en ce que** l'absorption d'eau du matériau composite est inférieure à 15 $\mu g/mm^3$, de préférence inférieure à 13 $\mu g/mm^3$, de manière particulièrement préférée inférieure à 10 $\mu g/mm^2$.

**15.** Matériau composite pour son utilisation selon l'une des revendications précédentes, comprenant un composant (a2) qui comprend

- une ou plusieurs premières fractions de microparticules qui possèdent respectivement une taille moyenne de particules comprise entre 1 µmet 10 µm, et
- une ou plusieurs deuxièmes fractions de microparticules qui possèdent respectivement une taille moyenne de particules comprises entre > 0,4 µm et < 1 µm.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 2301067
- US 6573312 B2
- US 20050288387 A1
- US 20090047633 A1
- EP 0969789 B1
- US 6899948 B2
- WO 2007028159 A2
- WO 0130307 A1
- US 20060063853 A1
- US 20070166450 A1
- US 20100016464 A1
- WO 2005094757 A1
- EP 1307173 B1
- DE 202006020483 U1
- DE 202006020480 U1
- DE 202006020479 U1
- DE 202006020477 U1
- DE 202006020476 U1
- EP 2023884 A1
- EP 1854445 A1
- EP 2145613 A1
- EP 2151229 A2
- DE 2816823 C2
- EP 0254185 A1
- DE 102007034457
- EP 2016931 A2
- DE 102005021332
- US 7601767 B2
- DE 3522005 A1
- DE 3522006 A1
- DE 3522005
- US 6617413 B1
- EP 1238993 A
- EP 0209700 A2
- EP 0000194 A1
- US 4160080 A
- EP 0682012 B1
- EP 1727846 B1
- EP 0712840 B1
- EP 0867457 B1
- DE 102007040240 A1
- EP 1645582 A1
- DE 102007040239 A1
- DE 102004060285 A1
- WO 2006063891 A1
- US 6670499 B1
- US 6670499 B
- JP 7206740 A
- EP 1112995 B1
- EP 0049631 B1
- DE 10352260 B3
- EP 0023686 B1
- DE 3338077 A1
- EP 0143613 B1
- DE 3703120 A1
- WO 2009065873 A2
- DE 3941629 A1
- EP 0980682 A1
- EP 0948955 A1
- DE 102006019092 A1
- DE 3941629 C2
- DE 102006019092
- DE 60116142
- DE 3801511 C2
- DE 102006050153 A1
- EP 0184095 B1
- DE 4231579 C2
- EP 0366977 B1
- US 7081485 B2
- DE 3236026 A1
- US 20070027229 A1
- EP 0262629 B1
- EP 0073413 A
- US 7148382 B2
- US 5761169 A
- DE 19708294 A1
- EP 0057474 A
- EP 0047902 A
- EP 0007508 A
- DE 60029481 T2
- EP 0980682 B1
- EP 0948955 B1
- EP 1236459 B1
- EP 0173567 A2
- US 4772530 A
- US 4954414 A
- US 4874450 A
- US 5055372 A
- US 5057393 A
- EP 1720506 A
- EP 1839640 A
- DE 1495520
- WO 02092021 A
- WO 02092023 A
- EP 0059451 A
- EP 0783880 B1
- DE 10119831 A1
- EP 1563821 A1

**In der Beschreibung aufgeführte Nicht-Patentliteratur**


- **J.-P. FOUASSIER.** Photoinitiation, Photopolymerization and Photocuring. Hanser Publishers, 1995
- Radiation Curing in Polymer Science and Technology. Elsevier Applied Science, 1993, vol. II
- Die Drei-Medien Acta Abrasion wurde gemäß. *J. Dent.,* 1994, vol. 1 (22), 21-27